# EUROPEAN PATENT SPECIFICATION

(11) **EP 2 963 116 B1**
(45) Date of publication and mention of the grant of the patent: **11.11.2020**
(21) Application number: 15180416.8
(22) Date of filing: 03.03.2010
(51) Int. Cl.: C12N 15/63, C12N 15/11, C07H 19/00, C12Q 1/68

(54) **TREATMENT OF SIRTUIN 1 (SIRT1) RELATED DISEASES BY INHIBITION OF NATURAL ANTISENSE TRANSCRIPT TO SIRT 1**
BEHANDLUNG VON SIRTUIN 1 (SIRT1)-BEDINGTEN ERKRANKUNGEN MITTELS HEMMUNG DES NATÜRLICHEN ANTISENSE-TRANSKRIPTS GEGEN SIRT 1
TRAITEMENT DE SIRTUINE 1 (SIRT1) ASSOCIÉE À DES MALADIES PAR INHIBITION DU TRANSCRIT ANTISENS NATUREL DE SIRT 1

(30) Priority: 04.03.2009 US 157255 P; 06.11.2009 US 259072 P; 02.12.2009 WO PCT/US2009/066445
(43) Date of publication of application: 06.01.2016
(62) Divisional of application: 10749298.5
(73) Proprietor: CuRNA, Inc., Miami, FL 33137 (US)
(72) Inventor: COLLARD, Joseph, Delray Beach, Florida 33483 (US); KHORKOVA SHERMAN, Olga, Tequesta, Florida 33469 (US); COITO, Carlos, West Palm Beach, Florida 33417 (US); DE LEON, Belinda, West Palm Beach, Florida 33401 (US)
(74) Representative: Mathys & Squire

(56) References cited:
- WO-A2-2010/065662
- DATABASE BIOSIS [Online] BIOSCIENCES INFORMATION SERVICE, PHILADELPHIA, PA, US; October 2008 (2008-10), KUEHBACHER ANGELIKA ET AL: "MicroRNA 92a Controls Vessel Growth And Functional Recovery After Ischemia", XP002684082, Database accession no. PREV200900197869
- BONAUER ANGELIKA ET AL: "MicroRNA-92a controls angiogenesis and functional recovery of ischemic tissues in mice", SCIENCE, vol. 324, no. 5935, 26 June 2009 (2009-06-26), pages 1710-1713, XP002575234, AMERICAN ASSOCIATION FOR THE ADVANCEMENT OF SCIENCE, US ISSN: 0036-8075
- SUN ET AL: "Downregulation of Sirt1 by antisense oligonucleotides induces apoptosis and enhances radiation sensitization in A549 lung cancer cells", LUNG CANCER, vol. 58, no. 1, 14 September 2007 (2007-09-14), pages 21-29, XP022242948, ELSEVIER, AMSTERDAM, NL ISSN: 0169-5002, DOI: 10.1016/J.LUNGCAN.2007.05.013
- WAHLESTEDT ET AL: "Natural antisense and noncoding RNA transcripts as potential drug targets", DRUG DISCOVERY TODAY, vol. 11, no. 11-12, 1 June 2006 (2006-06-01), pages 503-508, XP027889295, ELSEVIER, RAHWAY, NJ, US ISSN: 1359-6446 [retrieved on 2006-06-01]
- MUNEKAZU YAMAKUCHI ET AL: "miR-34a repression of SIRT1 regulates apoptosis", PROCEEDINGS OF THE NATIONAL ACADEMY OF SCIENCES, NATIONAL ACADEMY OF SCIENCES, US, vol. 105, no. 36, 9 September 2008 (2008-09-09), pages 13421-13426, XP008149911, ISSN: 0027-8424, DOI: 10.1073/PNAS.0801613105 [retrieved on 2008-08-28]
- SMALHEISER N R ET AL: "Natural antisense transcripts are co-expressed with sense mRNAs in synaptoneurosomes of adult mouse forebrain", NEUROSCIENCE RESEARCH, ELSEVIER, SHANNON, IR, vol. 62, no. 4, 1 December 2008 (2008-12-01), pages 236-239, XP025669354, ISSN: 0168-0102, DOI: 10.1016/J.NEURES.2008.08.010 [retrieved on 2008-09-04]
- THIMMAPPA S. ANEKONDA ET AL: "Neuronal protection by sirtuins in Alzheimer's disease", JOURNAL OF NEUROCHEMISTRY, vol. 96, no. 2, 1 January 2006 (2006-01-01), pages 305-313, XP055039113, ISSN: 0022-3042, DOI: 10.1111/j.1471-4159.2005.03492.x
- DATABASE ENA [Online] 23 September 2008 (2008-09-23), "Mus musculus 13 days embryo male testis cDNA, RIKEN full-length enriched library, clone:6030437B01 product:synembryn, full insert sequence.", XP002745421, retrieved from EBI accession no. EM_HTC:AK031466 Database accession no. AK031466
- DATABASE ENA [Online] 4 March 2000 (2000-03-04), "xj35b03.x1 Soares_NFL_T_GBC_S1 Homo sapiens cDNA clone IMAGE:2659181 3', mRNA sequence.", XP002745422, retrieved from EBI accession no. EM_EST:AW169958 Database accession no. AW169958
- APARNA PURUSHOTHAM ET AL: "Hepatocyte-Specific Deletion of SIRT1 Alters Fatty Acid Metabolism and Results in Hepatic Steatosis and Inflammation", CELL METABOLISM, vol. 9, no. 4, 8 April 2009 (2009-04-08), pages 327-338, XP055039151, ISSN: 1550-4131, DOI: 10.1016/j.cmet.2009.02.006
- DATABASE EMBL [Online] 23 September 2008 (2008-09-23), "Mus musculus adult retina cDNA, RIKEN full-length enriched library, clone:A930026H04 product:hypothetical protein, full insert sequence.", retrieved from EBI accession no. EM_EST:AK044604 Database accession no. AK044604
- L R Saunders ET AL: "Sirtuins: critical regulators at the crossroads between cancer and aging", ONCOGENE, vol. 26, no. 37, 1 August 2007 (2007-08-01), pages 5489-5504, XP055439842, ISSN: 0950-9232, DOI: 10.1038/sj.onc.1210616

### SEQUENCE LISTING

<110> CuRNA, Inc.
<120> TREATMENT OF SIRTUIN 1 (SIRT1) RELATED DISEASES BY INHIBITION OF NATURAL ANTISENSE TRANSCRIPT TO SIRT1
<130> P37642EP-D1-PCT
<140> Not yet assigned
   <141> 2010-03-03
<150> EP10749298.5
   <151> 2010-03-03
<150> US 61/157,255
   <151> 2009-03-04
<150> US 61/259,072
   <151> 2009-11-06
<150> PCT/US2009/066445
   <151> 2009-12-02
<160> 73
<170> PatentIn version 3.5
<210> 1
   <211> 4107
   <212> DNA
   <213> Homo sapiens
<300>
   <308> NM_012238.3
   <309> 2010-08-29
   <313> (1)..(4107)
<400> 1
   gtcgagcggg agcagaggag gcgagggagg agggccagag aggcagttgg aagatggcgg      60
   acgaggcggc cctcgccctt cagcccggcg gctccccctc ggcggcgggg gccgacaggg     120
   aggccgcgtc gtcccccgcc ggggagccgc tccgcaagag gccgcggaga gatggtcccg     180
   gcctcgagcg gagcccgggc gagcccggtg gggcggcccc agagcgtgag gtgccggcgg     240
   cggccagggg ctgcccgggt gcggcggcgg cggcgctgtg gcgggaggcg gaggcagagg     300
   cggcggcggc aggcggggag caagaggccc aggcgactgc ggcggctggg gaaggagaca     360
   atgggccggg cctgcagggc ccatctcggg agccaccgct ggccgacaac ttgtacgacg     420
   aagacgacga cgacgagggc gaggaggagg aagaggcggc ggcggcggcg attgggtacc     480
   gagataacct tctgttcggt gatgaaatta tcactaatgg ttttcattcc tgtgaaagtg     540
   atgaggagga tagagcctca catgcaagct ctagtgactg gactccaagg ccacggatag     600
   gtccatatac ttttgttcag caacatctta tgattggcac agatcctcga acaattctta     660
   aagatttatt gccggaaaca atacctccac ctgagttgga tgatatgaca ctgtggcaga     720
   ttgttattaa tatcctttca gaaccaccaa aaaggaaaaa aagaaaagat attaatacaa 780
   ttgaagatgc tgtgaaatta ctgcaagagt gcaaaaaaat tatagttcta actggagctg 840
   gggtgtctgt ttcatgtgga atacctgact tcaggtcaag ggatggtatt tatgctcgcc 900
   ttgctgtaga cttcccagat cttccagatc ctcaagcgat gtttgatatt gaatatttca 960
   gaaaagatcc aagaccattc ttcaagtttg caaaggaaat atatcctgga caattccagc 1020
   catctctctg tcacaaattc atagccttgt cagataagga aggaaaacta cttcgcaact 1080
   atacccagaa catagacacg ctggaacagg ttgcgggaat ccaaaggata attcagtgtc 1140
   atggttcctt tgcaacagca tcttgcctga tttgtaaata caaagttgac tgtgaagctg 1200
   tacgaggaga tatttttaat caggtagttc ctcgatgtcc taggtgccca gctgatgaac 1260
   cgcttgctat catgaaacca gagattgtgt tttttggtga aaatttacca gaacagtttc 1320
   atagagccat gaagtatgac aaagatgaag ttgacctcct cattgttatt gggtcttccc 1380
   tcaaagtaag accagtagca ctaattccaa gttccatacc ccatgaagtg cctcagatat 1440
   taattaatag agaacctttg cctcatctgc attttgatgt agagcttctt ggagactgtg 1500
   atgtcataat taatgaattg tgtcataggt taggtggtga atatgccaaa ctttgctgta 1560
   accctgtaaa gctttcagaa attactgaaa aacctccacg aacacaaaaa gaattggctt 1620
   atttgtcaga gttgccaccc acacctcttc atgtttcaga agactcaagt tcaccagaaa 1680
   gaacttcacc accagattct tcagtgattg tcacactttt agaccaagca gctaagagta 1740
   atgatgattt agatgtgtct gaatcaaaag gttgtatgga agaaaaacca caggaagtac 1800
   aaacttctag gaatgttgaa agtattgctg aacagatgga aaatccggat ttgaagaatg 1860
   ttggttctag tactggggag aaaaatgaaa gaacttcagt ggctggaaca gtgagaaaat 1920
   gctggcctaa tagagtggca aaggagcaga ttagtaggcg gcttgatggt aatcagtatc 1980
   tgtttttgcc accaaatcgt tacattttcc atggcgctga ggtatattca gactctgaag 2040
   atgacgtctt atcctctagt tcttgtggca gtaacagtga tagtgggaca tgccagagtc 2100
   caagtttaga agaacccatg gaggatgaaa gtgaaattga agaattctac aatggcttag 2160
   aagatgagcc tgatgttcca gagagagctg gaggagctgg atttgggact gatggagatg 2220
   atcaagaggc aattaatgaa gctatatctg tgaaacagga agtaacagac atgaactatc 2280
   catcaaacaa atcatagtgt aataattgtg caggtacagg aattgttcca ccagcattag 2340
   gaactttagc atgtcaaaat gaatgtttac ttgtgaactc gatagagcaa ggaaaccaga 2400
   aaggtgtaat atttataggt tggtaaaata gattgttttt catggataat ttttaacttc 2460
   attatttctg tacttgtaca aactcaacac taactttttt ttttttaaaa aaaaaaaggt 2520
   actaagtatc ttcaatcagc tgttggtcaa gactaacttt cttttaaagg ttcatttgta 2580
   tgataaattc atatgtgtat atataatttt ttttgttttg tctagtgagt ttcaacattt 2640
   ttaaagtttt caaaaagcca tcggaatgtt aaattaatgt aaagggacag ctaatctaga 2700
   ccaaagaatg gtattttcac ttttctttgt aacattgaat ggtttgaagt actcaaaatc 2760
   tgttacgcta aacttttgat tctttaacac aattattttt aaacactggc attttccaaa 2820
   actgtggcag ctaacttttt aaaatctcaa atgacatgca gtgtgagtag aaggaagtca 2880
   acaatatgtg gggagagcac tcggttgtct ttacttttaa aagtaatact tggtgctaag 2940
   aatttcagga ttattgtatt tacgttcaaa tgaagatggc ttttgtactt cctgtggaca 3000
   tgtagtaatg tctatattgg ctcataaaac taacctgaaa aacaaataaa tgctttggaa 3060
   atgtttcagt tgctttagaa acattagtgc ctgcctggat ccccttagtt ttgaaatatt 3120
   tgccattgtt gtttaaatac ctatcactgt ggtagagctt gcattgatct tttccacaag 3180
   tattaaactg ccaaaatgtg aatatgcaaa gcctttctga atctataata atggtacttc 3240
   tactggggag agtgtaatat tttggactgc tgttttccat taatgaggag agcaacaggc 3300
   ccctgattat acagttccaa agtaataaga tgttaattgt aattcagcca gaaagtacat 3360
   gtctcccatt gggaggattt ggtgttaaat accaaactgc tagccctagt attatggaga 3420
   tgaacatgat gatgtaactt gtaatagcag aatagttaat gaatgaaact agttcttata 3480
   atttatcttt atttaaaagc ttagcctgcc ttaaaactag agatcaactt tctcagctgc 3540
   aaaagcttct agtctttcaa gaagttcata ctttatgaaa ttgcacagta agcatttatt 3600
   tttcagacca tttttgaaca tcactcctaa attaataaag tattcctctg ttgctttagt 3660
   atttattaca ataaaaaggg tttgaaatat agctgttctt tatgcataaa acacccagct 3720
   aggaccatta ctgccagaga aaaaaatcgt attgaatggc catttcccta cttataagat 3780
   gtctcaatct gaatttattt ggctacacta aagaatgcag tatatttagt tttccatttg 3840
   catgatgttt gtgtgctata gatgatattt taaattgaaa agtttgtttt aaattatttt 3900
   tacagtgaag actgttttca gctcttttta tattgtacat agtcttttat gtaatttact 3960
   ggcatatgtt ttgtagactg tttaatgact ggatatcttc cttcaacttt tgaaatacaa 4020
   aaccagtgtt ttttacttgt acactgtttt aaagtctatt aaaattgtca tttgactttt 4080
   ttctgttaaa aaaaaaaaaa aaaaaaa 4107
<210> 2
   <211> 3806
   <212> DNA
   <213> Mus musculus
<300>
   <308> NM_001159589.1
   <309> 2010-08-29
   <313> (1)..(3806)
<400> 2
   gccagtgccg cgcgtcgagc ggagcagagg aggcgagggc ggagggccag agaggcagtt 60
   ggaagatggc ggacgaggtg gcgctcgccc ttcaggccgc cggctcccct tccgcggcgg 120
   ccgccatgga ggccgcgtcg cagccggcgg acgagccgct ccgcaagagg ccccgccgag 180
   acgggcctgg cctcgggcgc agcccgggcg agccgagcgc agcagtggcg ccggcggccg 240
   cggggtgtga ggcggcgagc gccgcggccc cggcggcgct gtggcgggag gcggcagggg 300
   cggcggcgag cgcggagcgg gaggccccgg cgacggccgt ggccggggac ggagacaatg 360
   ggtccggcct gcggcgggag ccgagggcgg ctgacgactt cgacgacgac gagggcgagg 420
   aggaggacga ggcggcggcg gcagcggcgg cggcagcgat cggctaccga ggtccatata 480
   cttttgttca gcaacatctc atgattggca ccgatcctcg aacaattctt aaagatttat 540
   taccagaaac aattcctcca cctgagctgg atgatatgac gctgtggcag attgttatta 600
   atatcctttc agaaccacca aagcggaaaa aaagaaaaga tatcaataca attgaagatg 660
   ctgtgaagtt actgcaggag tgtaaaaaga taatagttct gactggagct ggggtttctg 720
   tctcctgtgg gattcctgac ttcagatcaa gagacggtat ctatgctcgc cttgcggtgg 780
   acttcccaga cctcccagac cctcaagcca tgtttgatat tgagtatttt agaaaagacc 840
   caagaccatt cttcaagttt gcaaaggaaa tatatcccgg acagttccag ccgtctctgt 900
   gtcacaaatt catagctttg tcagataagg aaggaaaact acttcgaaat tatactcaaa 960
   atatagatac cttggagcag gttgcaggaa tccaaaggat ccttcagtgt catggttcct 1020
   ttgcaacagc atcttgcctg atttgtaaat acaaagttga ttgtgaagct gttcgtggag 1080
   acatttttaa tcaggtagtt cctcggtgcc ctaggtgccc agctgatgag ccacttgcca 1140
   tcatgaagcc agagattgtc ttctttggtg aaaacttacc agaacagttt catagagcca 1200
   tgaagtatga caaagatgaa gttgacctcc tcattgttat tggatcttct ctgaaagtga 1260
   gaccagtagc actaattcca agttctatac cccatgaagt gcctcaaata ttaataaata 1320
   gggaaccttt gcctcatcta cattttgatg tagagctcct tggagactgc gatgttataa 1380
   ttaatgagtt gtgtcatagg ctaggtggtg aatatgccaa actttgttgt aaccctgtaa 1440
   agctttcaga aattactgaa aaacctccac gcccacaaaa ggaattggtt catttatcag 1500
   agttgccacc aacacctctt catatttcgg aagactcaag ttcacctgaa agaactgtac 1560
   cacaagactc ttctgtgatt gctacacttg tagaccaagc aacaaacaac aatgttaatg 1620
   atttagaagt atctgaatca agttgtgtgg aagaaaaacc acaagaagta cagactagta 1680
   ggaatgttga gaacattaat gtggaaaatc cagattttaa ggctgttggt tccagtactg 1740
   cagacaaaaa tgaaagaact tcagttgcag aaacagtgag aaaatgctgg cctaatagac 1800
   ttgcaaagga gcagattagt aagcggcttg agggtaatca atacctgttt gtaccaccaa 1860
   atcgttacat attccacggt gctgaggtat actcagactc tgaagatgac gtcttgtcct 1920
   ctagttcctg tggcagtaac agtgacagtg gcacatgcca gagtccaagt ttagaagaac 1980
   ccttggaaga tgaaagtgaa attgaagaat tctacaatgg cttggaagat gatacggaga 2040
   ggcccgaatg tgctggagga tctggatttg gagctgatgg aggggatcaa gaggttgtta 2100
   atgaagctat agctacaaga caggaattga cagatgtaaa ctatccatca gacaaatcat 2160
   aacactattg aagctgtccg gattcaggaa ttgctccacc agcattggga actttagcat 2220
   gtcaaaaaat gaatgtttac ttgtgaactt gaacaaggaa atctgaaaga tgtattattt 2280
   atagactgga aaatagattg tcttcttgga taatttctaa agttccatca tttctgtttg 2340
   tacttgtaca ttcaacactg ttggttgact tcatcttcct ttcaaggttc atttgtatga 2400
   tacattcgta tgtatgtata attttgtttt ttgcctaatg agtttcaacc ttttaaagtt 2460
   ttcaaaagcc attggaatgt taatgtaaag ggaacagctt atctagacca aagaatggta 2520
   tttcacactt ttttgtttgt aacattgaat agtttaaagc cctcaatttc tgttctgctg 2580
   aacttttatt tttaggacag ttaacttttt aaacactggc attttccaaa acttgtggca 2640
   gctaactttt taaaatcaca gatgacttgt aatgtgagga gtcagcaccg tgtctggagc 2700
   actcaaaact tggtgctcag tgtgtgaagc gtacttactg catcgttttt gtacttgctg 2760
   cagacgtggt aatgtccaaa caggcccctg agactaatct gataaatgat ttggaaatgt 2820
   gtttcagttg ttctagaaac aatagtgcct gtctatatag gtccccttag tttgaatatt 2880
   tgccattgtt taattaaata cctatcactg tggtagagcc tgcatagatc ttcaccacaa 2940
   atactgccaa gatgtgaata tgcaaagcct ttctgaatct aataatggta cttctactgg 3000
   ggagagtgta atattttgga ctgctgtttt tccattaatg aggaaagcaa taggcctctt 3060
   aattaaagtc ccaaagtcat aagataaatt gtagctcaac cagaaagtac actgttgcct 3120
   gttgaggatt tggtgtaatg tatcccaagg tgttagcctt gtattatgga gatgaataca 3180
   gatccaatag tcaaatgaaa ctagttctta gttatttaaa agcttagctt gccttaaaac 3240
   tagggatcaa ttttctcaac tgcagaaact tttagccttt caaacagttc acacctcaga 3300
   aagtcagtat ttattttaca gacttctttg gaacattgcc cccaaattta aatattcatg 3360
   tgggtttagt atttattaca aaaaaatgat ttgaaatata gctgttcttt atgcataaaa 3420
   tacccagtta ggaccattac tgccagagga gaaaagtatt aagtagctca tttccctacc 3480
   taaaagataa ctgaatttat ttggctacac taaagaatgc agtatattta gttttccatt 3540
   tgcatgatgt gtttgtgcta tagacaatat tttaaattga aaaatttgtt ttaaattatt 3600
   tttacagtga agactgtttt cagctctttt tatattgtac atagactttt atgtaatctg 3660
   gcatatgttt tgtagaccgt ttaatgactg gattatcttc ctccaacttt tgaaatacaa 3720
   aaacagtgtt ttatacttgt atcttgtttt aaagtcttat attaaaattg tcatttgact 3780
   tttttcccgt taaaaaaaaa aaaaaa 3806
<210> 3
   <211> 26984
   <212> DNA
   <213> Homo sapiens
<400> 3
   gtgtctgttt catgtggaat acctgacttc aggtcaaggg atggtattta tgctcgcctt 60
   gctgtagact tcccagatct tccagatcct caagcgatgt ttgatattga atatttcaga 120
   aaagatccaa gaccattctt caagtttgca aaggtactat gaactcttct ggttgtttct 180
   ttggccttct ctcatgaaaa agtattttgt tcacatacag ccaccttaag gttatcgttc 240
   attgtttagt aaagtgaatg ctgctactgt ggcggagtaa gatcactcat tatggctaga 300
   attcctttat tcctagagga ggactattat ctacttcatt ttaggagtga gcttattttc 360
   aaagagatag ttcatatttt taaaatttgc actgcagcga tggtcgttat tctgcctggg 420
   cttttttaag aggtttgcac accatataaa agtaacataa cttgtgattt ttaatatttt 480
   attagagatt gtaaaggtta taacatcact ttggtgtttc gtagtcaagt tttaacataa 540
   ggatgtgcct gaaaaatcat ttgtaattag agaatgggaa gattcttggg ttgcattttt 600
   gtcagcaaat tgcagaggat cattattctg ctctagagtt gcaccgtcca gttcagaagc 660
   cactagccac atgtggctgt tgagtacttg aaatgtattg atatgtgcaa taagtgtaga 720
   agacatattg gattttagag atccagtatg gaaaatacaa agtatttcat tagttttatt 780
   catcaaatat taaacaaata ttggttttta tatggaaaaa atacttaaaa ttaattttga 840
   attcttttga aatacttttg atattgggtt aaataaaatg tattttgggc tgtgcgtgtt 900
   gcctcatgcc tgtaatttca gcactttggg aggccaaggt gggaggatca gttgagccca 960
   ggagtttgag accagcctga gcaacataat gagactccat ctctacaaaa taaataataa 1020
   aatttgctga gcatggtggt gtgcacctgt agacccatcc actcagtagt ctgaggtggg 1080
   aagatccctt gagctcagga gctcaagact gagtgaacca tgatctgcca ttgcactcca 1140
   ccctgggcaa cagagcaaga gtgtttccaa aatatatgta tgttttttga agtttacttt 1200
   tttttttttt ctttttttga gaagtagtct tcctttgtcc cccaggctgg agtgcaatgg 1260
   catgatcttg gctcatagca acctctgcct cccaggttca agtgattctc ctgcctcagc 1320
   atcctgagta gctgggatta caggcgcccg ctaccatgcc cagctaattt ctgtattttt 1380
   agtagagaca gggtttcacc aggttggcca ggctggtgtt gaactcctga cttcaggtga 1440
   tccaccctcc tcggcctccc aaggctctgg gattataggc gtgagccacc gcgcctggcc 1500
   agttttcacc ttaatgtggt tactaaacaa tttaaagtta tatgtatgtc tcacattatg 1560
   tacctattgt acagctctgc tttagcatag aaagtttaat aatgcattac accattctca 1620
   agagattgag tcattagaaa actgttttct ttcctgtatt tcagtctttt ggaaagaaat 1680
   tacaaaattt gttattgtta aacttggagg tatatatatt tgaaagagtc attttatttt 1740
   attttacttt aagtgccggg atacatgtgc agaatgcgca gatttgttaa cataggtata 1800
   catgtgccat ggtggtttgc tgcatctatc aacctgtcat ctaggttgta agccccacat 1860
   tcattaggta tttgtcctaa tgcttttcca ccccttgccc cccacctcct gacaggcctc 1920
   tgtgtgtggt gttccccacc ctgtgtccat gtgttcttat tgttcaactc tcacttatga 1980
   gtgaggacat gtggtgtttg gttttctgtt cttgtgttag tttgctgagg atgatggctt 2040
   ccagcgaagg agtcttgtat tagtggcttt ttccccacct aatcgttaga agttgtgaat 2100
   agggacttct ttaatgaatc cagaagttaa tgaacctagc tttttttttt ttttttttgg 2160
   agacggagtc tggctctgtt tcccaggctg gagtgtagtg gtgtgatctc tgctcaccat 2220
   acaacctcca cttcccgagt tcaagagatt ctcctgcctc agccttccga gttgttggga 2280
   ctacaggcgc gtgctgccat gcctggctaa tttttgtatt tttggtagag acggggattc 2340
   actatgttgg ccaggttggt cttgaactcc tgaccttgtg gtccgcccat ctcagcctcc 2400
   caaagtgctg ggattacagg tgcgaggcac cgcgccccgc gccctgccga tgaacctaaa 2460
   ttttaactaa acagtggcct tatctacttt cagaccatgt ggtatattta catgactaca 2520
   ggagaagctt tgctaattca gaataaatta tgttacttaa attggcgact gtctttaaaa 2580
   aaaaagtgat tttttggatg cagtagctcg tgcctataat cccagtgctt tgggaagctg 2640
   agataggaag atctcttgaa ctccggagtt tgagaccagc ctgcgcaaca cagtgagacc 2700
   ctaatcacta cacccctcca ccccatgtaa cttttgtttt atccaggttg tggtatatta 2760
   aatgggcatt agtgtaaagt gggaaaatta tattaattct tgaatatgat tactaaactg 2820
   aatttgaaaa gttttcaggc tatcaagaga atttttaact taaaacttat ataatttgaa 2880
   ctttttactt catatatccg taatgatgat ggtcatctat atctagcttt tagagcagac 2940
   aactggttca tacctggatt aaataaataa tgtaaagtta ttttttgtta attatggatt 3000
   agtgaagatt tactgtttta acctactcct gctagtggca ctactgcatg gttttgaaga 3060
   ccagtgaagt atgacttaaa ggtttcttga attggagcta ggacactggc atttataaaa 3120
   tctccacgta gcgcagacat tgacactatc agaagaccag caagtaacta gaagttactt 3180
   tgatcttaaa tcaactacaa aaaacttgac tcactagtta tggagaatac attttttatt 3240
   gttaaactga aaaaaaattc agtcacttat aaggtgtagc ctcttctaat cctgtttata 3300
   taaatttatt tattattatt attttttgag atggagtctt gctctgttgc caggctgtag 3360
   tgcattggcg cgatctcggc tcactgcatc ctccacctcc cgggttcaag caattctctt 3420
   gcctcagcct cccaagtagc tgggactaca ggcacgtgcc atcacgatca gcacttttag 3480
   tagagacggg gtttcaccat gttggcaagg acgtgtctcg tcgtgacctc gtgatccgcc 3540
   cgtttcagcc tcccaaagtg ttgggattac aggtgtgagc cacaatgtcc agctgataaa 3600
   tttaattttg cttttctgag ttttcattta tggtaatggt taaatcagct aggctttaca 3660
   gttgttactc acatataatt ctttgtccat cctttaattc tcacattggg aactgactaa 3720
   aaaaagaaag cttccagtct gggcacagtg gctcacacct gtaatcccag cactttggga 3780
   ggccgaggtg ggtggatcac ctgaggtcag gagttcaaga ccagcctggc caacacagca 3840
   ataccccgtc tctaataaaa aatacaaaaa tcagccaggc ttgggtggcg ggcgcctgtc 3900
   gtcccagcta cgcaggaggc tgagacgggg agaattgctt gaagccagga ggtggaggtt 3960
   gcagtgagcc gaaagcgctt aaggagaaat aagtaaggac aaagagtgca ggatagtatc 4020
   ctgacacgga ggatggggag ggtatgtaaa tattgtcgat attttactga atttctaaat 4080
   attacaagaa tagaatattc ataggataac ctaagctttt ctgtgagtca gtgtactagg 4140
   cagaaagttc actaaagtgg tttatgattt ttgctactta tgagaaaaca ttaaaaatga 4200
   agccatgaaa aggaaatatt ggtgaaattt agatacttca gaaggaaaga gagactttct 4260
   tttttttttg gaaagaattt ctataccatc tattatatca ttgattgtat gtgattattg 4320
   agaattaact tgtttttttt ttttttgttt tttttttttg agacagtttt gctcttgtca 4380
   cccaggctgg agtgccgtgg tgagatctcg gctcactaca accttcacct cccgagttca 4440
   agcaattctg ctgcctcagt ctactgagta gctgggatta caggtgccca tcaccacgcc 4500
   cagctaattt ttctaatttt agtagagacg ggatttcacc atgttggtca ggctggtctc 4560
   gaactcctga cctcaggtga tccgcccgcc taggccaaag tgctgggatt acaggcgtga 4620
   gtctcccacg cccagccctt aaatttcatt tagactggaa atttcaagta atggagaggt 4680
   gtgttaagga agttattgct ctgttagagc agtttcttaa atttggcact attgacattt 4740
   tgggctggat ttttcttttt gtttctgaga tagtttcact ctgtcgagcc atcacagctc 4800
   gttgcagcct tgaactcctg ggctccttga acaggaggct cctgcctgag cctcctgagt 4860
   agctgagacc ataggcacgt accatatacc tagctaaatg tgtttttgac tttctttttt 4920
   ctttgtagag tcaagttctc actatgttgt ccaggctggt cttgaaatcc tgggttccag 4980
   caattctcct gcttagcctt acagagtgtc gggattgtag gcataggcca ctgcacccgg 5040
   ccttgggctg atatttattt gctatgggga actttcctgt gcgttgtagg atgtttggca 5100
   atgtccctgg ccggccaggt gcagcggctc acacctgtaa tcccagcact ttgggaggct 5160
   gaggtgggcg gatcacttga ggctgggagt tcagaagaga ccagccaaca tggtgaaacc 5220
   ctgtctctac taaaaataca aaaattagcc gggtgtggtg acacacatga gaatcacttg 5280
   aactcaggag gctgagtttg ccgtgagctg agatcgcgct gctgtactcc agcctgggtg 5340
   acaaagcaag aatctgtctc aaaaaaaaaa aaaaaaaaaa aaaaaattcg ctggtctcca 5400
   cctacgtttg tatccccacc ccaagtcctg acaataaaaa atgtcttcaa aatgttcctt 5460
   gcaaaattgc tcattgaggc taactgaggt ggctcacaac tttaatccca gccctttgga 5520
   aggctgaggc tggcagatca cttaagccca ggtgactggc cctggccaac ttggcaaaac 5580
   cctgtctcta ctaagaatac aaaaaatagc cagacgtggt ggtgcacacc tgtaattcca 5640
   gctactcggg aggctgaggc agcagaatca cttgaacctg ggaggcggag gttgcagtga 5700
   gccgagatcg caccactcta ctccagcctg attgacagag tgagactcca tctcaaaaaa 5760
   aaaagaaatt actctctgag caccagtgtg ttacagtgtg cttagctgtg gtaccacttt 5820
   ggattctttc acacaagaac agttaaggcc agttttaaag ctgtggggcc atttctgaac 5880
   tgtattttgt tttttttttt ttggagatga agtttcactc ttgttgctca ggctggagtg 5940
   cgatggtgtg atctcggctc acttgcaacc tccgcctccc aggttcaagc aatctcctgc 6000
   cttagcctct tgagtagact tgattatggg atcttggctc actgcaacct ctgcttccca 6060
   ggttcaaaca gttctgcctt agcctgctga gtagctggga ttacaggtgc ccgccaccat 6120
   gcttggctaa ttttgtattt ttagtagaga cggggtttca ccatattggc caggctggtc 6180
   tcgaactctt gaccttgtga ttcacccacc tctgcctccc aaagtgctgg tattacaggc 6240
   atgagccact gtgcctagcc aattttttga atttttagta gagatggggt ttcatcatgt 6300
   tggccacgct ggtctcgaac tcctgacctc aggtgatcca cccaccttgg cctcctaaag 6360
   ttctgggatt acaggcgtga gccacttcgc ccagcctgca ctgtgttctt atcctgcata 6420
   atgacttaaa ttgataataa gttgtggtca tggttctcag aaacctaaaa taattcactg 6480
   atgttaataa aaatgagtgg ttgtatttag agtcaaggcg aaactgaatt aggatgtata 6540
   aaataacttt tttttttttt tttttttttt aagtaggact gttgttcggc tgggcgtggt 6600
   ggctcacgcc tgtaatccca gcactttggg aggccacagc gggcggtcaa aagtttgaga 6660
   ccagcctgac caacatggtg aaaccctgtc tctactaaaa atacaaaaat tagatggatg 6720
   tggtggcgca tgctgtaatc ccagctactc aggaggctga agcaggagaa tcacttgaac 6780
   ccaggaggcg gaggttgcag tgagctgaga tggcaccact gcactccagc ctgggcgaga 6840
   gagtgagact ctgtctcaaa aaaaaaaaaa aagaaaagaa aaaaaaagaa aagggactat 6900
   actatgttcg aagagtgtag tttctgtccc atactgaatt cactgttctt tccctagtga 6960
   ttaaaataca agaaaaggta gagggagaaa cacgaactga attttatgaa cttaattcca 7020
   agaatgtatt tttacatttg ttcaaaaaat tattacttta ttgataatat gcaaacatac 7080
   cagattatgt attaatagtt aaaaatttga gttctagaac aagcttatta aaaatcaacg 7140
   tcagtaaaaa gaaaatagat ggtagtcaca gtgccattca tctgaaaata atgttttaat 7200
   actcaacatg tatatggatt atatatctat acgtttatag tattgtagct ggatttatgt 7260
   tacattttta gcataaaatt tggcttttgc tgggcccagt ggcttgcgac tgtagtccca 7320
   gcaactcagg agggtaaggt gggaggattg cttgaaccaa agagtttgag gctgcagtga 7380
   gctgtgacct cacctctgca caccagcctg agtgacagag caagacccat ccctgaaaag 7440
   aaaaaacttg gctgttttac tttgctttca atttgtagta tattctactt aatataataa 7500
   acttactttc tgtgaggtgc ttgaatactt tttaatggtt atttatgaag tggtgaactg 7560
   taattctgta tttgtacatt tggatgtaca aatagtgttg ctacagttgt ccttttttgg 7620
   tatgtctcca gtgttcttag gataaattcc tgaaagtgaa tttcagtttt gaatttcaga 7680
   gaaacatgtc aaagttcttc tttaagcctt taagtctcct gatatgtttt gtcgtattgc 7740
   agcttggaaa ggttgaaata aaatcctcta ttagggctgg gtgcagtggc tcatgcctgt 7800
   aatccgagca ctttgggatt tagctgattt ggctgattta tcatgccact gccacctgta 7860
   tccacccaaa atccacaaaa accccaggat acattcttag cattaaaaaa aaagttatat 7920
   aaatatatat aattatatat atatttgaga cagggcctgt ctctgatacc cagacgtgtg 7980
   cagtatcttt caaaaatcat atatctatat atagtttgcc ccttccttca gtctttgctg 8040
   cccctaatat ctgtccttaa tggttaccat ggtgaaactc ttggaaatca ttcaggggga 8100
   aaaaaaattt gtgttgggat aggggaggga tgtcagcata agtttttaat acttgataaa 8160
   tgattgccaa cagaatcaaa aacaccttgt tttctatctt ttttcaccat ataatattct 8220
   tgagaataat ttcgtaacag cacttaacag atatacctta tttttcaaag aaggctgtgt 8280
   agcattctat tttaaagttg tttcacagta tatgagtctc ctattgatag gtatctgcat 8340
   ttttgtcttt ttgatctagg gttgttttta aggcagttta aaaactatag taagtaaaag 8400
   aggccaggtg cggtggctca cacctgtaaa cccagcactt tgggaggccg aggcgggtgg 8460
   atcacttgag gtaggagttt gagaccagcc tggcaaacat ggtgaaaccc ttctctacta 8520
   aaaatacaaa aaattagccg gccgtggtgg caagcgcctg tagtcccagc tgttcggaag 8580
   gctgagggag gagaatcgct tgaactcggg aggcggagat tgcagtgagc caataatatg 8640
   ctgctgtact ccagcctggg caacagagcg agactctatc tcaaaaataa atacaatttt 8700
   aaataaaatt ataaaaataa agcagcaccc agctttattt tttatttatt tatttatttt 8760
   ttgagacaga gtcgcattct gtcgctcaag ttggagtgca gtggtgccat cttggctcac 8820
   tgcaacctct gcctcccggg ttcaagtgat tctcccgctt cagcctccca ggtagctgag 8880
   attacaggtg tgtgccacca cgcccagcta atttgtattt ttagtagaga tgggggtttc 8940
   accatgtttg ccaggctggt cttgaactcc taacctcagg tgatccgccc accttggcct 9000
   cccaaagtgc tgggtttata ggtgtgagcc gccgcgcttg gctcaacatc ttattgtttg 9060
   agacggggtc tccttctgtt atccaagctg gagtgtagta gtgtgatctt acagctcact 9120
   gcagcctcca actcttgggc tcaaatgatt ctcctgtctc agcctcccaa gtagctagta 9180
   ctacaggtgt gcaccaccat gccctgctgt ttctttgttt tttttatatg gacagtcttg 9240
   ctatgttgcc caggctggtc tcaaactcct gagctcgagt gattctgctg cctcagctac 9300
   tcaaagtgct gggattgtag gcgttaacta tcaggttagg cctgttctgt ttttattagc 9360
   gtggatgact gctggttact actggccgtt tgtatatcct tgaattacct ctttgttttt 9420
   agccattttt ctccatttgg gtgctttttc acagtgactt ctgggaatat tatgttcttt 9480
   tcttttgatt tacctttcaa attttatttt tatggatata taatagttgt acgtgtacat 9540
   tatatattct tgaagagtta ggcttattgg gattccagcc tttagtagtc ccatgaaggt 9600
   gagcaaactg aaatgtactt actagtctta gtcatttggt tcgtaatatg taatagaaat 9660
   ggtgcttatc ctaaaagaaa aatagaaaac ttgagggtga aggatcagaa attgaagaca 9720
   gtaaatactg catagaggtt taaatgctag ggtttttagg ttttcttttt tttttgagat 9780
   agtcttgctc tgacacccag gctggactgc aacctctgtt ccctggcctc aagcgatcct 9840
   cccaccttag cctcctgact agctgtgacc acagatgtgc accaccatgc ccggctattt 9900
   ttttttgtat ttttggtaga gatggggtct cgccatgttg cccagtccag tgctggattt 9960
   tcatatcaaa acagggctct gctacttact tgctcttgtg accaaaatgg caagttaggt 10020
   ttgttttgta tttgttttca tctgcatcgg ggtatatgtt cacagctgga tcagcaaact 10080
   ttctgtagta aggtagggcc tggtagtaaa tatgtagtat gctttgcaga ctgcataggg 10140
   tctctgtcat atgttctttg ttttctttac agccttgaac aaattaaaaa acccagtctt 10200
   cagccttgtc tgtagcataa taaagaggaa attaaaagtg cttatgttat acgtggtaca 10260
   tactaaaatt tattaagtgg cgactggcag tttaactaat tgacattctc ttgtgttagg 10320
   ttaatacagg ggttggcgta ctatggcctc agtccaaatg tggcccactc catgtttttg 10380
   tataggctgt gagctaagca ttaagcatta atttattact taaatcaaca aataaagttt 10440
   atgtggtgta caacatgatg ttttgatgtg tgtacattat ggaatggcta actcaagcta 10500
   atattcatta cttcacatac ttttttttgg tggtgagaac atttaaaatg tactcttaat 10560
   aattttcagg tatacaatat ttgtttttgt ttttgttttt gttttgagac gaagtctcgc 10620
   tcttgtcccc aggctggagt gcagtggcgc tatcttggct cactgcaacc tccgcctccc 10680
   aggtacaagc gattctcctg cctcagcctc ccaagtagct gggattacag gcgcctgtct 10740
   ccacgcccgg ctagttattg tatttttagt aaagacaggg tttcaccatg ttggccaggc 10800
   tggtctcaaa ctcctgacct caggtgatcc gcctgcctca gcctcccaaa gtgctgggat 10860
   tacaggcgtg agccaccgcg cccggctaca atatgttctt aattatagtc agcaatagat 10920
   ctcctgaact tattcttccc agcgtcttcc caatcccacc tccaccccct gctaataatt 10980
   aattttaaat gactggggaa aaaattgaaa gaactgtttc atgacacttg aacattatat 11040
   aaaattttca tgtcagtgtt cataaataca aacttttatt agaacatagc catactattt 11100
   atatattgaa atacagttga atttgtcata tgaccaattt attagtattg attaagatgt 11160
   tgagcagttt attcaagtgt ggttttgtgg aatgaaagag acaggaggga ttatgaggac 11220
   tctccttagc aggattgatg catttcactt tttttgacag gaaggaaagt ttaggttcta 11280
   ttttctcagc aactctactt aactgcctcc atctgaacta tatataggag ggggtggata 11340
   tcagcaaaga ttaacatcag taaattttga tgaattctct aaaaattctg atttttaaag 11400
   agggaatttt ggctgggcac agtggctcac acctgtaatc ctaagcactt tgggaggcca 11460
   aggcaggtgg atcacctgag gtctggagtt tgagaccagc ctggccaaca tggtgaaacc 11520
   ctgtctctac taaaaacaaa ttagctgaat gtggtggcgc gcacctgtag tcccagctcc 11580
   ttgggaggct gaggtgggag aattgcctaa acccaggaga ggtggaggtt gcagtgagcc 11640
   gagattgtgc cattgcactc catcctgggt gacagagcga gactctcaaa aaaaaaggtg 11700
   ggtgtgaatt ttgttatgtt gtgcgggctg gagtgtagtg cccatccatt ggcacgatca 11760
   tagagtacta cagctcccta actcccagac tcaagtgatt ctccctcctc tgtctcccca 11820
   gtagcaggaa ctacaggcct gtgccaccat gcccagcttt cttagtcttg agggactgca 11880
   tttacaccta tgccttttac tctagggtct tttattcata ttgctagaaa tttggagaag 11940
   aaagaaaggc ataatctctg cagaaaagcc attatttctg cagatagttc tttttttttt 12000
   tgagatggag tctttctttg tcacccaggc tggagtgcag tggtgtgatc taggctcact 12060
   acaagctcca cctcctgggt tcacgccatt ctcctgcctt agcctcccga gtagtgggac 12120
   tacaggcacc cgccaccatg cccagctaat ttttttttgt attttttagt agagacgggg 12180
   tttcaccatc ttagccagga tggtctcgat cttctgacct tgtgatccgc cgacctcggc 12240
   cacccaaagt gctgggatta caggcatgag ccaccacatc cggccaattt ctgcggatag 12300
   ttctaagctt tagttttgac cacgttggtt gcctatggcc aattcctgga ttatttgcat 12360
   gtttccagac acagctatgt gaaagcaggg tttgttttaa tagtgttttg agcctttgat 12420
   tccagttctg cttccactct tggtctataa ggatccctat ggcagcaaca gaatggccca 12480
   gccctcatga ttggttgctt aaaaaggctt taggatattc aaactgggtg gcctgtcttg 12540
   tttggaatag gactaagcat gtcacacttt caccatggaa gagttaatcc atattctatc 12600
   tgtaagttgt atctccagtc attggttttc aacaagtggt cctatcttag gaggagtggt 12660
   atattagaac agtccttcca gagtaatttt tttcattatt tcagattttt tagttttttt 12720
   tgtttgtttt tttttttttt ttttttaaag gctctcactc tgtcacccag gctggagagc 12780
   agtgatacga tcatagctca ctggaacctc tacctccctg ggctcaggtg atcctcccac 12840
   ctcagccttc tgagtttctg ggactacagg catgaaccgt gaggcccagc taatttttgt 12900
   gatttttgtg ttttttgtag agtcagggtt tctccatgtt gcccaggctg gtctcgtact 12960
   cctggtctca agtaacctgc ccgcctcggc cttccaaagt gctgggatta gaggtgtgag 13020
   ccaccacacc tgtactacta ctttagtttg aacactatat ttaaatctaa taattcagac 13080
   atggcccatt tcatacttga cagaaagtat gagtcaaaga ctcaaatcat tcagattctc 13140
   acatatgtaa aacgtacgct tggccgggtg cagtggctca cgcctgtaat cccagcactt 13200
   tgtgagacca aggtgggcgg atcacctgag gtcaggagtt caagaccagc ctgaccaaca 13260
   tggagaaacc ccgtctctac taaaaataca aaattagctg gggtggtggc acatgcctgt 13320
   aatcccagct attcaggaag ctgaggcagg agaatcactt gaacccggga ggtggaggtt 13380
   gtggtgagct gagatcgcgc cattgcactc cagcctgggc aaaaagagcg aaactctgtc 13440
   tcaaaaaaaa aaaaaaaagc aggcttataa aataaaacaa aaatgtccct tatgatttgt 13500
   cttaaaggta gttgaattaa taagagctaa atctagtgtc ccatacatct ttgtgtttct 13560
   tactctaccc tttagaaagg gtgggggtgg acgtggataa ggcacacagt taattcaggg 13620
   acggtggagt cttggtactt gctctttttc cctgaccttg ctttggagtt attttatttt 13680
   ttaaattctg catttggcat gctattatca cttgcttact attataggtc ctattttatc 13740
   tgttgcctag catattctca tagtgcctaa aatactctaa aagctatttc cagagggagc 13800
   tttaatgaga catacaatgc ttaatctctc aaatgtaaat gtactagaac aaagcattct 13860
   ggggaagaaa acagtggttg atttaactga actcactttt ccaaaacagt ggcttttgat 13920
   tttctgtaaa cattgacttt attaagcttt tcccttgcta ctatatgcac tctctatttc 13980
   ttaaattgca aagagttatt catgaaattt gtgataaata tttgaatcta aggcaacgtt 14040
   tttggattct ttatacgagg tttttagcag catgtgtaca tgtatgtgat agctgttaac 14100
   ggaattcact taagttttac ttagaagcct gggtagtaga acctagaagt tgcagtggga 14160
   attttttttt aatttgaaag attaaatagg taaaaagtca tctttaatct ctattaaact 14220
   tttttgtata atgtcagaag tgaaagttcc ccacatctgc ttacagcctg tttgtatcct 14280
   tcctgacttg attgattaaa tggacatctc tatatacata tgcatataat ttcaattttt 14340
   gcttagtaac aaaaacaaaa gttacacata ctggtgacta atttgctttt ttcacgtatg 14400
   tcatgaacca ttttccaggg cagttcatct agatctccct caattctttt taatggctac 14460
   atattccact attatggatg taccatattt atacaaccgg ttccctattg atggacatat 14520
   ggttgtgtct agttttttgc tagcattgtg atagattaga gcctgtaatg aatggtaagg 14580
   aagtaagtag atgatagcac tactactttg aaggaaaaag tagagaataa acttcctttg 14640
   ccatagtcac ttactaaatg gaatttaata aaaacactgt caaaagttgg gaggaccaaa 14700
   attgatactt tttctctgat ctttttgcca tgtgtatatc tgaattcttt gtttttaaag 14760
   aagaaacagc attgaagcat tatttggggg gaaaaacaca cacacaaaat ccagcaactc 14820
   agcattcatg agcaactcta tactatacca gtatgtgcct gtgcagtgga aggaaaacaa 14880
   ttttggtaag gattaaaact ttagctttaa acttccagca ggttgatatt ctaatgaatg 14940
   ataaatcaaa aaaaaatttt aaatattctt gtattgacag tgcttttttt tttaaatcac 15000
   cctaccttga tatctgtaat tttagtttaa actttcccat ttttctttaa agaaaaaaat 15060
   ctgaatttgc agccaacaaa aattagatat cctaaggttg tattacttct gacttttatt 15120
   aaaatatcaa catttcttag agatgtttat gggccgactt tgtctttttc ttcaaggggc 15180
   caagttcact aattgctgag ttttatgcat atgacagcaa ccgtcctttt gtaggtgtgt 15240
   gtcgcatcca tctagatact ttaaaatgct catctatttc atttttaaaa ttatgtgtgt 15300
   gggattatca gtattttttt gttaaacata tgacatctgt agtttatttc actaatgtaa 15360
   attttttcta ccatttgctt gatacaggaa atatatcctg gacaattcca gccatctctc 15420
   tgtcacaaat tcatagcctt gtcagataag gaaggaaaac tacttcgcaa ctatacccag 15480
   aacatagaca cgctggaaca ggttgcggga atccaaagga taattcagtg tcatggttag 15540
   taaacttcag agtggttttc tgtaatttat tttagtttta taggaagata tttcctataa 15600
   agctgactgc catcgagaag tggagataaa gcattattta atcatgttat ctcatttatc 15660
   gataacctca gaaaagtaga aaacaaaaat aataaaaaca gaagtattgg ccttgacagt 15720
   taattataga aaacctcaga tattaatttg cttttgattt caaaagatgc tgcagttgca 15780
   tgcattcaat tattttaaat aatcttttct ggctgagtgc agtggctcac gcctgtaatc 15840
   ctagcacttt gggaagccga ggcgggcaga tcccatgaag ccaagagttc gagaacagct 15900
   tggccaacat ggtgaaatcc caactctact agaaatacta aaattagccg ggcatggtgg 15960
   caggcgcctg taatcccagc tactcaggag gctgaggcac gagaatcact tgaacctggg 16020
   agttggaggt tgcagcgagc caagattgca ccattgcgct ccagcctggg agacagtgag 16080
   actctgtcaa aaaaaaaaaa aaaaaagaaa aagaaattct gaatacaaga gtagtattag 16140
   ctgttaatga agaaatgtga catctttagt ttatgaaact aaaagaactg gatagttgag 16200
   atgtacagga ttcagagatt cagaaatgtt taaaacaagt atcaacaggc cattaggtat 16260
   ctaaagtgtt tctaagaact gccgagctaa ggtaatagag ttggaactgt ccaactctgc 16320
   aggattttag ccctggagga gtgagctgtt acagttttgt tgaaaagagt agctttagaa 16380
   ggcatcatta tattagtgtc tcagagattg agaatcatat tcattctatg tataaatata 16440
   taaattcttc atcccctaga ttctcctgta gtatatcaca aaatctgcag tgtgttctga 16500
   ggtttaaaat caaagttaaa aacaaaaaca aaaatcctta aaccctctta acatttgtga 16560
   tgttaaactt tataacgttt gtggtgtgtt caagaaacag aaatacttct ttaataaagc 16620
   atatatatgt tgtttgtttt taggttcctt tgcaacagca tcttgcctga tttgtaaata 16680
   caaagttgac tgtgaagctg tacgaggaga tatttttaat caggtaattt gttgcccata 16740
   ttttaggaat tgttcatgtc tctgaagtat ttcttctttt gcctcaaaat ccttttttac 16800
   ccctttaaag tatatatggt acagaaagat tcaggaagaa aatagttagc atttgggaat 16860
   tttggtaaaa tacacgagaa cctttcaata ccttatatag aaaacagccc tataaaggct 16920
   cttcaaatta tgaaattggc ttcttagtat ctaagccgaa cataaaatgt cagatcataa 16980
   gcaagttgtt gttgtagtag tttttctcct tcctctccct ttttgtgcct gtcagatttg 17040
   gaccttaaat tagttatggt aatctaaaat tatttcatac atgttcattg ccaaaaaata 17100
   taggaaaatt tggcagaaaa taaaaattat ccagaaatat gtcctgtgtg cttagcaata 17160
   tatggaggta atcatgtcat tacgaaagag gaagaatagt atattaaaat ggagtcattg 17220
   gtcgggtgca gtggctcatg cttgtaatcc cagcactttg ggaggacgag gtgggtcgat 17280
   catttgaggt cagaagtgag accagcctgg ccaacatggc aaaaccccgt ctctcttaaa 17340
   aatacaaaaa ttagcctggc atggtggcat atgcctttaa tctcagctgc ttgggaggct 17400
   gaggcaggag aattatttga actcgggagg tggaggttgt agtgagctga gatcatgcca 17460
   cttcactcca ggctgggcaa cagaacaaga ctccatctca aataaataaa atggaatcat 17520
   tttatttgac aaatgtctat ttttttttga aaggatcaat tcccatttag tgtatataca 17580
   cacattatgt ttttcctctt aatatatgat ggactcttca atgtcaagtg tttttctaca 17640
   tagatattat aacaagagca tagtatttca ttgtttcata gtatgttaat aaactaccca 17700
   tcattgacag ttgctgttta tccatagctt ttctgttttg tttttttttt aataattctg 17760
   aaatgtattc ttagaggtat ggaaatgttg actatttcta acttgggctt actctttgct 17820
   tctctacctc aaccaaaatc tgaaaatatg taggtagttc ctcgatgtcc taggtgccca 17880
   gctgatgaac cgcttgctat catgaaacca gagattgtgt tttttggtga aaatttacca 17940
   gaacagtttc atagagccat gaagtatgac aaagatgaag ttgacctcct cattgttatt 18000
   gggtcttccc tcaaagtaag accagtagca ctaattccaa gtaagttggt gatggttttt 18060
   ggagaacatt tctatatata atgtcatggg ttgtgggtct gtataataga cgctagtaat 18120
   cttaactctg cttctgtttg aaagagtggt gaagagctaa ttttagaaat tgtttgtttg 18180
   tttgtttatt gagatggagt ttccctcttg ttgcccaggc tggagtgcag tcgcatatct 18240
   tggctcactg caacctccgc ctcccgggtt caagcgattc ttctgcctca gccttctgag 18300
   tagctgggat cacaggcgtc caccaacatg cctggctagt ttttgtattt ttagtataga 18360
   ccgggtttca ccatgttggc caggctggtc tcgaactcct cagctcaggt gatccgcctg 18420
   tctctgcctt ctaaagtgct gggattacag gcatgagccc ccatgcctgg ccagaaattc 18480
   tttattttta gtagagatga ggtcttgttg tgttgcccag gctggattcg aactcctggg 18540
   ctcaagtgat cctcctgcct cagccccttg agtagctagg attacaggca cgagcctcca 18600
   catctggctg aactgttttt ttaggtggca ttgttcattg agactggtga atctgacatt 18660
   ttgatggggg gtggagggtt gtcaaaacgc aagtaatgtt ggtggctcgt gcctgtaatc 18720
   tcagcacttt gggaggccga ggtaggtgga tcgcttgagg gcagcagttt gagaccagcc 18780
   tggccaacat ggtgaaaccc tgtctctact aaaaatacaa aaattagctg ggcatagtgg 18840
   tgtgcacatg tagtcccagc tacttgggag gttggggcat gagaatcgct tgaactcagg 18900
   aggcagaggt tgcaatgagc cgagaatgtg ccactgcact ccagcctggg tgacagagtg 18960
   ggactctgtc tcaaaaaatc aaaaacaaac ccagaagtaa tgctaaactc tacttctaat 19020
   ttatgtgaaa aattaagaca aaggtagagt tcaacttaga ctttttggtg gaatgtgttt 19080
   ttaatgttgc aagggttagc atcaattaat ttatgaaatg gtcctttatc cttatatttt 19140
   ttgattacag ttttgatctt tcaaatttaa ttgatttcac atttaataaa ttcaaatgta 19200
   tagtttggta gaagtgtaac ataccatgta agatggaatt tgggggctca gaatgattgg 19260
   ttcattcttg tagtaacagc catgccataa cggtgatatg tcgatatagt ttacccaaaa 19320
   gggtgtgtat ataaaagtgt tcacataaaa tttaaatcct tattactctc aggaaaattt 19380
   ctttgtcata tatattctca tactgtctgt ttcctcctct agtttagaat cagaggagga 19440
   gagagagaga tttcagttgc atcagatgtg tcctttcata agtgagggaa agaggagagg 19500
   aaaaatatta aatccctagc cagagaattg aattagaccc cacatcagca gtcaaggcag 19560
   ccagagtaaa cagttggaag aaacatggag tcaagctctt caattttgtg tctatcccat 19620
   atctcgtggc tttagcagtt aactcagtct tacctaatta gctgtgaaat tctgttaaag 19680
   tagaatacaa gacaatttgc aattaaatga aacatgaaaa tttaatatac aaatctcttt 19740
   gttcttgagt tgctcttctt aaagcaaaat caaaggcact ctgcttaaaa gcttttcttt 19800
   ttatttttta gagacgggtg tcttgctgta gctcaggcta gagtgcagtg gtgcagtttc 19860
   agctcactgc agccttcaac tcctggggac aagcagtcct tccacttcag cttcctgagt 19920
   agttaggacc agaggcgcac accatgcttg gcaaattttt aaattttttt ttgtagaaac 19980
   gggattcact ttgttgccca ggctggtcac aaactcctgg cctcaagtga tccttcctcc 20040
   tctgcctctc aaagcattag gattacaggc atgagctacc atgcctggtc cttaaaaact 20100
   tttctttcaa aagctttctg tgggatagta tgatgtgttg caaagataat taaaaaaaga 20160
   aacaggttta taatagcgta gaatatgttt atatgacttt ttccctcccc ccctcccctc 20220
   cctccctccc ttccgtttgt ccttccttcc attcatcctt ccttctgtta gtccttcctt 20280
   ctgtccatcc ttcccgtccg ttcttccttc cttccttcgt ccatccttct gtctgtcctt 20340
   ccttccgttc atctgtccgt ccttccttcc tcccaccctc cctccctccc tcctatcctc 20400
   cctccctccc tcattgccca ggctggagtg caatggcatg atctcggctc actgcagtct 20460
   cccgtgttca agcagttctc ctcccaagta gttgggatta gaggcatgcg ctaccacacg 20520
   cccggctaat tttgtgtttt tagtagagat ggggtttcac catgttggtc gggttggtct 20580
   tgaactcctg acctcaggtg atccgcctgc ctcggcctcc caaagtgctg ggattacagg 20640
   cgtgagccac tgtacccagc tcaccttctt tatatcagga gctacttaag tagaacattt 20700
   atgtaccaag aactcttctg tgcacttgac taactcattt aatcctcacc acagtcttgt 20760
   ggagaagtac tattatcatc cccattctgc agacaaggaa atttgaggtt cagagtggga 20820
   aagtctggga agattgctca ggggtaacca ggtgatagga gcagaacttg agcgttttat 20880
   aaaagacact taactgccca tctgcttgct tgatgaaatg taatggcttg gttaagtatt 20940
   tagtgcatgg gtcttttttg ggaatttgga gctcaagccc ttgttggatt tttgcataat 21000
   gtatctgttg tggttttatt agcttacttc ctcctccctt tttctaactc ttatttttca 21060
   ccctatttta ggttccatac cccatgaagt gcctcagata ttaattaata gagaaccttt 21120
   gcctcatctg cattttgatg tagagcttct tggagactgt gatgtcataa ttaatgaatt 21180
   gtgtcatagg ttaggtggtg aatatgccaa actttgctgt aaccctgtaa agctttcaga 21240
   aattactgaa aaacctccac gaacacaaaa agaattggct tatttgtcag agttgccacc 21300
   cacacctctt catgtttcag aagactcaag ttcaccagaa agaacttcac caccagattc 21360
   ttcagtgatt gtcacacttt tagaccaagc agctaagagt aatgatgatt tagatgtgtc 21420
   tgaatcaaaa ggttgtatgg aagaaaaacc acaggaagta caaacttcta ggaatgttga 21480
   aagtattgct gaacagatgg aaaatccgga tttgaagaat gttggttcta gtactgggga 21540
   gaaaaatgaa agaacttcag tggctggaac agtgagaaaa tgctggccta atagagtggc 21600
   aaaggagcag attagtaggc ggcttgatgg taagaaaggc agtcggacca ttttgaaagt 21660
   ataaatgtca taacagtatt tccaaaaaat tagctatttc ggcaggttaa tcgatagggt 21720
   agctttatgt agttgattct gtttagagaa actgtacagt tcgtaatcag aaaggtaaat 21780
   cttctggtat cttaacatga tatggagaag gaagtgttta atagtgctct gtatgttgtg 21840
   tttctctagg ggatggaaaa ataagaatgg gttattagct ggcagaaatg atgacagatt 21900
   tgagtgctta ctgtaggtca gctgctttac atatgttacc tgattttaat ctgaggtagg 21960
   aactattact cttcttcagg tgggaaaact caggaccata gaggttaaat acctcatgca 22020
   cagtaactac taagaagtgg aagaaccaga ttaaaatcca gtctatttat gcttagagcc 22080
   tgccccctta actactatgc ggtgttgtct cagatgtaga acacatcttt gttttctctg 22140
   aggacttaga aaaaacacgc gtgattcttc tccactgaat ataggaatag tttctaccct 22200
   gtttagaaca tgcatagtct ttatcaataa gtgttaactg acatgtgaaa tcactttcta 22260
   ctctttggaa gccgatgttt taagtccaag tagtctgtat ggtggaaatt cctaacttat 22320
   gttaaaaaaa agaaaaccac tgtaacttag ctataacagt cttatataac ttagaacttt 22380
   aagtctcatt ccatttacca tacttggcga aaactgacaa attttagaca actgtatttg 22440
   agtattccag tcatttgctg taaatacatt ttagttcagt tgaataaacc tttggccttt 22500
   ttcatattag gcactgtggt aagttatgga gacacagtga ttaatgttgt gtataattat 22560
   aaatgtgagt gatactcatt aatactttgg tataggtgct gctgaagaac aaagtacttt 22620
   attattgttt tgaacaaaac atcacacaaa aggtagattt tttttttttt tttttttttt 22680
   tttttttgtg acggagtctc actctgtcgc ccaggctgga gtgcagtggc acgatctcag 22740
   ctcactgcaa cctctgcgtc ccaggttcaa gagattctcc tgcctcagcc ttctgagtag 22800
   ctgaaattac aggcatgcgc caccttgctc ggctgattct tgtattttta gtagagacgg 22860
   ggtttcgcca tgttggtcag gctggtctca aactcctgac cccatgatcc atccacctcg 22920
   gcctcccaaa gtgctggaat tacaggcgtg agccacggcg cccagcccca aaaggtagat 22980
   tctacttgga gattagatta cagaaggctt tctaaggagc aaaatattta acaataagta 23040
   gggatttaaa aagagcagac gtgttcacag gggaaactag aaaaagcata gagggatgct 23100
   tacgtttgca aatcgtggca gaagtcagaa agtagaaaaa ttgctactga cttagataca 23160
   cagttgtctc tagcgtatac aaagtctacc tatgcactaa gacttgcact gggaactttt 23220
   cttgaatgtc attttgacag atgtgtcaaa cggacaatct ctttgcccag gaaacagagt 23280
   taacctggaa cagtcatttt tttttaaatt tatttatttt ttgagacgga gttttgctct 23340
   tgttgtccag gctgaagtac aatggtgtga tctcggttca cggcaacctc cgcctcccgg 23400
   gttcaagcga ttctcctgcc tcactctcca gagtagctgt agctgagacc acaggcgcat 23460
   gccctcatgc tcagctattt tttttttttt tttttttagt ttttgtagaa acaaggtctt 23520
   gccacattgc ccaggctgat accaaactcc tgggctcaag cagtctgcca gccttggcct 23580
   cccaaagtgc tgggattaca ggtgtaagcc actgtgccct gcctgtggtg tcttgggaaa 23640
   ctcatgagta ctatgtgtct gttgtaatag agggaaataa gtggttttca cagtgatttg 23700
   tagtggactg tgaaatttta gggattcagg tcagagttgt cacacaggtt gtagtcaggg 23760
   tgagaactgg gtcatgatgc agtatgaaaa agttcgagag ccactttgga gagaacttga 23820
   gataggccac ctaccagtgt ggtaaccagg cttttgagaa ttcgtctggg atacggtaca 23880
   ataaatacta catctattat gtgtgaagag atgttaagtt agggacatac tgtgaattca 23940
   aggatagaaa acttttccat cagtttttag ggatcctact ctttcactta aaccccaaat 24000
   ggccaagcta ggattgattt ggtgtgctgt agaaagaact tcattggtat tcatggattc 24060
   acattacatc ttagaggagt tttcaaaagc gtcttagact gtatgtgtat atacacacac 24120
   attctgaagc agtaggtggg tcttggggcc tgagatctcg ggtgaatgta aatttaggtt 24180
   cacaggtgat actgtagatt cacagtgtct acagagtaca ctatgaattt gtggtgacta 24240
   cattattgac aaaatatttt aggtttataa tcagaaaaaa gttaaaatag ttaatgaaga 24300
   tgctttaaaa gcctgtgtac tttagagaag ctacttaaca caaattgggt atctaatgta 24360
   ggctgggctg gatacttcat tttcatcaaa tctttttaaa ataattggtg aaataacctt 24420
   tattgaatat ggttttctac atttttcaca cttccctcct tcatagggtt gtgaaaattt 24480
   atttcatatt ctagatgagg aaattgaggc acagaggtac acttacaaag atacaataaa 24540
   tggcagaact aagatttgaa cccaggacta agtgtattgc ttgtatttat ttaattaatt 24600
   aatttttaag agacagggcc tcactctgtt gcctaggctg gcccttgaac tcctgggctc 24660
   aagcagtcca cctgcctcag cctcctgagt agctgggact gcaggcacac catgcctccc 24720
   agttgttttt aaacactaat agtagtcttt cataaggaca cttataataa aggcagagct 24780
   ggaacccaca cttcattcca gactgctcag actgagttag tgttagaaaa ctgaaagtaa 24840
   catttttatt actgtatttc aggtaatcag tatctgtttt tgccaccaaa tcgttacatt 24900
   ttccatggcg ctgaggtata ttcagactct gaagatgacg tcttatcctc tagttcttgt 24960
   ggcagtaaca gtgatagtgg gacatgccag agtccaagtt tagaagaacc catggaggat 25020
   gaaagtgaaa ttgaagaatt ctacaatggc ttagaagatg agcctgatgt tccagagaga 25080
   gctggaggag ctggatttgg gactgatgga gatgatcaag aggcaattaa tgaagctata 25140
   tctgtgaaac aggaagtaac agacatgaac tatccatcaa acaaatcata gtgtaataat 25200
   tgtgcaggta caggaattgt tccaccagca ttaggaactt tagcatgtca aaatgaatgt 25260
   ttacttgtga actcgataga gcaaggaaac cagaaaggtg taatatttat aggttggtaa 25320
   aatagattgt ttttcatgga taatttttaa cttcattatt tctgtacttg tacaaactca 25380
   acactaactt tttttttttt aaaaaaaaaa aggtactaag tatcttcaat cagctgttgg 25440
   tcaagactaa ctttctttta aaggttcatt tgtatgataa attcatatgt gtatatataa 25500
   ttttttttgt tttgtctagt gagtttcaac atttttaaag ttttcaaaaa gccatcggaa 25560
   tgttaaatta atgtaaaggg aacagctaat ctagaccaaa gaatggtatt ttcacttttc 25620
   tttgtaacat tgaatggttt gaagtactca aaatctgtta cgctaaactt ttgattcttt 25680
   aacacaatta tttttaaaca ctggcatttt ccaaaactgt ggcagctaac tttttaaaat 25740
   ctcaaatgac atgcagtgtg agtagaagga agtcaacaat atgtggggag agcactcggt 25800
   tgtctttact tttaaaagta atacttggtg ctaagaattt caggattatt gtatttacgt 25860
   tcaaatgaag atggcttttg tacttcctgt ggacatgtag caatgtctat attggctcat 25920
   aaaactaacc tgaaaaacaa ataaatgctt tggaaatgtt tcagttgctt tagaaacatt 25980
   agtgcctgcc tggatcccct tagttttgaa atatttgcca ttgttgttta aatacctatc 26040
   actgtggtag agcttgcatt gatcttttcc acaagtatta aactgccaaa atgtgaatat 26100
   gcaaagcctt tctgaatcta taataatggt acttctactg gggagagtgt aatattttgg 26160
   actgctgttt tccattaatg aggagagcaa caggcccctg attatacagt tccaaagtaa 26220
   taagatgtta attgtaattc agccagaaag tacatgtctc ccattgggag gatttggtgt 26280
   taaataccaa actgctagcc ctagtattat ggagatgaac atgatgatgt aacttgtaat 26340
   agcagaatag ttaatgaatg aaactagttc ttataattta tctttattta aaagcttagc 26400
   ctgccttaaa actagagatc aactttctca gctgcaaaag cttctagtct ttcaagaagt 26460
   tcatacttta tgaaattgca cagtaagcat ttatttttca gaccattttt gaacatcact 26520
   cctaaattaa taaagtattc ctctgttgct ttagtattta ttacaataaa aagggtttga 26580
   aatatagctg ttctttatgc ataaaacacc cagctaggac cattactgcc agagaaaaaa 26640
   atcgtattga atggccattt ccctacttat aagatgtctc aatctgaatt tatttggcta 26700
   cactaaagaa tgcagtatat ttagttttcc atttgcatga tgtttgtgtg ctatagatga 26760
   tattttaaat tgaaaagttt gttttaaatt atttttacag tgaagactgt tttcagctct 26820
   ttttatattg tacatagtct tttatgtaat ttactggcat atgttttgta gactgtttaa 26880
   tgactggata tcttccttca acttttgaaa tacaaaacca gtgtttttta cttgtacact 26940
   gttttaaagt ctattaaaat tgtcatttga cttttttctg ttaa 26984
<210> 4
   <211> 20029
   <212> DNA
   <213> Mus musculus
<400> 4
   gccagtgccg cgcgtcgagc ggagcagagg aggcgagggc ggagggccag agaggcagtt      60
   ggaagatggc ggacgaggtg gcgctcgccc ttcaggccgc cggctcccct tccgcggcgg     120
   ccgccatgga ggccgcgtcg cagccggcgg acgagccgct ccgcaagagg ccccgccgag     180
   acgggcctgg cctcgggcgc agcccgggcg agccgagcgc agcagtggcg ccggcggccg     240
   cggggtgtga ggcggcgagc gccgcggccc cggcggcgct gtggcgggag gcggcagggg     300
   cggcggcgag cgcggagcgg gaggccccgg cgacggccgt ggccggggac ggagacaatg     360
   ggtccggcct gcggcgggag ccgagggcgg ctgacgactt cgacgacgac gagggcgagg     420
   aggaggacga ggcggcggcg gcagcggcgg cggcagcgat cggctaccga ggtgagtccg     480
   cgcgcgctgc cgcgcagccg cgtccctccg cgtccccgcc tgtgacccgg tcccaggttg     540
   cccagggccc cgagagctcc gcgggctccg cggcggcggc ggcggcagcg gcgcagctcg     600
   gccctggctc ggccgcgcgc cgccgagctc gccggccctg ctgcggagcc gcgcgcggac     660
   tcgccgctcg cctgggccgc cttgcgctgc tgcgcgcagt cgccgggacc gcggttctcg     720
   cagtttggcg ttagagtgaa gttttccctc cctcggtttg gtttccctcc cgatgcgcct     780
   ttagtgaagt tatagttttc ctcactgcac actttgcttt gcgactttgg ggctgtcccg     840
   tggatcgaca tgcgatttcg ttttgcaaac tgacacctgt gttgcttttt tttttttttt     900
   tcttttttgg atagacctat gtcttttttt gaagttagaa attttaatac accaaagcta 960
   tttcttgaga cacagtctaa aaaatgtaag gaaatggcta ttcctaaaaa tgttgctttt 1020
   ctgcgattgc atttatcact tcgatataga ggagttatag ttgtactgta ctttcgtaag 1080
   ttttcttttc ctttcaaaat catgctcgca acatttttag attaacaaac ctgaaacaaa 1140
   cctgcttggt tttgccctat tgacgccaaa cgtttgggtt acttttttgt tttgtttttg 1200
   taataatgcc acttatcgtc ttcctgaaaa gctttgtaca gaatttatca gagtctgtca 1260
   gtgcccttgt agatgtttgt ggctcgtata atggtgccat gaattaaaaa acaacaaaac 1320
   gtgttaggga ctgaaatagg aaatgaccct tgatgtcagg tattaaaggg gaaacaagtc 1380
   gcataaagta tgcttcgtgt tctttaaatc atacatttac taacacccag tggctgaagc 1440
   aggtagattg ctacttgaaa gggctacgga aagagacttt ctctggttgg ggcagtgaga 1500
   acacagccag ggattgattt ttatctcagt gatatttaga agtaatttct atttgatgca 1560
   tttcatgaag ttgtatttct ttgtcctggg catggaaccc tgagctgctt ttgtagggct 1620
   acacagattt tatttgtgtg tgtgtgtgtg tgtcttcatt ttggattttt tgaggtaggg 1680
   tccctgttta gcccaggctg gtccggaact cactatttta tatatgtcag gctggtttcc 1740
   aatgtctgtg ccttccaggt gctggagtta cagatgtgtg tgcttctgtg cctttctggt 1800
   tttttgtttt tgtctcaaga acctgtgtgt gtctcaccat acaggccaaa cgggcctcaa 1860
   agtacctggg attgcaggtg tggaccgcag ggcccagctg gggattgtct gagccgcagc 1920
   cttttgatct ctttgacaga caacctcctg ttgaccgatg gactcctcac taatggcttt 1980
   cattcctgtg aaagtgatga cgatgacaga acgtcacacg ccagctctag tgactggact 2040
   ccgcggccgc ggataggtat gcttcaggca tgtgcatctt tgaaacaatt gcaaacttcc 2100
   ttcattcctt ccgtccttct gtccgtcctt cctttttgag acaagatatt ttatagccca 2160
   agctagttaa gctactctgc agtctctgcc cagcctctgg agtacatcac aaccaaagct 2220
   ctttttgact tactcttcct tctctctttt cttgattttt tttttttttt tttttgacat 2280
   tttcatctat cctatgatgg ctttaaactt gtggagtcta ggatgtaagg atgacattga 2340
   tatcctgact ttaccagcag tccagagtgc tgggattata ggtgtgctgt atgtaccatg 2400
   tccgtttgtc agtgcctgag gttaggtcct cacctctttt cttctgtaca ttgtttttgc 2460
   tttctgtgta cagcctttgg gggttttgct tgcttgtttt gttgtggttg ttgtcgttcg 2520
   tctgcttgct tgttgagaca ggaacacgta tagcctagac tggcttgaac tcattggctg 2580
   ggtagccagc cagcacatat tagccttgaa tttctgatct tgtgtctaaa tgctggggat 2640
   tatagttatt tggtaccacc ccaacagctg cttttgtacc aactttaaat tctctgtcac 2700
   ctttaccatt tagatttagt tgctcaggta ttggtaagat tttttttttc ttggcctcct 2760
   gcatggtaat ccttgagtcg ctcaccacgt cctcccatct aaggcagtca gaatacctag 2820
   acagtccagg cagtagtggc aggcatcttt aatcctagca ctcaggaggc agaggcagga 2880
   gaatcttgag ttctaggcca gcctgatcta cagagcaagt tccaggagag ccagggctac 2940
   acagaaaccc tgtccccaac caaccaacca aaaaaacccc ttagacttaa gcacaccctc 3000
   cccaaactga attagtcaga tcttgttagg gatatgacat gaaatacata taaattgtat 3060
   tctttgcata aaaatgtata tattgctaac tcattgcttg aaaaaatagc tttttattag 3120
   aagtggaatt gatgtgagtc tttcttccct taatataggt ccatatactt ttgttcagca 3180
   acatctcatg attggcaccg atcctcgaac aattcttaaa gatttattac cagaaacaat 3240
   tcctccacct gagctggatg atatgacgct gtggcagatt gttattaata tcctttcaga 3300
   accaccaaag cggaaaaaaa gaaaagatat caatacaatt gaagatgctg tgaagttact 3360
   gcaggagtgt aaaaagataa tagttctgac tggagctggg gtatgtaaga cgcagaaaaa 3420
   cagtaaagag aaagctagta gatattcctt ttccagctaa ctttttctgc tcttgatgta 3480
   gcctttgttc ataaaaactg aagagagcct catttgttaa actagttcag tgttttgaaa 3540
   agtttgacaa ctttaaagat ttgtatattt atagaacaat tggtaagtgg aattatggtt 3600
   tatttttttg tttttattta agatcgtgtg tgtgtgtgtg tgtgtgtgtg tgagagagag 3660
   agagagagag agagagagag agagaaacag actcattcat tcattgattt ttgcctacat 3720
   gcataaatgt gcatggtacc cacagaggcc agaaatgggt gttgggttcc cagggatttg 3780
   agttagtcag atgtaggcca ccatgtggat gctagcaaac ctggattctt ttcaaggagc 3840
   agcaagttct ctttagtggc tagactctct agctcctgga cttgtttggt tttaatttta 3900
   tgtatatgga tgttttatct atatgtatgt ttttgtacca ttgtgtgatg tgcccaagga 3960
   gtccagaggc atcagatccc ctgaaactgg agtgatgatg tttgtgagcc tcccaagtcc 4020
   tctgcaagag cctcaagcct ctttcaaacc tggaattttt acacattata tgctggctcc 4080
   taaagatgat ttagaagaat tagagctttt tgaagtctat ggtagcaaga gtctagtgga 4140
   gaaagttttc tttcattctt ttttaaagat ttatttatta aaatgagtac actgttgctg 4200
   tactgatggt tgtgagcctt catgtggttg ctgggaatga aagttgctca ctcaggtcaa 4260
   ccccgctggc tctggcctaa agatttatta ttatatctaa gtacactgta gctatattca 4320
   gacgcaccag aagaggacgt ccaatctctt tatgggtagt tgtgagctcc atgtggttgc 4380
   tgggatttga actcaggacc tttggaagtg taattgtact cttaaccgct gagccccctg 4440
   ccctccttca ttttaccttt gtaagattgt agagacttgg tctagaacca agtcatactg 4500
   tgttgtgttg tatgttaggc attatgttag caaccagagc agtttgaaca gagtgaaaag 4560
   tatgaatgaa tcagaatctc agttgtggga ggcagaggca ggtgagcatg ccaaagcagg 4620
   tcggtgtgtg ctggcccctc agccccagac gagggctagg gttgcttcag gtgtgattta 4680
   gactagggtt cgcctcttat ttttattttt atttatttat tttttttatt tttagggaca 4740
   aacattaagt tttgtgtttg ggtgcgtgca tgtggtggtt tgtgagctat agcactgtgg 4800
   gagtcagtac accctgagtt gtgagttgct tgtccttcct cactgtcagt cttggctcca 4860
   ggcttcatca gaagcacctt aactggctga gctattctgt aggcccccag ttttagttat 4920
   tcttgaaacg ggcttactca cagccctgga tggcctggaa cttggcagag atcctcccaa 4980
   ctattgggat tatttcaggt atgtactacc atgcctggct ttattttttt tgagacggtc 5040
   tctatagttt aggatggttt caaacttgca gcagtccttc tgtttcagaa aaattgggat 5100
   taggagtata tagcaggaga caatctgttt gttttgttcc aagttgaatg gatctgtaaa 5160
   actggaatgg aagaaagctt gtccattccc ctatcgggag tgtcaagatt tatctacaga 5220
   aacgggtatt gtcttaattt atttggagac aggattatac tatgtagctc tggctggcct 5280
   agaacttcct ttgtagacca ggctggcctc aaacttgtag agatccagtc ctattctggc 5340
   tcccaaatgc aaagattaaa ggccagtgcc atcatacctg gttattaaaa aaattttgtg 5400
   tagccatgtg tagtaacaca tgcttttaat tctagcactt gggaggcaga gactggtgaa 5460
   tctgtgagtt ggaggccagc ctggtctaca gagcaagttc caggacagcc agggctcaca 5520
   gagaaagcct gtcttgaaaa cctcccaatt tttttgtttt cttgtgtggt gtgtatatgt 5580
   gggtatgcac gcacatgagt gtgtcagagg acaacttgca agtattacat gtggtcatcc 5640
   agtacggtag cctttagctg ctgagacatc tcactggccc tttgttgtaa tttattattt 5700
   tcactgcttg agcctggaca gacactctac agttgaccta tccttagccc tttttaggtt 5760
   ccttagggtg gctacctgaa cttaaaagat cctgcctcag ctatcttagc tctgactata 5820
   aacttgtgcc actgtcctgg gttttatcat tataatttat aattaaatgc atatccttag 5880
   ctggctatgg tgatgcattt tctgttatcc cagcatttgc ctttcagggc cttagtgact 5940
   gtaacctcag cattaggatg tagacacagg cagatccctg gagcccaatg gctacccagc 6000
   ctaactaaag acactatctc aatgagaatc gaggtggccc atgccactag ttctagaggc 6060
   aggtaggtgt gtgtgtgtgt ttgtgtttgt gtttgtgtgt ttgtagtgag accatgtcaa 6120
   acaaaagaca aaaattaagc tattaataag taagtaaatg attcattctt gggctagaga 6180
   gatgacttag aatttaagag gtcttactat aaggccaggg gcccagattc agttcccagt 6240
   acccatatgg caattcacaa ctgcatgatt gatgcattct gacctccgtg gacaccaggc 6300
   acctgtgtag aacacatata catgtaggct tccctatgtt catataaaat aataaagcct 6360
   aatggcttct gaaaaaaaaa aaaaccaaac aaacccaaca aactttgctg tgatatgtgt 6420
   aaggtagttc taactcctgg acaacataac aaagatccca aacccttttt tgagaatgat 6480
   tttgctgctg tgattgtccc caggtagtct tggaaatcag agcccaaaca gcccttcagt 6540
   ctccctgagt atctgagact ttaagcatga accagtgtgc tcagtctata atacagtatt 6600
   gaataaagtt tcaaaacttg agtattcttg aggtagtaaa gctgcctatg tgaggtcagt 6660
   ttggaatgat tggtaaaaag ttgtacctac tttttggtta aaaaatgaaa ttgagctggg 6720
   tgtggtggcg cacaccttta atcccagcac tcgggaggca ggggcaggcg gatttctgag 6780
   ttcgaggcca gcctggtcta caaagtgaga aagtgagttc taggacaggc agggctataa 6840
   agagaaaccc tgtctcgaaa aaccaaaaaa aaaaaaaaaa aaaagattta ttccaaccaa 6900
   ttgtatttgt tttaatttgt ttaaatgctc aaagggttaa gattagccca ttaaagcagt 6960
   atgtggcaga tttaattata cttatttacg tattttacct gtatttgaaa ttagaaagct 7020
   tgtggtgttt ctgttcaaac tttgattttt catctcttcc cactaaattg tatgtagtaa 7080
   aggtacttac tgtgaaggta gacagttaat gggttgactt aggtcttgtc tgtttcaggt 7140
   ttctgtctcc tgtgggattc ctgacttcag atcaagagac ggtatctatg ctcgccttgc 7200
   ggtggacttc ccagacctcc cagaccctca agccatgttt gatattgagt attttagaaa 7260
   agacccaaga ccattcttca agtttgcaaa ggtatcacac gtttttgtct ttatgataaa 7320
   gagttctcac gtgtaggctt tttaagttta cattcattgc ccagtgattt ggaggctacc 7380
   agtgtggtag ttagaactta agttgtttag gactgatttt aaataatgta attttggtct 7440
   tttgcctgtg tgtcttgccc tctcctttgg aagtttgtag atctgaaaaa aaaaagaaag 7500
   acttgttttc acttactctt ttttttctga atcataaagg ttctatgact actatgtggt 7560
   atgttttata atggacaggt cttaatatat gcatatgtct aaaatgattt ttaataactc 7620
   aaggttgaga ttacatgttt tgggaaacat tacaagggac gtctttctag ttgataagtc 7680
   agtcaccacc atgtgactgc atgtggctat tgaatgtgtg taatgacaca gtgagggcat 7740
   gcttacttaa ggattttaaa tactttgcta aagaaaagtg aaatatacta attgtactta 7800
   ttaaatacta acaagcaaac atttttatgt ttgggactat gttcaggagc caggcataat 7860
   aatacaagtc ctgcagctcc gggatttcag gggcacagga tacttcatga cctgccaggg 7920
   agggctacat catgagactc agcttcaaaa acaaagcagt agcaattttt tattaggcat 7980
   ttgaaaattt cactttcatg tgaactaaag aaattgaaca cttggctcct atgtctgctg 8040
   tgcagctctg ctctgctgta gaatataaag tttatataaa ttgtctcaag attttagtca 8100
   ctagaaactt tgcaaggttt tatttgtttt gtttctttgt ttttttgttt tttttttcca 8160
   gtttaatctt aaggtttttt gttttgaggc agtctgatgt aggccaggcc ggtcttgaat 8220
   tttgcttagc catggctttt gaatttcctg agcctcctgc gtctaactcc catctgttgg 8280
   gattagagca ggagctgcta tgcctggctg ttatgttcac tgttagggaa cttggaaata 8340
   catatgcaag gcttttctga tttagagaga tgttttagag acgtctttaa aagtttcatc 8400
   tgtggagggg tggagtggtg aatggtgttg cactcccaag tgcaccggtg gatgtgacag 8460
   cactacctta gtttaggggg ttgggttccc ccacatcaca cgagagttcc tggattagaa 8520
   ctcaggtcag cagattaggc aacagactta tctcccaatc cacctcccat cttgtaagcc 8580
   ctaaggtgga ttagaaaggc tgtcttcctt cagtacagtc tcagaactgt aagggtgctt 8640
   tagttcttgt ctccgtatcc tggtgctggg attagaggat ggccagactt tgcagctaag 8700
   gtgtactatt ggaagctgtg ctgctacact gtttaaagaa taccagtcaa agattgtttt 8760
   aatggatgca ggaccctggt gtatcatggt gtctactata agtagacaca gatttaggaa 8820
   ggccagcaag taaaagttac tttcatctta tacaagcata gctggttagt gattatatgt 8880
   tagtattccc acaacttttc ttataaattc tatgttgtga gttggggaaa ctgcattgtc 8940
   ataactagtc tttttttttt tcccccccag tattttaccg ggattgtcaa gtgcagatac 9000
   atatctaaaa ttgaaagttc cacatgttct taaaattaca tttagttatt tattgcttat 9060
   gtaggtgtat gggcaagcat cattttgttc ctctttaggg gattataagg actagaattc 9120
   cagtaaactt agctctccaa taatgcgtaa caagctaacc cttttatgtc cctaatccag 9180
   ggtttaggag gtggaggcag aagggtctca agagttaagg gcagtctctg ctaaacactt 9240
   gagttccagg ctctcctggg ttacagagtg aaatagcaca cagtggtaga ccatttttag 9300
   cacaaggccc cagattttgc tacccacaaa gaatacacat gctaaagtat gtttttccta 9360
   aattttaatt gtgttttagg gcagattaaa ggcataaaaa attagtttga ttaaaaaaag 9420
   atagatggga taattaaagt gaccttagtt tgtggatgat attctatatg tagaccctaa 9480
   aagcctcacc agagaactcc ccctttttat gtaccaccta acaaacatgg gcagggagga 9540
   aatctggaaa aagtcttcat agtcatctca gacaacagag gctggagagg cgagccagtg 9600
   gttatgtgtg catgctgctg cttgcacctt tcagaggtca gtttccagca tctgtatcca 9660
   gaggctctac agctgcagtt atccccaggc ccagttgagt cacgtgctgc tggccttcag 9720
   gcctccactt catggcacct gcactcaccc acatcctcta ccccagacat gcagataatt 9780
   aaaagtgtta aaatgggggc cgttgagagc agcacttgtt cttgcacaga ggatcagagt 9840
   ttggttccta ccactcacat ggtggctcat aaccatccat tactccactt ctctccaact 9900
   cccctttctg tcatccttgg gcaccaagca agcatgtggt acatatatat taccttcagg 9960
   caaagcactc atttatataa aattacttct taatataaaa aaccaagggt taggtatggt 10020
   agtgcaggtc tttaatctca gtcctttgga gttagaggca ggtggaccat ctagggctac 10080
   ataataagac cctaccttta attcagtttt atccatatga actatgtata acatgaagta 10140
   tgtgtaatgc tctgcattat catatgtgtg tgagttttgc atgttcatgt tcaagttcag 10200
   taagaattag ttcatagctt ccctagttga aattctttta tttggttagt cggtttggca 10260
   tacatactat caaaattatt tcctgaaatg tgatgaaata ttgtataatt ctccatgagt 10320
   gatgacctat ggattacttt aagtggacag ataagtgcat tttttatggt atacattata 10380
   ttgtatttat aggatggcta tatcgttaac atccattact ttacataggt atttctactc 10440
   ttacatcaag acattgtaga tacacaatgt tagtaattgt aatctctagt ctaaatattg 10500
   ttctccctca gagattatgt gtcggctcac tagcttcttc ccaatcttac tctaccatat 10560
   tagctgagaa ttaattgtta ataactggga gctgtcacag gaccactgtt ttacatttac 10620
   tattctgtcc ttgagtcagt tttatgaccg ccacgtctat ttacagatca tctttgactt 10680
   ctttttctat atgagaccat atggcaaatg tatagcccat aaagcctaaa atattccctg 10740
   ttgtgaccct gtacagaaaa tatgttctgt taaaattaaa taaccttcaa gaggtagctg 10800
   tcaagaaatt gagtggagtt taatgaataa gtaggaggag ggttcttagt ggaattttgt 10860
   ttcacattgc atgtgtgtgg tgtgtatgca gtgtgcagtg tagatctctt gttttgaggc 10920
   agtctgatgt aggccaggct ctcgccatct cttgggacag gggatcctgc tagactgttc 10980
   agctggagtc ccttgtttct gcctcctgca ttcagagtta gagcactggg cctcaaactg 11040
   acagtgtaaa tacttcccac tgctctagtt tctctacccc ttcacatttt tatgtgctgt 11100
   tgggattaaa ctctgtgcct cccatgccac cagccttcta gtgtttatag aatgaaaatg 11160
   taggttataa ccaaacaaac tcaactgttt tcctaggttc tacaactcta agattaggtg 11220
   aattttgcct ctacctccga gtgctgggat taaaggtgta tgccaccaac actacgcagg 11280
   ctattatctt ttaacaaaaa aatttaaact tttgatctca gcagaatacc caacatagtt 11340
   ggtttcttaa aagcctttag gatgtttaga cttgagatgg gtgacctatc tttttgattt 11400
   ggaataatta acccatgccc tataaaaaaa ctccaagcta tttgagttac agcagaattt 11460
   ttagattcag tctatataag gcaaaatttt cattaaaagt ttttagaatt tttagaattt 11520
   atagaatatt tccagctgtt tcttaatggg attccaggca tgtgccactc agtccagctt 11580
   catgagtgct ttctgaatgc actgtaatgg agagcaaaat taaagacagg accatccata 11640
   tgtatataca cataacttca gtttttgctt aatacaacca gaaagattac accctggtga 11700
   ctaacttgct ttgttcatgt gtgtcatggt ctatttttag gtcagtacat gagagccctc 11760
   tcattctctc agtggctgaa tattccactg tatggttgta ccttgcacaa ctagtttccg 11820
   tattgatgga catacatatg gttgtgttta gtttttgcta acattataat gggttagata 11880
   aagcctataa tgtaaggaaa cagatagtgt aagctacttg aaagaaaaaa gaaacttcct 11940
   tgccacagtc actcactaaa tgaaattgaa taagaacact atcaggagtt gtgaggatga 12000
   ggggctgaag agatggctca gcacttaaga gcactggctg ctcttccaaa gggcctgggt 12060
   tcagttccca gcacccacat ggcatgtgga tctgacaccc tcacacagac atacatgcag 12120
   gcaaaatatc aatgcacata taaatgagta aattaataaa tgtatatatt taaaaagttg 12180
   ggaggaccaa aattgatact ttttctctga tctttttgcc atgtgtatat ctgaattctt 12240
   tgtttttaaa gaagaaacag cattgaagca ttatttgggg ggaaaaacac acacacaaaa 12300
   tccagcaact caacattcat gagcagctct gttctatacc agtatgtgcc tgtgcagtgg 12360
   aaggaaagca attttggtaa ggaattaaaa cttcagcttt aaactcccag caggttgata 12420
   tttatcaatg atgaatcaaa cagaaagttt taaataattc attgacagcc ttttttttag 12480
   ttgtaaagtc accatacctt taatatctgt aatttcagtg taaagttttc cccccttttt 12540
   ccttaaagaa aaactatgaa tttgcagcca acaaagttta gatgtatcta aggtatgtat 12600
   ttcttctgac attaagtagt ccattacgat gtgttatctt gcacttcaag ggaccaagtt 12660
   tactaattgc tgagttttca taaagtaaca agctttttct aggggccatc catttagatg 12720
   ctttaaaatg ttcatgtatt ttggttatta cagaatattt attgttagta cctgtttgac 12780
   taaacataag caggatatct gttatgcttc tttcattaat taaacttttc tgccttttgc 12840
   attacatagg aaatatatcc cggacagttc cagccgtctc tgtgtcacaa attcatagct 12900
   ttgtcagata aggaaggaaa actacttcga aattatactc aaaatataga taccttggag 12960
   caggttgcag gaatccaaag gatccttcag tgtcatggtt agtgacgtca cagtggctct 13020
   cgtagggcgt tcaataggta accgggcttg gaaagaagct gctgtctagt gacgagacta 13080
   agcgttattt agtaaccttg agtggacaaa attgaaaagg acacaaacaa agctgaaggc 13140
   ctcagatgtg gtgggtatac tttgataaca agttcaaggc tagccttgac taaacaagac 13200
   acagccaagc taaggtaact cagttggaac cttccagctg cagttttggc acttgcagag 13260
   ttatgttatt gcaaatagta gctttagaag ataaggtcta tatacacatg tattagtaga 13320
   taaactatat cacatgtatt atattcctta aatacgtcac aggagagttc aggttagtct 13380
   tcatggtggg gctagagaga tggctctgca tttaagagca ttaagagcac tgactgctct 13440
   tccagaggtc ctgtgttcaa tccccagcaa ccacatggtg gctcacaact atctgtaatg 13500
   aatgccctct tctggcatgt agcagtgcag gcagatacag cattcataca taaaactgta 13560
   aaaagtcttc atggagcaag ccacaaatag cccttcaata aatgttctat ttggtgctgg 13620
   agagatggct cagtggttaa gagcaccaac tgctcttcca aaggtcctga gttcaaatcc 13680
   cagcaaccac atggtggctc acaaccatct gtaatgagat ctgacgctcc tctggtacat 13740
   ctgaaatctg aagacagcta cagtgtactt agatataata ataaataaat attaaaaaaa 13800
   aaaataaatg ttctattttg gttgttttag gttcctttgc aacagcatct tgcctgattt 13860
   gtaaatacaa agttgattgt gaagctgttc gtggagacat ttttaatcag gtaatttaat 13920
   tcatctcatt ttaggaattg tgtaaacttc acttttggtt gtatcttcat cccccccccc 13980
   ccccccttct gagaagcatg agtgttagaa atcctgatgt aaacaggtct tttttgcaag 14040
   agcatgttag agcacacttg aaggctgagg cagggcactg taagagtcag gctaacctga 14100
   gctggatagc aagagtctca ttttttaaaa aaaaagtttt tctgttgctc agttggtaga 14160
   tcatggctgc ttagcatttg tagtcctggg attgctctcc tgcctgaaat cctaccattt 14220
   aaggaggttg gagaataaga agttgaaggt catggtcacc tacaaactct ttctcaaacc 14280
   aaccaaccta aagtcctcct taatacacct aagcacctcc ttatataatc taggcctaag 14340
   tcacaagttg cagggaacac ctctggaatt agtcttacat tagttgtggt aagatttaaa 14400
   acacttttca aaatgataat gaagtattta ttacaggaaa atatagcaac acacatccag 14460
   atatatgttt taactttgaa ctcatgtata accaggaaaa aaaaagacag tatactcaaa 14520
   tggagtcatt ttcataaata tttttaaaag gtcttgtttt cattctttac cacgctcttt 14580
   gcatatttat agtatcttgt gggtgcacag tgcgtatggg gaaatgatag catgtgtgga 14640
   gactggataa gagagatcag gctgtggagg tcagaagatc aacctgggag tcagttctca 14700
   atttctacct gggagttaaa tttggttgtc cggcttggtg aaaagtgtcc ctgcctggta 14760
   ggtcacccac ccccaggctc cacactggct ccacagcaga cactatcaaa ttcacagcgc 14820
   tttgtttgct gggaatgtgc cgagtgctaa cctgggctac ttgactttca tgttttgtgc 14880
   aggtagttcc tcggtgccct aggtgcccag ctgatgagcc acttgccatc atgaagccag 14940
   agattgtctt ctttggtgaa aacttaccag aacagtttca tagagccatg aagtatgaca 15000
   aagatgaagt tgacctcctc attgttattg gatcttctct gaaagtgaga ccagtagcac 15060
   taattccaag taagttgatg tttaggggac agttgtgcat gctgtgagtt gtggatctgt 15120
   gtaagatgtg attttactct tttcatttag aaaatagttg aaggtctaag ttttactttt 15180
   taggtgatac tcataaagtg acattttggt atgggaatgg gactgtgaag acagaatatc 15240
   agattttaat tgtagttcat ataaaatcaa agcaaaactg tttgacgcat gctttttagt 15300
   agtgtgcata ttaaagttgc agaggtcagc tttaattaat tccaggaata gtctcttctt 15360
   tttattttat gtgtgggggt gtttgcctgc atgtctgcct actttggcca gacaagaatg 15420
   ttaagagtgt ccttccaagg ggctggtgag atggcttagc agttaagagc actgattgct 15480
   cttccaaaat aacactttaa aaaaaaaaag tgtcgccccc tcccccaaac agtagttctc 15540
   aaccttccta atgatgtagt cccttttgta caattcatgt tataacctcc aatgataaaa 15600
   aatttgttac ttaacctata atttttgcta gttataaatc acaatgtaaa tatctatttt 15660
   ctgatggtct taggcaagcc ctgtgaaagg gtcattcact cccaaaggag ttgtaaccac 15720
   aggtcgtgtg acccactgct ctagaatatg tacagtatta gtatcccctc attgctattt 15780
   tatgtgctta tgtatgaaag aagtgtgtgt gtgtggtgtg tgttccagag atggactgat 15840
   cagtagtgaa gaacgctatt cttgagtgga cccaggactc acacccaaat tttgtcagtt 15900
   tataggggat ctggtgcttt gtttggccaa gtacttacat tgtacactta catggatgta 15960
   ggcacataac ccgtccccct ctcccccaac acactccatt tcttgaaaca gatctcacta 16020
   tacttggcac ctgggatacc tatgtagact aggctagccc cacattccag tctcctgagt 16080
   gatggatggg atcaaagctg ttcccagcta atctttggta gccaggagtg gtagtatttg 16140
   gtagcagggg tgcctgtaaa ccatgtggta tagggcagga agagtcagga gttctactca 16200
   gcagatttga agccagccta ggatagatga aaaaaatgag ttctattcca taatagttgt 16260
   agttccacaa gaatatttac ttatgtctag gagttcagcc tatgggccaa cataatccca 16320
   aagggtaaat tttttgaatt gaaaccactc ctccatgtcc aagtttctgt cattcttgat 16380
   gtcctcaaag atccacccgc ctctccctcc caagtgttaa agccatgagc cttcacaacc 16440
   agctagactt ttattttttt aagctaattt aaaggattag tttaagcttt ctgaactgga 16500
   acacctagga tctcagctaa gtcttgaaca gaagtgccat gagtttaagg cctgagcggg 16560
   aggaaagtct actgtttgtt attgagagga agttgctttt gaaaatgtga tttgtatttc 16620
   tgcttgcccc tctttttgtt gttgggatta gcgggggtgt gctaccatgc ccacctcttg 16680
   acagttgtta aaattaactt ctcttttcta actccccttt tcaccccatt tttaggttct 16740
   ataccccatg aagtgcctca aatattaata aatagggaac ctttgcctca tctacatttt 16800
   gatgtagagc tccttggaga ctgcgatgtt ataattaatg agttgtgtca taggctaggt 16860
   ggtgaatatg ccaaactttg ttgtaaccct gtaaagcttt cagaaattac tgaaaaacct 16920
   ccacgcccac aaaaggaatt ggttcattta tcagagttgc caccaacacc tcttcatatt 16980
   tcggaagact caagttcacc tgaaagaact gtaccacaag actcttctgt gattgctaca 17040
   cttgtagacc aagcaacaaa caacaatgtt aatgatttag aagtatctga atcaagttgt 17100
   gtggaagaaa aaccacaaga agtacagact agtaggaatg ttgagaacat taatgtggaa 17160
   aatccagatt ttaaggctgt tggttccagt actgcagaca aaaatgaaag aacttcagtt 17220
   gcagaaacag tgagaaaatg ctggcctaat agacttgcaa aggagcagat tagtaagcgg 17280
   cttgagggta tggaatgcgt tttgttgaat cactttcaaa gtactgtgat cagaggaggg 17340
   tgattcccaa tgtcagataa ctgttttgac attagttgat gaaggtagtg ctatgtgttt 17400
   gtgtgtatta agttaaagta agtttgggcg atgagtttgt ctttgctggc agtagtgtgt 17460
   atgattaaac ctggggcttc aagcagctag ggaacgagat ctgtcagctg agttatatcc 17520
   caaaagtggg ttttactccg ttctcccacc tccccagcaa atgattaaga cttgggtcag 17580
   tttatgatct tatagggtgg gaaccatgtt ctgggtggga aaactcaggg ttgaagatgt 17640
   accccagaag gtaattaggc ctaggtctgc cttaaggctg taccctcttc taccttcttg 17700
   ctcaaacaag gctgccttgt ttaagatctc gaaaacaggg ttcctatcta ctggacacct 17760
   attttgtcct gtcaactgtt agggtggtgc aaacactgct ccctcccctc taacacaggc 17820
   acaatctgtt catacacaat gggaggcttg tgctctggga attgcacttg gcctgtatca 17880
   tatttgcccc tggaacaagc tttggaagta tgtttctggt cattggtgca aacaagggag 17940
   ctgttagact tgaaaagatg taatcccagc cttcaggagg cagaagtaag agttgagttc 18000
   aagagtaagc caacaagtta ggtactaagg gacacataaa attctaagtg ctattttctt 18060
   ctgtatttca ggtaatcaat acctgtttgt accaccaaat cgttacatat tccacggtgc 18120
   tgaggtatac tcagactctg aagatgacgt cttgtcctct agttcctgtg gcagtaacag 18180
   tgacagtggc acatgccaga gtccaagttt agaagaaccc ttggaagatg aaagtgaaat 18240
   tgaagaattc tacaatggct tggaagatga tacggagagg cccgaatgtg ctggaggatc 18300
   tggatttgga gctgatggag gggatcaaga ggttgttaat gaagctatag ctacaagaca 18360
   ggaattgaca gatgtaaact atccatcaga caaatcataa cactattgaa gctgtccgga 18420
   ttcaggaatt gctccaccag cattgggaac tttagcatgt caaaaaatga atgtttactt 18480
   gtgaacttga acaaggaaat ctgaaagatg tattatttat agactggaaa atagattgtc 18540
   ttcttggata atttctaaag ttccatcatt tctgtttgta cttgtacatt caacactgtt 18600
   ggttgacttc atcttccttt caaggttcat ttgtatgata cattcgtatg tatgtataat 18660
   tttgtttttt gcctaatgag tttcaacctt ttaaagtttt caaaagccat tggaatgtta 18720
   atgtaaaggg aacagcttat ctagaccaaa gaatggtatt tcacactttt ttgtttgtaa 18780
   cattgaatag tttaaagccc tcaatttctg ttctgctgaa cttttatttt taggacagtt 18840
   aactttttaa acactggcat tttccaaaac ttgtggcagc taacttttta aaatcacaga 18900
   tgacttgtaa tgtgaggagt cagcaccgtg tctggagcac tcaaaacttg gtgctcagtg 18960
   tgtgaagcgt acttactgca tcgtttttgt acttgctgca gacgtggtaa tgtccaaaca 19020
   ggcccctgag actaatctga taaatgattt ggaaatgtgt ttcagttgtt ctagaaacaa 19080
   tagtgcctgt ctatataggt ccccttagtt tgaatatttg ccattgttta attaaatacc 19140
   tatcactgtg gtagagcctg catagatctt caccacaaat actgccaaga tgtgaatatg 19200
   caaagccttt ctgaatctaa taatggtact tctactgggg agagtgtaat attttggact 19260
   gctgtttttc cattaatgag gaaagcaata ggcctcttaa ttaaagtccc aaagtcataa 19320
   gataaattgt agctcaacca gaaagtacac tgttgcctgt tgaggatttg gtgtaatgta 19380
   tcccaaggtg ttagccttgt attatggaga tgaatacaga tccaatagtc aaatgaaact 19440
   agttcttagt tatttaaaag cttagcttgc cttaaaacta gggatcaatt ttctcaactg 19500
   cagaaacttt tagcctttca aacagttcac acctcagaaa gtcagtattt attttacaga 19560
   cttctttgga acattgcccc caaatttaaa tattcatgtg ggtttagtat ttattacaaa 19620
   aaaatgattt gaaatatagc tgttctttat gcataaaata cccagttagg accattactg 19680
   ccagaggaga aaagtattaa gtagctcatt tccctaccta aaagataact gaatttattt 19740
   ggctacacta aagaatgcag tatatttagt tttccatttg catgatgtgt ttgtgctata 19800
   gacaatattt taaattgaaa aatttgtttt aaattatttt tacagtgaag actgttttca 19860
   gctcttttta tattgtacat agacttttat gtaatctggc atatgttttg tagaccgttt 19920
   aatgactgga ttatcttcct ccaacttttg aaatacaaaa acagtgtttt atacttgtat 19980
   cttgttttaa agtcttatat taaaattgtc atttgacttt tttcccgtt 20029
<210> 5
   <211> 1028
   <212> **DNA**
   <213> Homo sapiens
<400> 5
   cttttcactt gtgaatacca attaggtttc cagtttctca taaagatcta acaaataccc 60
   aatttctcca tcagactgac atcccttaac aaaagcagag tttcaattcc ctgcatctcc 120
   tttaggagct atgatataat gtaggtagaa atcttgcctt aactccattt acccactgtg 180
   ctataaataa gcagaagcaa atattttttt aaggctggag aggttttaaa aatctgaact 240
   aatttagcaa ctgctgctgc actcagtttt tggcagttcc caaacatcca ttatcatgta 300
   aggataaatc cttctaaacc agaaaaatgt ttcctacttg gaaaaggcat aagaaaatac 360
   atatacgacc tccccatgta ctagtcttac ataccccagc tccagttaga actataattt 420
   ttttgcactc ttgcagtaat ttcacagcat cttcaattgt attaatatct tttctttttt 480
   tcctttttgg tggttctgaa aggatattaa taacaatctg ccacagtgtc atatcatcca 540
   actcaggtgg aggtattgtt tccggcaata aatctttaag aattgttcga ggatctgtgc 600
   caatcataag atgttgctga acaaaagtat atggacctac aataaggggg aaaaggctta 660
   aagtcaactt atcaagtaat tcaaaatctc atttattttc tgaagtaatg agttagcatt 720
   ctgtgagggt tttttgcaaa gtaagaaaat gcaatttaat ggtatttcat tctcggtaca 780
   ctcagaatta atgctatatc ccaatgagat taggaagatc taatgaagag ttgggaagac 840
   ccccttcagc tgtaagtata tatttcaaga gtctaattaa ttaacaacca gaattaagtt 900
   cttatggtta atatctagaa acacacacca taataccaaa agtatttaca aaagggttct 960
   acgacataga aaaatcgtac cagtcctaaa agcctgtact acttatcatt aaaaccacac 1020
   aggaaaaa 1028
<210> 6
   <211> 429
   <212> DNA
   <213> Homo sapiens
<220>
   <221> variant
   <222> (7).. (7)
   <223> can be a, c, g, or t
<220>
   <221> variant
   <222> (373)..(373)
   <223> can be a, c, g, or t
<220>
   <221> variant
   <222> (400)..(400)
   <223> can be a, c, g, or t
<400> 6
   cgacganaac ataagcactt ttaatttcct ctctattatg ctacagacaa ggccgaagac      60
   tgggtttttt aggttgttta aggctgtaaa gaaaacaaag aacatatgac agagacccta     120
   tgcagtctgc aaagcatact acatatttac taccaggccc taccttacta cagaaagttt     180
   gctgatccag ctgtgaacat ataccccgat gcagatgaaa acaaatacaa aacaaaccta     240
   acttgccatt ttggtcacaa gagcaagtaa gtagcagagc cctgttttga tatgaaaatc     300
   cagcactgga ctgggcaaca tggcgagacc ccatctctac caaaaatact aaaaaaatag     360
   ccgggcatgg tgnggcacat ctgtagtact agctacttgn gaggctgaga caggagaatc     420
   atttgagcc                                                             429
<210> 7
   <211> 156
   <212> DNA
   <213> Homo sapiens
<400> 7
   cctgtatata cacacactat gcaatagtct taggtaacta attagctgca accctaaggt      60
   agatcaaata gaaaatgtca agtcgccaca atcacatcat cttaattaat atggagggaa     120
   ggtaggaatc tgttactctt cccaacacta agcttt                               156
<210> 8
   <211> 593
   <212> DNA
   <213> Homo sapiens
<220>
   <221> variant
   <222> (460)..(460)
   <223> can be a, c, g, or t
<220>
   <221> variant
   <222> (571)..(571)
   <223> can be a, c, g, or t
<400> 8
   tctcactgtc tcccaggctg gagcgcaatg gtgcaatctt ggctcgctgc aacctccaac        60
   tcccaggttc aagtgattct cgtgcctcag cctcctgagt agctgggatt acaggcgcct       120
   gccaccatgc ccggctaatt ttagtatttc tagtagagtt gggatttcac catgttggcc       180
   aagctgttct cgaactcttg gcttcatggg atctgcccgc ctcggcttcc caaagtgcta       240
   ggattacagg cgtgagccac tgcactcagc cagaaaagat tatttaaaat aattgaatgc       300
   atgcaactgc agcatctttt gaaatcaaaa gcaaattaat atctgaggtt ttctataatt       360
   aactgtcaag gccaatactt ctggttttat tatttttggt ttctactttt ctgaggttat       420
   cgataaatgg agaaacatga ttaaataaat gctttatctn cacttctcga tggcagtcag       480
   ctttaatgga aaatattttc ctataaacct aaattaattt ccggaaaccc cttttgaggt       540
   taactaccat tgcactggaa taatctttgg natcccggaa ccctgttcaa ggg              593
<210> 9
   <211> 373
   <212> DNA
   <213> Homo sapiens
<400> 9
   ggcacttcat ggggtatgga acctaaaata gggtgaaaaa taagagttag aaaaagggag        60
   gaggaagtaa gctaataaaa ccacaacaga tacattatgc aaaaatccaa caagggcttg       120
   agctccaaat tcccaaaaaa gacccatgca ctaaatactt aaccaagcca ttacatttca       180
   tcaagcaagc agatgggcag ttaagtgtct tttataaaac gctcaagttc tgctcctatc       240
   acctggttac ccctgagcaa tcttcccaga ctttcccact ctgaacctca aatttccttg       300
   tctgcagaat ggggatgata atagtacttc tccacaagac tgtggtgagg attaaatgag       360
   ttagtcaagt gca                                                          373
<210> 10
   <211> 1713
   <212> DNA
   <213> Mus musculus
<400> 10
   gggctcccct cagcggcctc tggcgcctcc cgcccgcccg acccgttcgc tcgctcgctc 60
   gctcgctcgc ttgctcgtcc gggatcgccg cggtggttca agtttgcgat ggcgccgcca 120
   cttcccacct gggcctcacg cgtgcacctt gcctgcctgc gcctcttcgc ctcaagtcgg 180
   cttttacctc aggggctctg gagagcccaa cctggccgac gccggccttc ctgaggagaa 240
   ctcctccacc tgccttgccc ttgctctgtg acagctcttc ctcaggttac ccctgtggtc 300
   tctcctcagg aagtttgcgc tctctcccaa tctcccttct caagtgcaat ggaatgccca 360
   agccagccct cggggcctgt tgccctcctg gaaagatctg gcgattgagg acccgcccta 420
   tctgctctct ggacccacca ggtcctctgt acctcgcttt agtctttggt aaaattcatc 480
   tcttggggca gcaagagaga ggacagaagg gagagtggtt ggttctccac aaacttctgt 540
   gttaagagtc agattgggcc tgggctcttg tgacttgggc gattgactga accttttcta 600
   agcccagttt ttaatcatct ctaaaatgac agggccagga ccgaaagaga ctgtagctca 660
   gttgtaaagt cacgcttgcc agacaacccc gaagccctag agagagggag gaaggagggt 720
   aagttgaagg taatctccaa ctacttagga agttcaaaaa aggcctggaa tacataagac 780
   ctcgtctcaa aaacgaaatt taaaacgata gaccatgaga aatcagctag tcaggtttaa 840
   agtaaatgac attagtttta aaatcctagg cagttgatgg tggcacaggc ctttaatccc 900
   agcaagctgg aggagacagg aggaggttca ctaggacagc caaggctaca caaagaaacc 960
   ctgtctcgaa aaaataatct tacttctaga attgtagaaa tggctctgta gttaacagca 1020
   cttgttgctc ctgcagaggc cctaggtttg actcccatca tccacatgac agctcatacc 1080
   ttcagatctg acacctgctt ttggtaaaca cagacatgta tggagccaaa agacccaaac 1140
   acataaaaat cctctttgtt gttgttttat gagttagggt ttctctgtgt agccctggct 1200
   gtccaggaac tctgtagatc aggctgtcct tgaactcaga ggccacctgc ctctgcttct 1260
   tgaactgctg ggattaaaga tgtacaccag caagcccagc ataaaaatac atatttaaat 1320
   aattttttaa ataatcctta gttccttcac aactctaagc cccttcactt tctagttacc 1380
   atgaaattct gagcacctgt atccatttgg atcattaggg ctcaattgca catggttcaa 1440
   ttacagtggg gtttccccag attttagagt tagaggcagc aggatcagaa aattaaatcc 1500
   atttgcacta ggtaataaat ttgatcccac cctatctcaa aaacaaaaca ctagccacac 1560
   gtggcagcac acacctttta caacaggact caggagcctg gcatgatggg acagaccttt 1620
   actccctgca cttgaggcag atgcaggcaa atctaggcat cctggtgtac atatgaagtt 1680
   caggcaagcc agggccacgt aggctcaaag acg 1713
<210> 11
   <211> 14
   <212> DNA
   <213> Artificial Sequence
<220>
   <223> Antisense oligonucleotide
<400> 11
   ttggtattca caag 14
<210> 12
   <211> 14
   <212> DNA
   <213> Artificial Sequence
<220>
   <223> Antisense oligonucleotide
<400> 12
   aaactggaaa ccta 14
<210> 13
   <211> 14
   <212> DNA
   <213> Artificial Sequence
<220>
   <223> Antisense oligonucleotide
<400> 13
   gatctttatg agaa 14
<210> 14
   <211> 14
   <212> DNA
   <213> Artificial Sequence
<220>
   <223> Antisense oligonucleotide
<400> 14
   gatggagaaa ttgg 14
<210> 15
   <211> 14
   <212> DNA
   <213> Artificial Sequence
<220>
   <223> Antisense oligonucleotide
<400> 15
   agtctgatgg agaa 14
<210> 16
   <211> 14
   <212> DNA
   <213> Artificial Sequence
<220>
   <223> Antisense oligonucleotide
<400> 16
   tgttaaggga tgtc 14
<210> 17
   <211> 14
   <212> DNA
   <213> Artificial Sequence
<220>
   <223> Antisense oligonucleotide
<400> 17
   aatctgcttt tgtt 14
<210> 18
   <211> 14
   <212> DNA
   <213> Artificial Sequence
<220>
   <223> Antisense oligonucleotide
<400> 18
   agggaattga aatc 14
<210> 19
   <211> 14
   <212> DNA
   <213> Artificial Sequence
<220>
   <223> Antisense oligonucleotide
<400> 19
   taaggcaaga tttc 14
<210> 20
   <211> 14
   <212> DNA
   <213> Artificial Sequence
<220>
   <223> Antisense oligonucleotide
<400> 20
   taaatggagt taag 14
<210> 21
   <211> 14
   <212> DNA
   <213> Artificial Sequence
<220>
   <223> Antisense oligonucleotide
<400> 21
   ttatttatag caca 14
<210> 22
   <211> 14
   <212> DNA
   <213> Artificial Sequence
<220>
   <223> Antisense oligonucleotide
<400> 22
   ttgcttctgc ttat 14
<210> 23
   <211> 14
   <212> DNA
   <213> Artificial Sequence
<220>
   <223> Antisense oligonucleotide
<400> 23
   aaaaaaatat ttgc 14
<210> 24
   <211> 14
   <212> DNA
   <213> Artificial Sequence
<220>
   <223> Antisense oligonucleotide
<400> 24
   cagccttaaa aaaa 14
<210> 25
   <211> 14
   <212> DNA
   <213> Artificial Sequence
<220>
   <223> Antisense oligonucleotide
<400> 25
   ttttaaaacc tctc 14
<210> 26
   <211> 14
   <212> DNA
   <213> Artificial Sequence
<220>
   <223> Antisense oligonucleotide
<400> 26
   tagttcagat tttt 14
<210> 27
   <211> 14
   <212> DNA
   <213> Artificial Sequence
<220>
   <223> Antisense oligonucleotide
<400> 27
   agcagttgct aaat 14
<210> 28
   <211> 14
   <212> DNA
   <213> Artificial Sequence
<220>
   <223> Antisense oligonucleotide
<400> 28
   ctgagtgcag cagc 14
<210> 29
   <211> 12
   <212> DNA
   <213> Artificial Sequence
<220>
   <223> Antisense oligonucleotide
<400> 29
   gtctgatgga ga 12
<210> 30
   <211> 12
   <212> DNA
   <213> Artificial Sequence
<220>
   <223> Antisense oligonucleotide
<400> 30
   gtctgatgga ga 12
<210> 31
   <211> 12
   <212> DNA
   <213> Artificial Sequence
<220>
   <223> Antisense oligonucleotide
<400> 31
   gtctgatgga ga 12
<210> 32
   <211> 20
   <212> DNA
   <213> Artificial Sequence
<220>
   <223> Antisense oligonucleotide
<400> 32
   gmumctmgma tmgmgamgma 20
<210> 33
   <211> 24
   <212> DNA
   <213> Artificial Sequence
<220>
   <223> Antisense oligonucleotide
<400> 33
   mgmumcmumg mamumgmgma mgma 24
<210> 34
   <211> 27
   <212> DNA
   <213> Artificial Sequence
<220>
   <223> Antisense oligonucleotide
<400> 34
   actgacacct aattgtattc acatgaa 27
<210> 35
   <211> 26
   <212> DNA
   <213> Artificial Sequence
<220>
   <223> Antisense oligonucleotide
<400> 35
   tgagcagcag ttgctaaatt agttca 26
<210> 36
   <211> 27
   <212> DNA
   <213> Artificial Sequence
<220>
   <223> Antisense oligonucleotide
<400> 36
   tctacctaca ttatatcata gctccta 27
<210> 37
   <211> 19
   <212> DNA
   <213> Artificial Sequence
<220>
   <223> Antisense oligonucleotide
<400> 37
   ttggtattca caagtgaaa 19
<210> 38
   <211> 19
   <212> DNA
   <213> Artificial Sequence
<220>
   <223> Antisense oligonucleotide
<400> 38
   ttgctaaatt agttcagat 19
<210> 39
   <211> 19
   <212> DNA
   <213> Artificial Sequence
<220>
   <223> Antisense oligonucleotide
<400> 39
   gcagcagcag ttgctaaat 19
<210> 40
   <211> 19
   <212> DNA
   <213> Artificial Sequence
<220>
   <223> Antisense oligonucleotide
<400> 40
   gcagttgcta aattagttc 19
<210> 41
   <211> 20
   <212> DNA
   <213> Artificial Sequence
<220>
   <223> Antisense oligonucleotide
<400> 41
   gccatgttgc ccagtccagt 20
<210> 42
   <211> 20
   <212> DNA
   <213> Artificial Sequence
<220>
   <223> Antisense oligonucleotide
<400> 42
   gggctctgct acttacttgc 20
<210> 43
   <211> 20
   <212> DNA
   <213> Artificial Sequence
<220>
   <223> Antisense oligonucleotide
<400> 43
   cccagtcttc agccttgtct 20
<210> 44
   <211> 21
   <212> DNA
   <213> Artificial Sequence
<220>
   <223> Antisense oligonucleotide
<400> 44
   gggtctctgt catatgttct t 21
<210> 45
   <211> 19
   <212> DNA
   <213> Artificial Sequence
<220>
   <223> Antisense oligonucleotide
<400> 45
   ttcctacctt ccctccata 19
<210> 46
   <211> 19
   <212> DNA
   <213> Artificial Sequence
<220>
   <223> Antisense oligonucleotide
<400> 46
   attcctacct tccctccat 19
<210> 47
   <211> 20
   <212> DNA
   <213> Artificial Sequence
<220>
   <223> Antisense oligonucleotide
<400> 47
   ccttagggtt gcagctaatt 20
<210> 48
   <211> 20
   <212> DNA
   <213> Artificial Sequence
<220>
   <223> Antisense oligonucleotide
<400> 48
   atcccagcta ctcaggaggc 20
<210> 49
   <211> 20
   <212> DNA
   <213> Artificial Sequence
<220>
   <223> Antisense oligonucleotide
<400> 49
   tctggctgag tgcagtggct 20
<210> 50
   <211> 20
   <212> DNA
   <213> Artificial Sequence
<220>
   <223> Antisense oligonucleotide
<400> 50
   cctgggagtt ggaggttgca 20
<210> 51
   <211> 21
   <212> DNA
   <213> Artificial Sequence
<220>
   <223> Antisense oligonucleotide
<400> 51
   cagatcccat gaagccaaga g 21
<210> 52
   <211> 21
   <212> DNA
   <213> Artificial Sequence
<220>
   <223> Antisense oligonucleotide
<400> 52
   ctgactgcca tcgagaagtg g 21
<210> 53
   <211> 19
   <212> DNA
   <213> Artificial Sequence
<220>
   <223> Antisense oligonucleotide
<400> 53
   gcccatctgc ttgcttgat 19
<210> 54
   <211> 19
   <212> DNA
   <213> Artificial Sequence
<220>
   <223> Antisense oligonucleotide
<400> 54
   atcctcacca cagtcttgt 19
<210> 55
   <211> 20
   <212> DNA
   <213> Artificial Sequence
<220>
   <223> Antisense oligonucleotide
<400> 55
   gcttacttcc tcctcccttt 20
<210> 56
   <211> 21
   <212> DNA
   <213> Artificial Sequence
<220>
   <223> Antisense oligonucleotide
<400> 56
   ccaggtgata ggagcagaac t 21
<210> 57
   <211> 20
   <212> DNA
   <213> Artificial Sequence
<220>
   <223> Antisense oligonucleotide
<400> 57
   accctccttc ctccctctct 20
<210> 58
   <211> 21
   <212> DNA
   <213> Artificial Sequence
<220>
   <223> Antisense oligonucleotide
<400> 58
   ccactctccc ttctgtcctc t 21
<210> 59
   <211> 20
   <212> DNA
   <213> Artificial Sequence
<220>
   <223> Antisense oligonucleotide
<400> 59
   cctccttcct ccctctctct 20
<210> 60
   <211> 20
   <212> DNA
   <213> Artificial Sequence
<220>
   <223> Antisense oligonucleotide
<400> 60
   gtctgtccca tcatgccagg 20
<210> 61
   <211> 20
   <212> DNA
   <213> Artificial Sequence
<220>
   <223> Antisense oligonucleotide
<400> 61
   tttctgatcc tgctgcctct 20
<210> 62
   <211> 15
   <212> DNA
   <213> Artificial Sequence
<220>
   <223> Antisense oligonucleotide
<400> 62
   accctccttc ctccc 15
<210> 63
   <211> 14
   <212> DNA
   <213> Artificial Sequence
<220>
   <223> Antisense oligonucleotide
<400> 63
   ctccttcctc cctc 14
<210> 64
   <211> 11
   <212> DNA
   <213> Artificial Sequence
<220>
   <223> Antisense oligonucleotide
<400> 64
   ctccttcctc c 11
<210> 65
   <211> 15
   <212> DNA
   <213> Artificial Sequence
<220>
   <223> Antisense oligonucleotide
<400> 65
   cttcctccct ctctc 15
<210> 66
   <211> 14
   <212> DNA
   <213> Artificial Sequence
<220>
   <223> Antisense oligonucleotide
<400> 66
   atcctgctgc ctct 14
<210> 67
   <211> 14
   <212> DNA
   <213> Artificial Sequence
<220>
   <223> Antisense oligonucleotide
<400> 67
   ctccttcctc cctc 14
<210> 68
   <211> 15
   <212> DNA
   <213> Artificial Sequence
<220>
   <223> Antisense oligonucleotide
<400> 68
   accctccttc ctccc 15
<210> 69
   <211> 25
   <212> DNA
   <213> Artificial Sequence
<220>
   <223> Exon 4 of the SIRT1 natural antisense CV396200
<400> 69
   aactggagct ggggtgtctg tttca 25
<210> 70
   <211> 25
   <212> DNA
   <213> Artificial Sequence
<220>
   <223> Forward primer sequence
<400> 70
   ccatcagacg acatccctta acaaa 25
<210> 71
   <211> 31
   <212> DNA
   <213> Artificial Sequence
<220>
   <223> Reverse primer sequence
<400> 71
   acattatatc atagctccta aaggagatgc a 31
<210> 72
   <211> 16
   <212> DNA
   <213> Artificial Sequence
<220>
   <223> Reporter Sequence
<400> 72
   cagagtttca attccc 16
<210> 73
   <211> 12
   <212> DNA
   <213> Artificial Sequence
<220>
   <223> Corresponds to CUR 962
<400> 73
   gctagtctgt tg 12

## Description

Instances of the disclosure comprise oligonucleotides modulating expression and/or function of SIRT1 and associated molecules.

### BACKGROUND

DNA-RNA and RNA-RNA hybridization are important to many aspects of nucleic acid function including DNA replication, transcription, and translation. Hybridization is also central to a variety of technologies that either detect a particular nucleic acid or alter its expression. Antisense nucleotides, for example, disrupt gene expression by hybridizing to target RNA, thereby interfering with RNA splicing, transcription, translation, and replication. Antisense DNA has the added feature that DNA-RNA hybrids serve as a substrate for digestion by ribonuclease H, an activity that is present in most cell types. Antisense molecules can be delivered into cells, as is the case for oligodeoxynucleotides (ODNs), or they can be expressed from endogenous genes as RNA molecules. The FDA recently approved an antisense drug, VITRAVENE™ (for treatment of cytomegalovirus retinitis), reflecting that antisense has therapeutic utility.
WO 2010/065662 A2 relates to treatment of sirtuin 1 (SIRT1) related diseases by inhibition of natural antisense transcript to sirtuin 1.
Bonauer et al. (Science, vol. 324, no. 5935, 26 June 2009, pages 1710-1713) relates to microRNA-92a controlling angiogenesis and functional recovery of ischemic tissues in mice.
DATABASE EMBL [Online] 4 March 2000, "xj35b03.xl Soares_NFL_T_GBC_S1 Homo sapiens cDNA clone IMAGE: 2659181 3', mRNA sequence.", retrieved from EBI accession no. EM_EST:AW169958, database accession no. AW169958.
DATABASE EMBL [Online] 23 September 2008. "Mus musculus adult retina cDNA, RIKEN full-length enriched library, clone:A930026H04 product:hypothetical protein, full insert sequence.", retrieved from EBI accession no. EM_EST:AK044604; database accession no. AK044604.

### SUMMARY

The invention is defined by the claims. Those aspects/instances of the present disclosure which constitute the invention are defined by the claims.

This Summary is provided to present a summary of the disclosure to briefly indicate the nature and substance of the disclosure. It is submitted with the understanding that it will not be used to interpret or limit the scope or meaning of the claims.

In one instance, the disclosure provides methods for inhibiting the action of a natural antisense transcript by using antisense oligonucleotide(s) targeted to any region of the natural antisense transcript resulting in up-regulation of the corresponding sense gene. It is also contemplated herein that inhibition of the natural antisense transcript can be achieved by siRNA, ribozymes and small molecules, which are considered to be within the scope of the present disclosure.

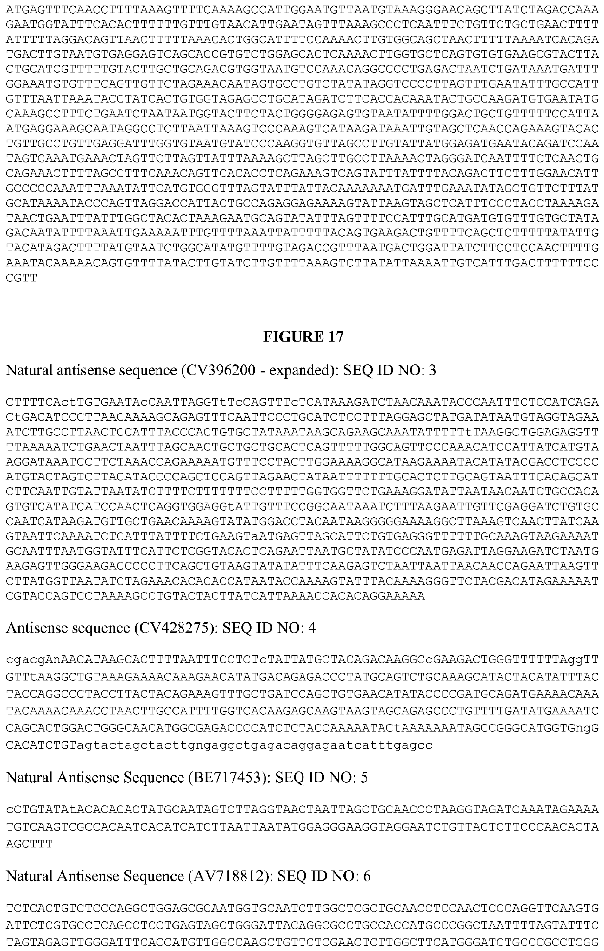

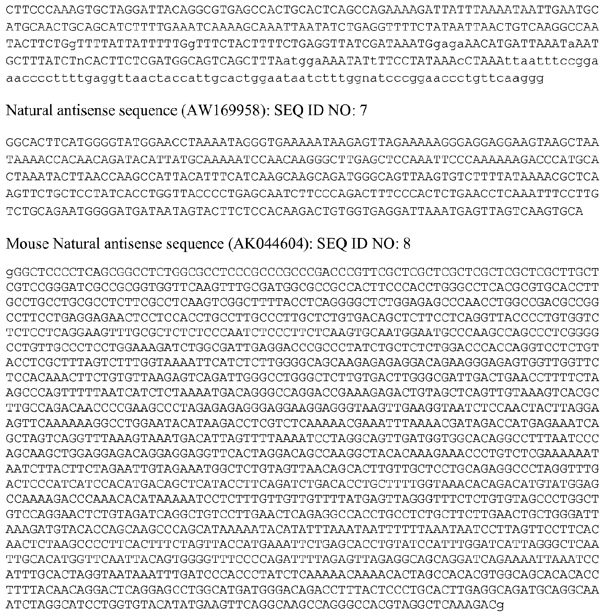

One instance provides a method of modulating function and/or expression of an SIRT1 polynucleotide in patient cells or tissues in vivo or in vitro comprising contacting said cells or tissues with an antisense oligonucleotide 5 to 30 nucleotides in length wherein said oligonucleotide has at least 50% sequence identity to a reverse complement of a polynucleotide comprising 5 to 30 consecutive nucleotides within nucleotides 1 to 1028 of SEQ ID NO: 3 or nucleotides 1 to 429 of SEQ ID NO: 4, or nucleotides 1 to 156 of SEQ ID NO: 5 or nucleotides 1 to 593 of SEQ ID NO:6, 1 to 373 of SEQ ID NO: 7 and 1 to 1713 of SEQ ID NO: 8 (Figure 17) thereby modulating function and/or expression of the SIRT1 polynucleotide in patient cells or tissues *in vivo* or *in vitro.*

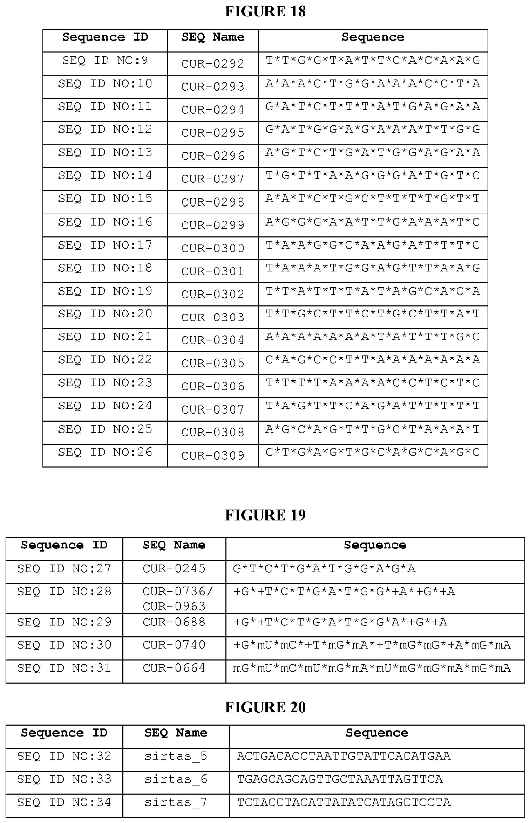

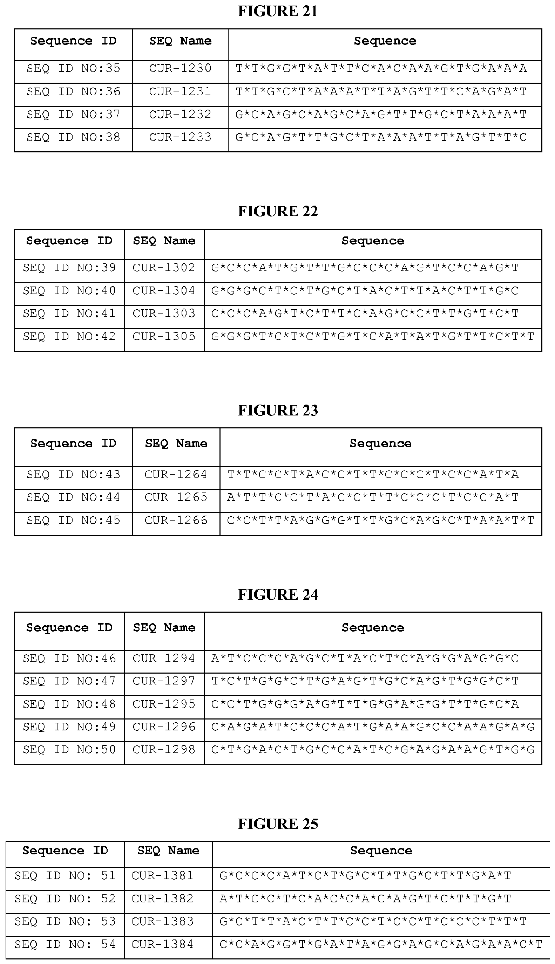

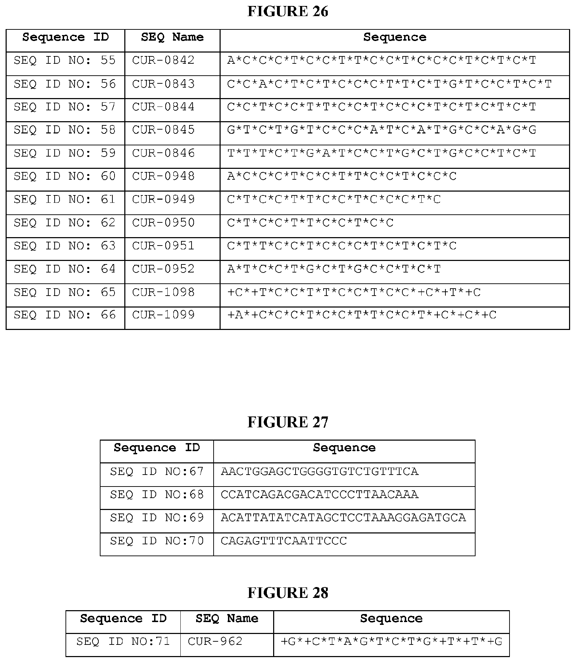

In another preferred instance, an oligonucleotide targets a natural antisense sequence of SIRT1 polynucleotides, for example, nucleotides set forth in SEQ ID NO: 3 to 8, and any variants, alleles, homologs, mutants, derivatives, fragments and complementary sequences thereto. Examples of antisense oligonucleotides are set forth as SEQ ID NOS: 9 to 66 (Figures 19 to 26).

Another instance provides a method of modulating function and/or expression of an SIRT1 polynucleotide in patient cells or tissues in vivo or in vitro comprising contacting said cells or tissues with an antisense oligonucleotide 5 to 30 nucleotides in length wherein said oligonucleotide has at least 50% sequence identity to a reverse complement of the an antisense of the SIRT1 polynucleotide; thereby modulating function and/or expression of the SIRT1 polynucleotide in patient cells or tissues in vivo or in vitro.

Another instance provides a method of modulating function and/or expression of an SIRT1 polynucleotide in patient cells or tissues in vivo or in vitro comprising contacting said cells or tissues with an antisense oligonucleotide 5 to 30 nucleotides in length wherein said oligonucleotide has at least 50% sequence identity to an antisense oligonucleotide to an SIRT1 antisense polynucleotide; thereby modulating function and/or expression of the SIRT1 polynucleotide in patient cells or tissues in vivo or in vitro.

In a preferred instance, a composition comprises one or more antisense oligonucleotides which bind to sense and/or antisense SIRT1 polynucleotides.

In another preferred instance, the oligonucleotides comprise one or more modified or substituted nucleotides.

In another preferred instance, the oligonucleotides comprise one or more modified bonds.

In yet another instance, the modified nucleotides comprise modified bases comprising phosphorothioate, methylphosphonate, peptide nucleic acids, 2'-O-methyl, fluoro-or carbon, methylene or other locked nucleic acid (LNA) molecules. Preferably, the modified nucleotides are locked nucleic acid molecules, including α-L-LNA.

In another preferred instance, the oligonucleotides are administered to a patient subcutaneously, intramuscularly, intravenously or intraperitoneally.

In another preferred instance, the oligonucleotides are administered in a pharmaceutical composition. A treatment regimen comprises administering the antisense compounds at least once to patient; however, this treatment can be modified to include multiple doses over a period of time. The treatment can be combined with one or more other types of therapies.

In another preferred instance, the oligonucleotides are encapsulated in a liposome or attached to a carrier molecule (e.g. cholesterol, TAT peptide).

Other aspects are described *infra.*

### BRIEF DESCRIPTION OF THE DRAWINGS

The novel features of the invention are set forth with particularity in the appended claims. A better understanding of the features and advantages of the present disclosure will be 20 obtained by reference to the following detailed description that sets forth illustrative instances, in which the principles of the disclosure are utilized, and the accompanying drawings of which:

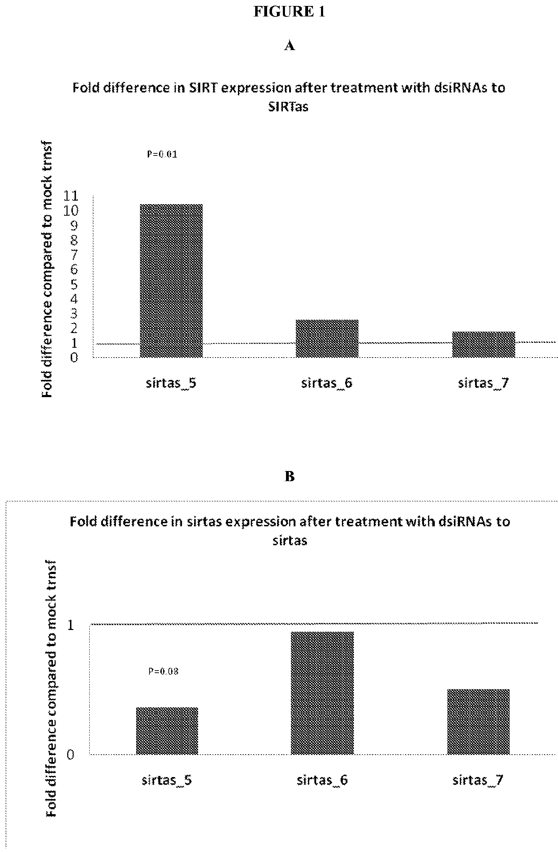

Figure 1 shows Real time PCR results of oligonucleotides designed to SIRT antisense CV396200. The results show that the levels of the SIRT1 mRNA in HepG2 cells are significantly increased 48 h after treatment with one of the siRNAs designed to sirtas (sirtas_5, P=0.01). In the same samples the levels of sirtas RNA were significantly decreased after treatment with sirtas_5, but unchanged after treatment with sirtas_6 and sirtas_7, which also had no effect on the SIRT1 mRNA levels (Fig. 1B). sirtas_5, sirtas_6 and sirtas_7 correspond to SEQ ID NOs: 32, 33 and 34 respectively.

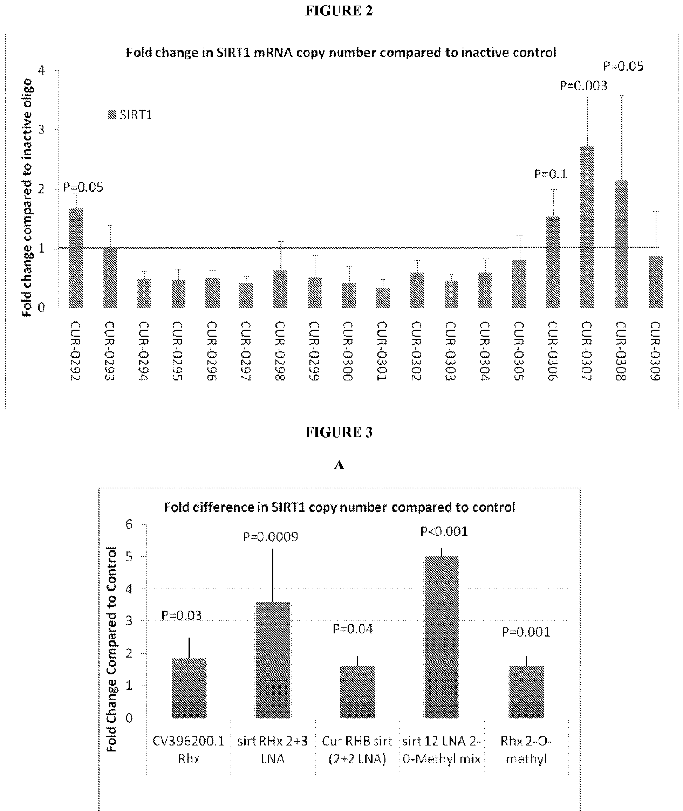

Figure 2 shows results for the oligonucleotide walk across the SIRT antisense CV396200.1. Real time PCR results show that the levels of the SIRT1 mRNA in HepG2 cells are significantly increased 48 h after treatment with three of the antisense oligonucleotides designed to sirtas. CUR-0292 to CUR-0309 correspond to SEQ ID NOs: 9 to 26 respectively.

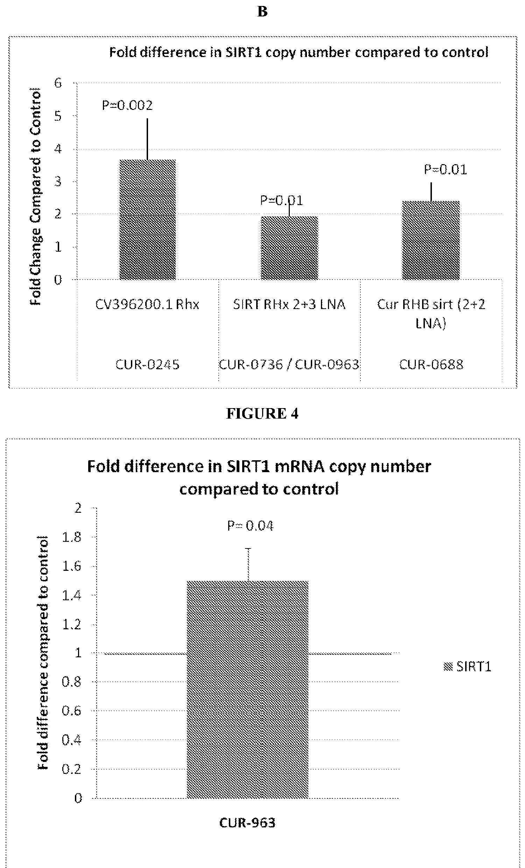

Figure 3 shows results for PS, LNA and 2'O Me Modified oligonucleotides in HepG2 (Fig. 3A) and Vero76 (Fig. 3B) cells. Real time PCR results show that the levels of the SIRT1 mRNA in HepG2 cells are significantly increased 48 h after treatment with PS, LNA, 2'O Me and 2'O Me mixmer designed antisense oligonucleotides to SIRT1 antisense. Levels of SIRT1 mRNA in Vero cells also increased 48 hours after treatment with PS and LNA modified antisense oligonucleotides to SIRT1 antisense. Bars denoted as CUR-0245, CUR-0736, CUR 0688, CUR-0740 and CUR-0664 correspond to SEQ ID NOs: 27 to 31 respectively.
Figure 4 shows PCR results of Monkey Fat Biopsies. Real time PCR results show an increase in SIRT1 mRNA levels in fat biopsies from monkeys dosed with CUR-963, an oligonucleotide designed to SIRT1 antisense CV396200.1. CUR-963 corresponds to SEQ ID NO: 28.

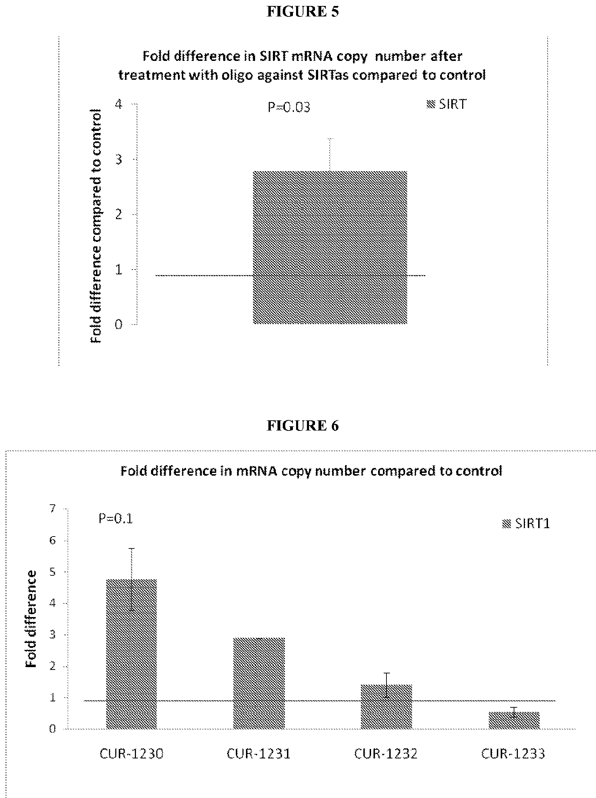

Figure 5 shows PCR results of primary monkey liver hepatocytes. Real time PCR results show an increase in SIRT1 mRNA levels after treatment with an oligonucleotide against SIRT1 antisense. Bar denoted as CUR-0245 corresponds to SEQ ID NO: 27.
Figure 6 shows results for oligonucleotides designed to SIRT antisense CV396200. Real Time PCR results show that levels of SIRT1 mRNA in HepG2 cells are significantly increased in one of the oligonucleotides designed to SIRT1 antisense CV396200. The bars denoted as CUR-1230, CUR-1231, CUR-1232 and CUR-1233 correspond to SEQ ID NOs: 35 to 38.

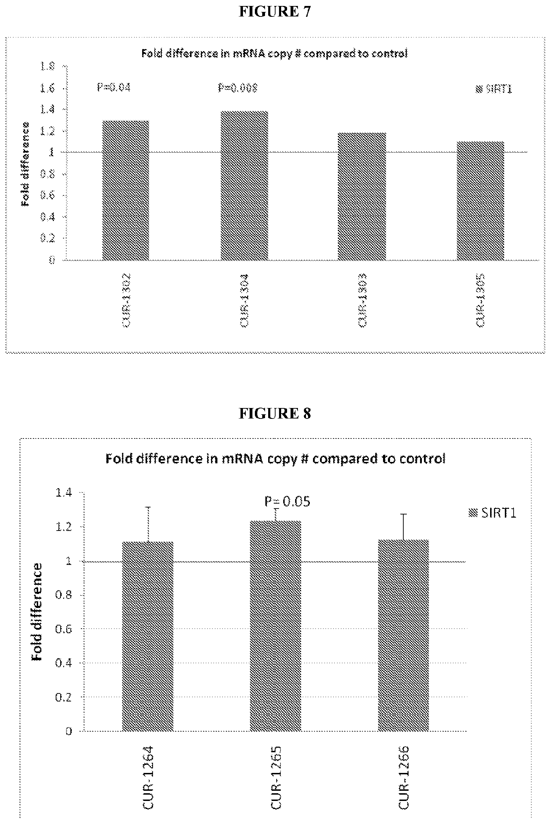

Figure 7 shows results for oligonucleotides designed to SIRT antisense CV428275. Real Time PCR results show that levels of SIRT1 mRNA in HepG2 cells are significantly increased in two of the oligonucleotides designed to SIRT1 antisense CV428275. The bars denoted as CUR-1302, CUR-1304, CUR-1303 and CUR-1305 correspond to SEQ ID NOs: 39 to 42.
Figure 8 shows Real time PCR results. The results show that a significant increase in SIRT1 mRNA levels in HepG2 cells 48 hours after treatment with one of the oligonucleotides designed to SIRT antisense BE717453. The bars denoted as CUR-1264, CUR1265 and CUR-1266 correspond to SEQ ID NOs: 43 to 45 respectively.

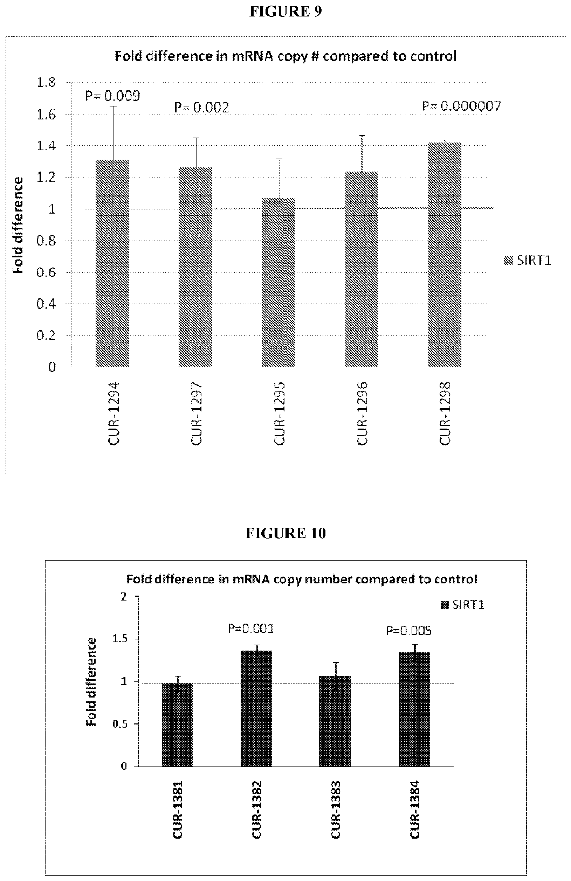

Figure 9 shows Real time PCR results. The results show that show that the levels of the SIRT1 mRNA in HepG2 cells are significantly increased 48 h after treatment with three of the oligonucleotides designed to SIRT1 antisense AV718812. The bars denoted as CUR-1294, CUR-1297, CUR-1295, CUR-1296 and CUR-1298 correspond to SEQ ID NOs: 46 to 50 respectively.
Figure 10 is a graph of real time PCR results showing the fold change + standard deviation in SIRT1 mRNA after treatment of HepG2 cells with phosphorothioate oligonucleotides introduced using Lipofectamine 2000, as compared to control. Real time PCR results show that the levels of SIRT1 mRNA are significantly increased in HepG2 cells 48 h after treatment with two of the oligos designed to SIRT1 antisense AW169958. Bars denoted as CUR-1381, CUR-1382, CUR-1383 and CUR-1384 correspond to samples treated with SEQ ID NOS: 51, 52, 53 and 54 respectively.

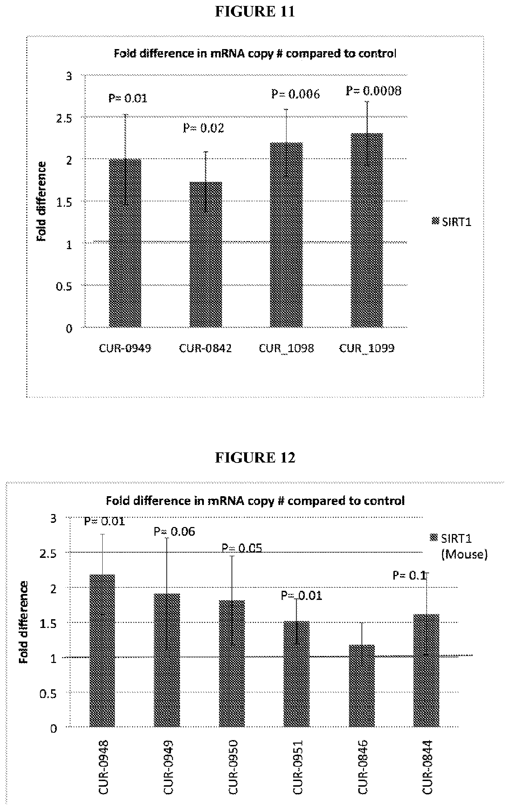

Figure 11 is a graph of real time PCR results showing the fold change + standard deviation in SIRT1 mRNA after treatment of 3T3 cells with phosphorothioate oligonucleotides introduced using Lipofectamine 2000, as compared to control. Real time PCR results show that the levels of SIRT1 mRNA are significantly increased in 3T3 cells 48 h after treatment with three of the oligonucleotides designed to SIRT1 mouse antisense AK044604. Bars denoted as CUR-0949, CUR-0842, CUR-1098 and CUR-1099 correspond to samples treated with SEQ ID NOS: 61, 55, 65 and 66 respectively.
Figure 12 is a graph of real time PCR results showing the fold change + standard deviation in SIRT1 mRNA after treatment of 3T3 cells with phosphorothioate oligonucleotides introduced using Lipofectamine 2000, as compared to control. Real time PCR results show that the levels of SIRT1 mRNA are significantly increased in 3T3 cells 48 h after treatment with five of the oligonucleotides designed to SIRT1 mouse antisense AK044604. Bars denoted as CUR-0948, CUR-0949, CUR-0950, CUR-0951, CUR-0846, and CUR-0844 correspond to samples treated with SEQ ID NOS: 60, 61, 62, 63, 59 and 57 respectively.

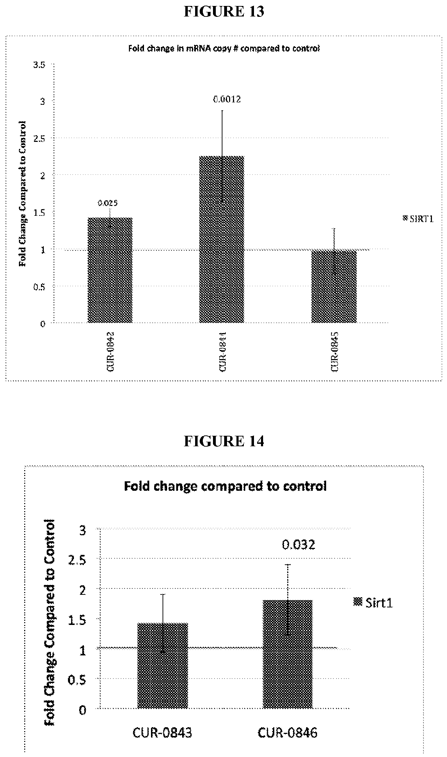

Figure 13 is a graph of real time PCR results showing the fold change + standard deviation in SIRT1 mRNA after treatment of 3T3 cells with phosphorothioate oligonucleotides introduced using Lipofectamine 2000, as compared to control. Real time PCR results show that the levels of SIRT1 mRNA are significantly increased in HepG2 cells 48 h after treatment with two of the oligonucleotides designed to SIRT1 mouse antisense AK044604. Bars denoted as CUR-0842, CUR-0844, and CUR-0845 correspond to samples treated with SEQ ID NOS: 55, 57 and 58 respectively.
Figure 14 is a graph of real time PCR results showing the fold change + standard deviation in SIRT1 mRNA after treatment of 3T3 cells with phosphorothioate oligonucleotides introduced using Lipofectamine 2000, as compared to control. Real time PCR results show that the levels of SIRT1 mRNA are significantly increased in HepG2 cells 48 h after treatment with two of the oligonucleotides designed to SIRT1 mouse antisense AK044604. Bars denoted as CUR-0843, CUR-0846 correspond to samples treated with SEQ ID NOS: 56 and 59 respectively.

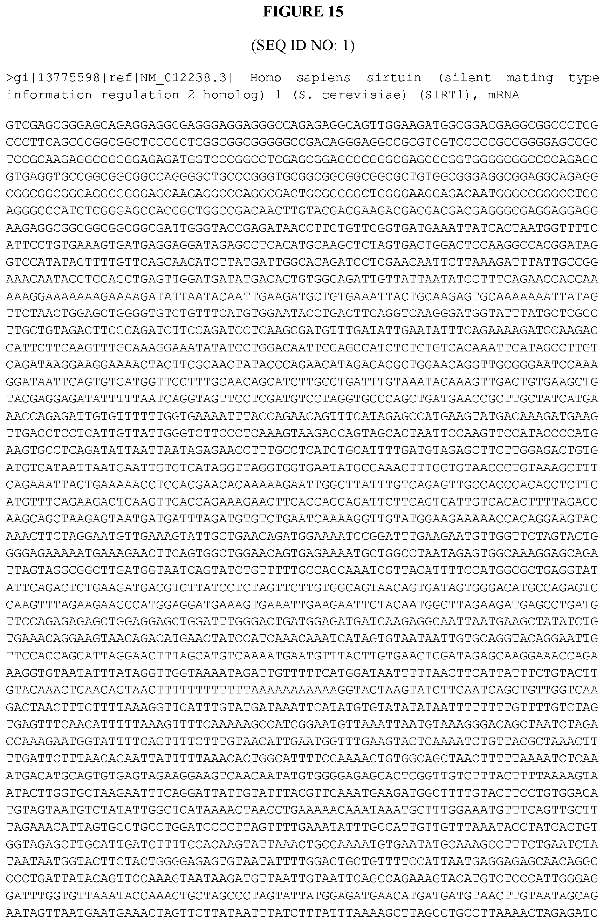

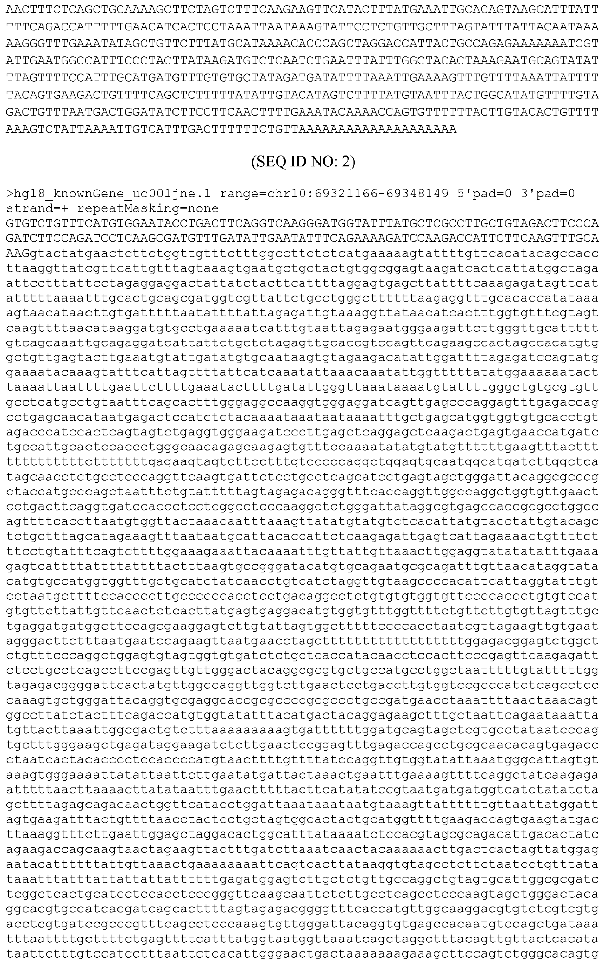

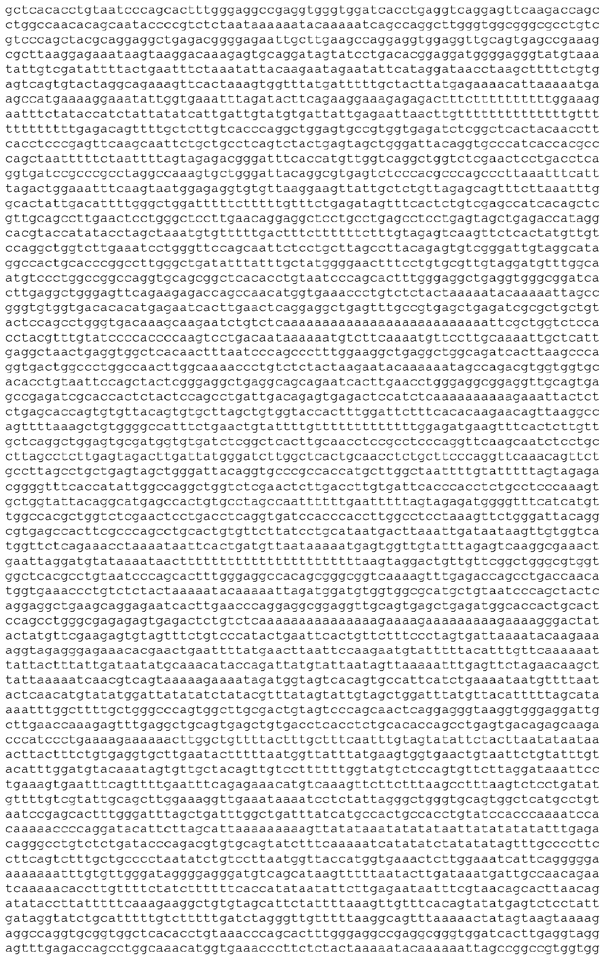

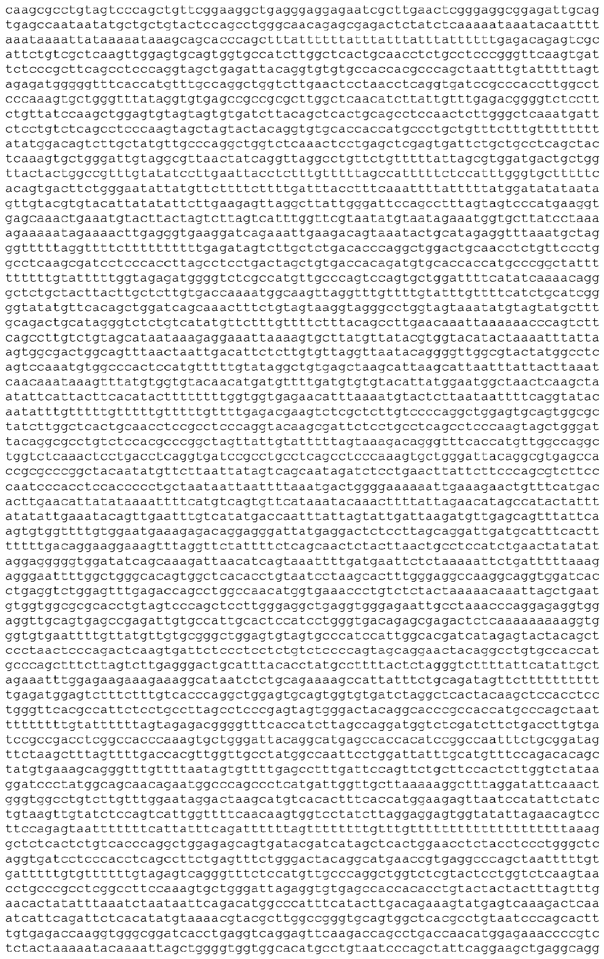

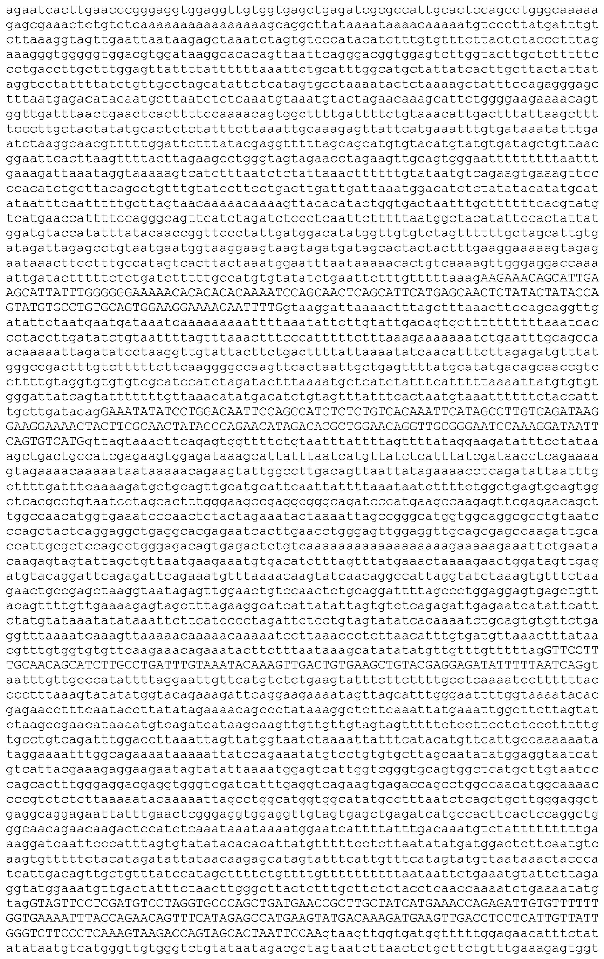

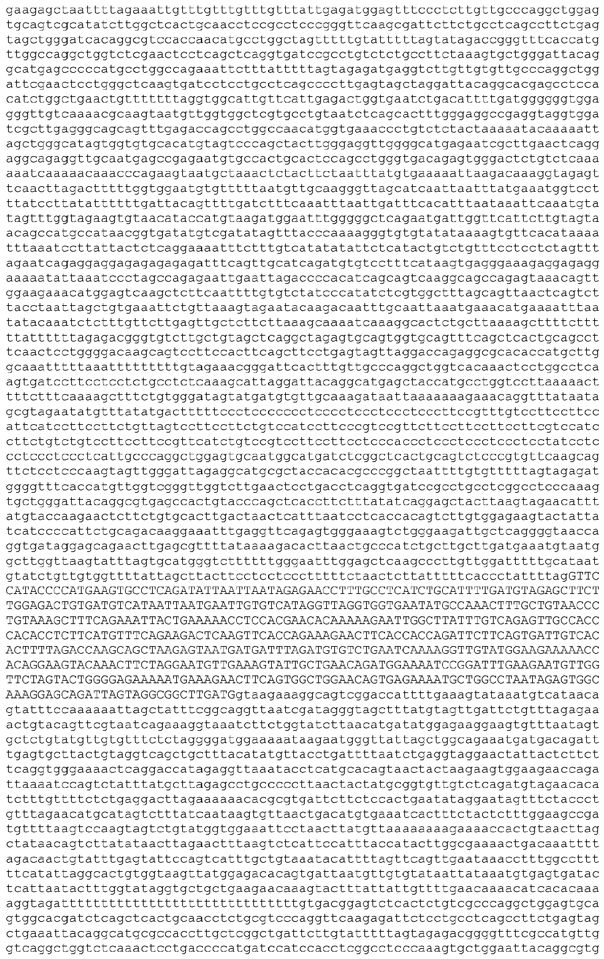

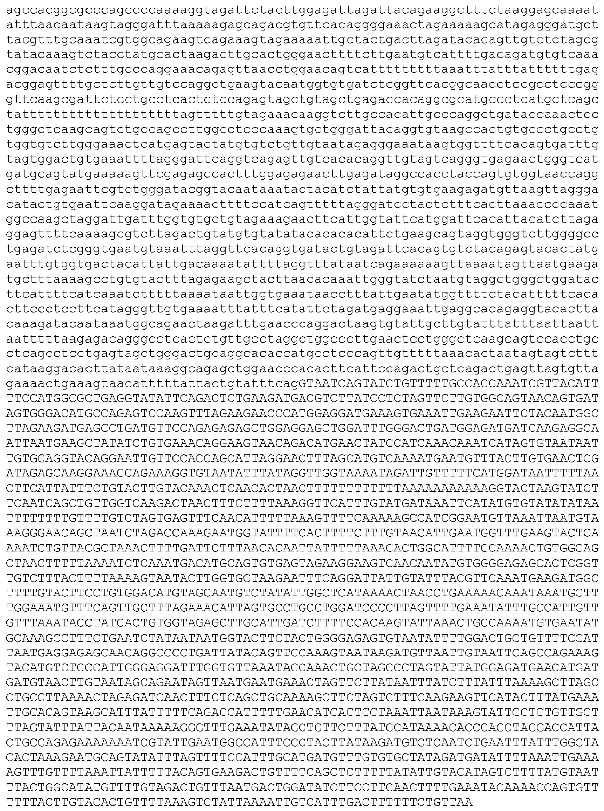

Figure 15 shows
   SEQ ID NO: 1: Homo sapiens sirtuin (silent mating type information regulation 2 homolog) 1 (S. cerevisiae) (SIRT1), mRNA (NCBI Accession Number: NM_012238.3)
   SEQ ID NO: 2: Genomic sequence of SIRT (exons are shown in capital letters, introns in small).

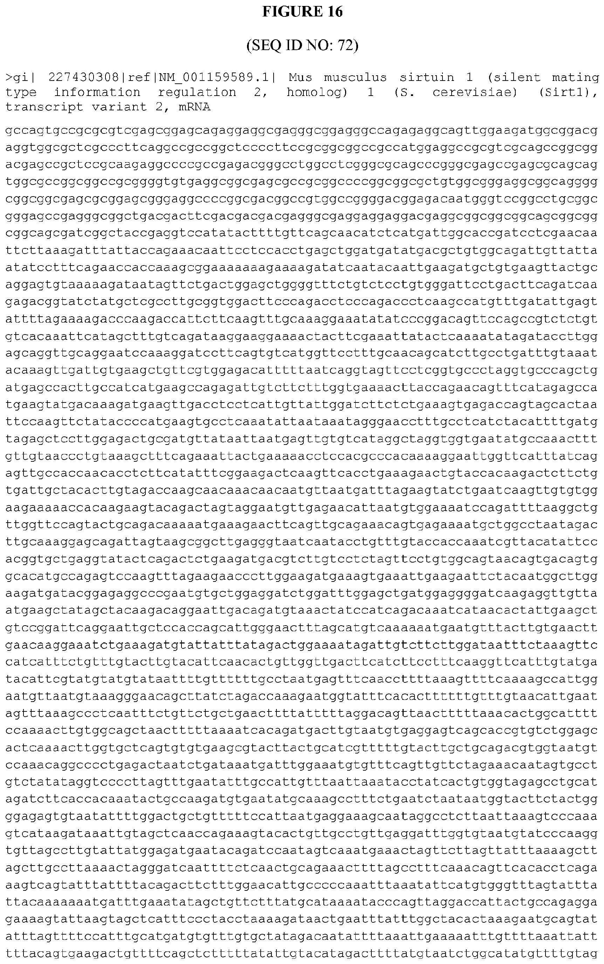

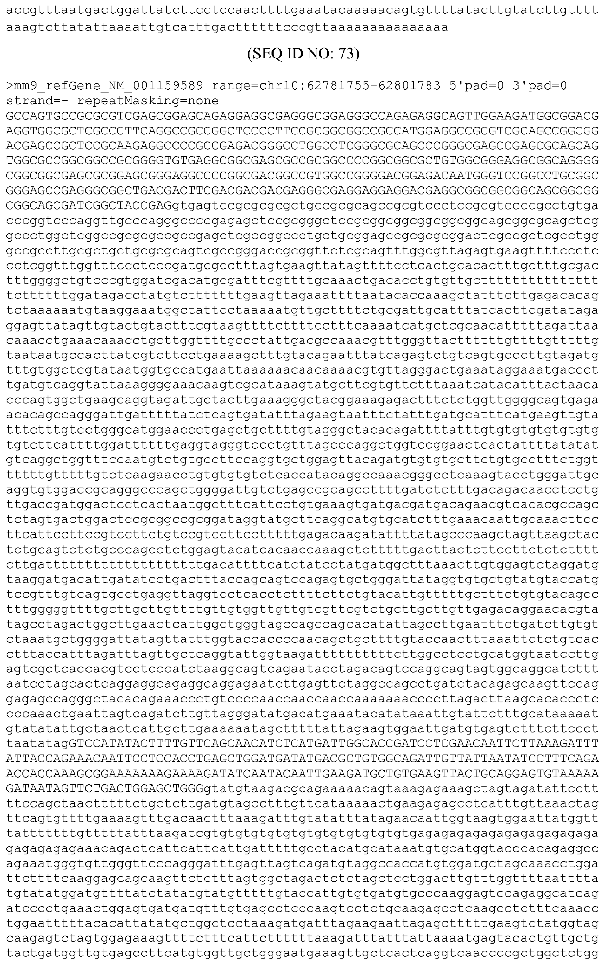

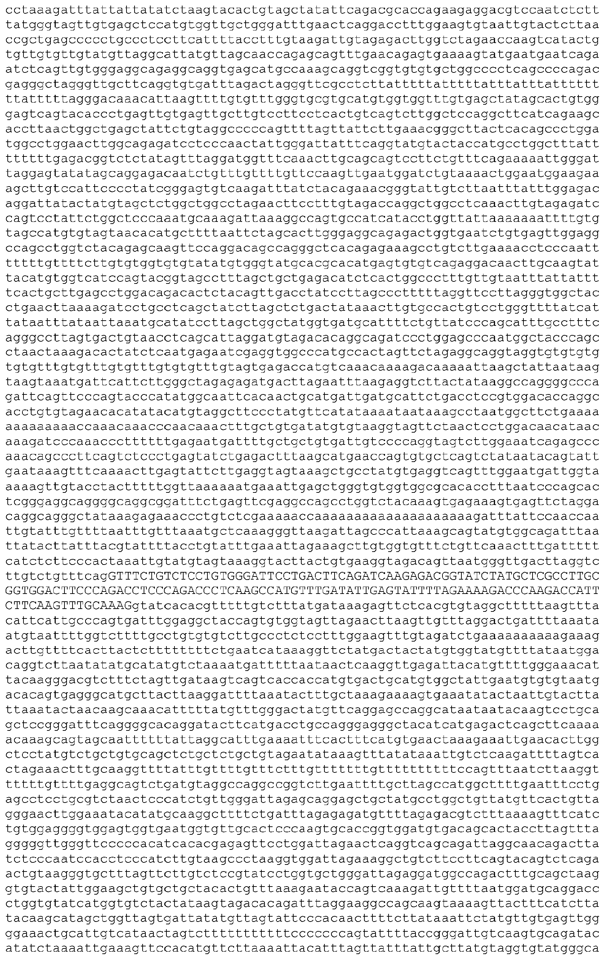

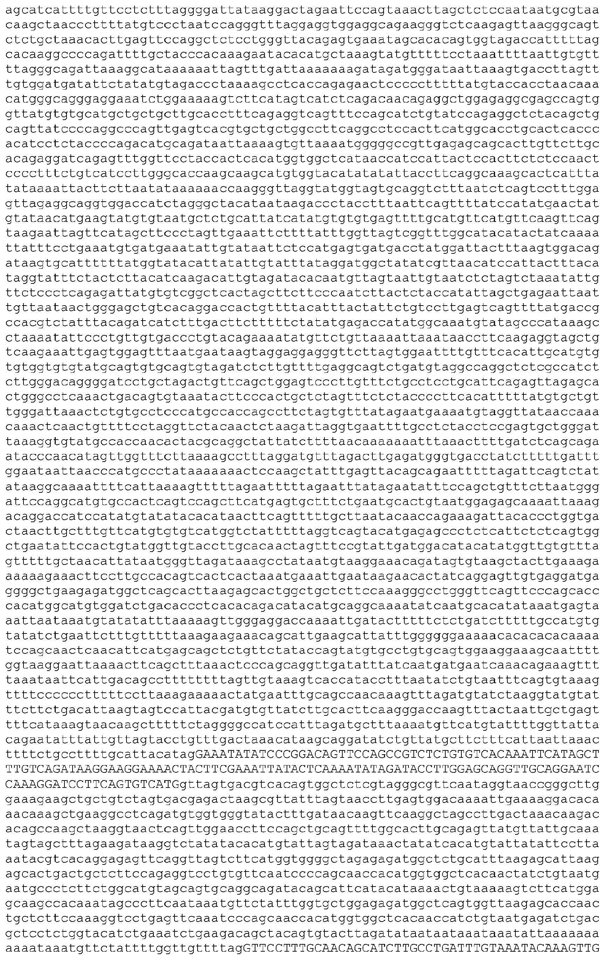

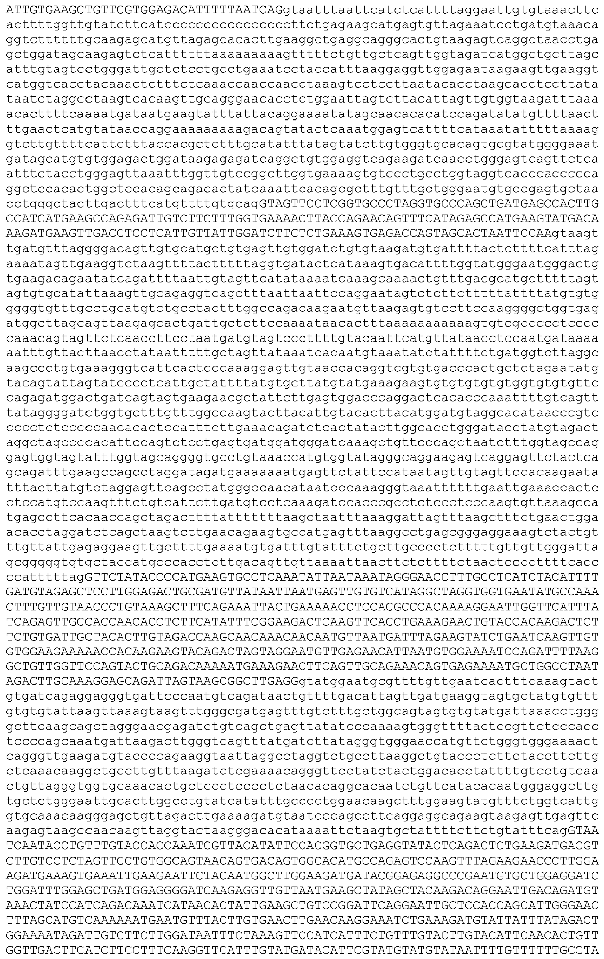

Figure 16 shows
   SEQ ID NO: 72: Mus musculus sirtuin 1 (silent mating type information regulation 2, homolog) 1 (S. cerevisiae) (SIRT1) mRNA (NCBI Accession Number: NM_001159589)
   SEQ ID NO: 73: Genomic sequence of SIRT (exons are shown in capital letters, introns in small).
Figure 17 shows
   SEQ ID NO: 3: Expanded natural antisense sequence (CV396200 - expanded).
   SEQ ID NO: 4 Natural antisense sequence (CV428275)
   SEQ ID NO: 5: Natural antisense sequence (BE717453)
   SEQ ID NO: 6: Natural Antisense sequence (AV718812)
   SEQ ID NO: 7: Natural Antisense Sequence (AW169958)
   SEQ ID NO: 8: Mouse Natural Antisense Sequence (AK044604)
Figure 18 shows SEQ ID NOs: 9 to 26,* indicates phosphothioate bond
Figure 19 shows SEQ ID NOs: 27 to 31, * indicates phosphothioate bond, + indicates LNA and m indicates 2'O Me
Figure 20 shows SEQ ID NOs: 32 to 34, the double stranded test oligonucleotides designed to SIRT antisense CV396200 which correspond to sirtas_5, sirtas_6 and sirtas_7 respectively
Figure 21 shows SEQ ID NOs: 35 to 38 designed to SIRT1 antisense CV396200.
Figure 22 shows SEQ ID NOs: 39 to 42 designed to SIRT1 antisense CV428275.
Figure 23 shows SEQ ID NOs: 43 to 45 designed to SIRT1 antisense BE717453.
Figure 24 shows SEQ ID NOs: 46 to50 designed to SIRT1 antisense AV718812.
Figure 25 shows the antisense oligonucleotides, SEQ ID NOs: 51 to 54. * indicates phosphothioate bond.
Figure 26 shows the antisense oligonucleotides, SEQ ID NOs: 55 to 66. * indicates phosphothioate bond, + indicates LNA
Figure 27 shows SEQ ID NO: 67 to 70. SEQ ID NO: 67 correspond to the exon 4 of the SIRT1 natural antisense CV396200, SEQ ID NO: 68, 69 and 70 correspond to the forward primer sequence, reverse primer sequence and the reporter sequence respectively.
Figure 28 shows SEQ ID NO: 71 that correspond to CUR 962, * indicates phosphothioate bond and + indicates LNA.

### DETAILED DESCRIPTION

Several aspects of the disclosure are described below with reference to example applications for illustration. It should be understood that numerous specific details, relationships, and methods are set forth to provide a full understanding of the disclosure. One having ordinary skill in the relevant art, however, will readily recognize that the disclosure can be practiced without one or more of the specific details or with other methods. The present disclosure is not limited by the ordering of acts or events, as some acts may occur in different orders and/or concurrently with other acts or events. Furthermore, not all illustrated acts or events are required to implement a methodology in accordance with the present disclosure.

All genes, gene names, and gene products disclosed herein are intended to correspond to homologs from any species for which the compositions and methods disclosed herein are applicable. Thus, the terms include, but are not limited to genes and gene products from humans and mice. It is understood that when a gene or gene product from a particular species is disclosed, this disclosure is intended to be exemplary only, and is not to be interpreted as a limitation unless the context in which it appears clearly indicates. Thus, for example, for the genes disclosed herein, which in some instances relate to mammalian nucleic acid and amino acid sequences are intended to encompass homologous and/or orthologous genes and gene products from other animals including, but not limited to other mammals, fish, amphibians, reptiles, and birds. In preferred instances, the genes or nucleic acid sequences are human.

### Definitions

The terminology used herein is for the purpose of describing particular instances only and is not intended to be limiting of the disclosure. As used herein, the singular forms "a", "an" and "the" are intended to include the plural forms as well, unless the context clearly indicates otherwise. Furthermore, to the extent that the terms "including", "includes", "having", "has", "with", or variants thereof are used in either the detailed description and/or the claims, such terms are intended to be inclusive in a manner similar to the term "comprising."

The term "about" or "approximately" means within an acceptable error range for the particular value as determined by one of ordinary skill in the art, which will depend in part on how the value is measured or determined, i.e., the limitations of the measurement system. For example, "about" can mean within 1 or more than 1 standard deviation, per the practice in the art. Alternatively, "about" can mean a range of up to 20%, preferably up to 10%, more preferably up to 5%, and more preferably still up to 1% of a given value. Alternatively, particularly with respect to biological systems or processes, the term can mean within an order of magnitude, preferably within 5-fold, and more preferably within 2-fold, of a value. Where particular values are described in the application and claims, unless otherwise stated the term "about" meaning within an acceptable error range for the particular value should be assumed.

As used herein, the term "mRNA" means the presently known mRNA transcript(s) of a targeted gene, and any further transcripts which may be elucidated.

By "antisense oligonucleotides" or "antisense compound" is meant an RNA or DNA molecule that binds to another RNA or DNA (target RNA, DNA). For example, if it is an RNA oligonucleotide it binds to another RNA target by means of RNA-RNA interactions and alters the activity of the target RNA (Eguchi et al., (1991) Ann. Rev. Biochem. 60, 631-652). An antisense oligonucleotide can upregulate or downregulate expression and/or function of a particular polynucleotide. The definition is meant to include any foreign RNA or DNA molecule which is useful from a therapeutic, diagnostic, or other viewpoint. Such molecules include, for example, antisense RNA or DNA molecules, interference RNA (RNAi), micro RNA, decoy RNA molecules, siRNA, enzymatic RNA, therapeutic editing RNA and agonist and antagonist RNA, antisense oligomeric compounds, antisense oligonucleotides, external guide sequence (EGS) oligonucleotides, alternate splicers, primers, probes, and other oligomeric compounds that hybridize to at least a portion of the target nucleic acid. As such, these compounds may be introduced in the form of single-stranded, double-stranded, partially singlc-strandcd, or circular oligomeric compounds.

In the context of this disclosure, the term "oligonucleotide" refers to an oligomer or polymer of ribonucleic acid (RNA) or deoxyribonucleic acid (DNA) or mimetics thereof. The term "oligonucleotide", also includes linear or circular oligomers of natural and/or modified monomers or linkages, including deoxyribonucleosides, ribonucleosides, substituted and alpha-anomeric forms thereof, peptide nucleic acids (PNA), locked nucleic acids (LNA), phosphorothioate, methylphosphonate, and the like. Oligonucleotides are capable of specifically binding to a target polynucleotide by way of a regular pattern of monomer-to-monomer interactions, such as Watson-Crick type of base pairing, Hoogsteen or reverse Hoogsteen types of base pairing, or the like.

The oligonucleotide may be "chimeric", that is, composed of different regions. In the context of this disclosure "chimeric" compounds are oligonucleotides, which contain two or more chemical regions, for example, DNA region(s), RNA region(s), PNA region(s) etc. Each chemical region is made up of at least one monomer unit, i.e., a nucleotide in the case of an oligonucleotides compound. These oligonucleotides typically comprise at least one region wherein the oligonucleotide is modified in order to exhibit one or more desired properties. The desired properties of the oligonucleotide include, but are not limited, for example, to increased resistance to nuclease degradation, increased cellular uptake, and/or increased binding affinity for the target nucleic acid. Different regions of the oligonucleotide may therefore have different properties. The chimeric oligonucleotides of the present disclosure can be formed as mixed structures of two or more oligonucleotides, modified oligonucleotides, oligonucleosides and/or oligonucleotide analogs as described above.

The oligonucleotide can be composed of regions that can be linked in "register", that is, when the monomers are linked consecutively, as in native DNA, or linked via spacers. The spacers are intended to constitute a covalent "bridge" between the regions and have in preferred cases a length not exceeding about 100 carbon atoms. The spacers may carry different functionalities, for example, having positive or negative charge, carry special nucleic acid binding properties (intercalators, groove binders, toxins, fluorophors etc.), being lipophilic, inducing special secondary structures like, for example, alanine containing peptides that induce alpha-helices.

As used herein "SIRT1" and "Sirtuin 1" are inclusive of all family members, mutants, alleles, fragments, species, coding and noncoding sequences, sense and antisense polynucleotide strands, etc.

As used herein "SIRT1" shall refer to Silencing mating type information regulator 2 homolog and is a member of the SIRTuin deacetylase protein family. The amino acid sequence of SIRT1 may be found at Genbank Accession number NP.sub.--08509. SIRT1 is the human homolog of the yeast Sir2 protein and exhibits NAD-dependent deacetylase activity.

As used herein, the words Sirtuin1, SIRT1, sirtuin, silent mating type information regulation 2 homolog 1, hSIR2, hSIRT1, NAD-dependent deacetylase sirtuin-1, SIR2L1, SIR2-like protein 1, are considered the same in the literature and are used interchangeably in the present application.

As used herein, the term "oligonucleotide specific for" or "oligonucleotide which targets" refers to an oligonucleotide having a sequence (i) capable of forming a stable complex with a portion of the targeted gene, or (ii) capable of forming a stable duplex with a portion of a mRNA transcript of the targeted gene. Stability of the complexes and duplexes can be determined by theoretical calculations and/or in vitro assays. Exemplary assays for determining stability of hybridization complexes and duplexes are described in the Examples below.

As used herein, the term "target nucleic acid" encompasses DNA, RNA (comprising premRNA and mRNA) transcribed from such DNA, and also cDNA derived from such RNA, coding, noncoding sequences, sense or antisense polynucleotides. The specific hybridization of an oligomeric compound with its target nucleic acid interferes with the normal function of the nucleic acid. This modulation of function of a target nucleic acid by compounds, which specifically hybridize to it, is generally referred to as "antisense". The functions of DNA to be interfered include, for example, replication and transcription. The functions of RNA to be interfered, include all vital functions such as, for example, translocation of the RNA to the site of protein translation, translation of protein from the RNA, splicing of the RNA to yield one or more mRNA species, and catalytic activity which may be engaged in or facilitated by the RNA. The overall effect of such interference with target nucleic acid function is modulation of the expression of an encoded product or oligonucleotides.

RNA interference "RNAi" is mediated by double stranded RNA (dsRNA) molecules that have sequence-specific homology to their "target" nucleic acid sequences (Caplen, N. J., et al. (2001) Proc. Natl. Acad. Sci. USA 98:9742-9747). In certain instances of the present disclosure, the mediators are 5-25 nucleotide "small interfering" RNA duplexes (siRNAs). The siRNAs are derived from the processing of dsRNA by an RNase enzyme known as Dicer (Bernstein, E., et al. (2001) Nature 409:363-366). siRNA duplex products are recruited into a multi-protein siRNA complex termed RISC (RNA Induced Silencing Complex). Without wishing to be bound by any particular theory, a RISC is then believed to be guided to a target nucleic acid (suitably mRNA), where the siRNA duplex interacts in a sequence-specific way to mediate cleavage in a catalytic fashion (Bernstein, E., et al. (2001) Nature 409:363-366; Boutla, A., et al. (2001) Curr. Biol. 11:1776-1780). Small interfering RNAs that can be used in accordance with the present disclosure can be synthesized and used according to procedures that are well known in the art and that will be familiar to the ordinarily skilled artisan. Small interfering RNAs for use in the methods of the present disclosure suitably comprise between about 1 to about 50 nucleotides (nt). In examples of non limiting instances, siRNAs can comprise about 5 to about 40 nt, about 5 to about 30 nt, about 10 to about 30 nt, about 15 to about 25 nt, or about 20-25 nucleotides.

Selection of appropriate oligonucleotides is facilitated by using computer programs that automatically align nucleic acid sequences and indicate regions of identity or homology. Such programs are used to compare nucleic acid sequences obtained, for example, by searching databases such as GenBank or by sequencing PCR products. Comparison of nucleic acid sequences from a range of species allows the selection of nucleic acid sequences that display an appropriate degree of identity between species. In the case of genes that have not been sequenced, Southern blots are performed to allow a determination of the degree of identity between genes in target species and other species. By performing Southern blots at varying degrees of stringency, as is well known in the art, it is possible to obtain an approximate measure of identity. These procedures allow the selection of oligonucleotides that exhibit a high degree of complementarity to target nucleic acid sequences in a subject to be controlled and a lower degree of complementarity to corresponding nucleic acid sequences in other species. One skilled in the art will realize that there is considerable latitude in selecting appropriate regions of genes for use in the present disclosure.

By "enzymatic RNA" is meant an RNA molecule with enzymatic activity (Cech, (1988) J. American. Med. Assoc. 260, 3030-3035). Enzymatic nucleic acids (ribozymes) act by first binding to a target RNA. Such binding occurs through the target binding portion of an enzymatic nucleic acid which is held in close proximity to an enzymatic portion of the molecule that acts to cleave the target RNA. Thus, the enzymatic nucleic acid first recognizes and then binds a target RNA through base pairing, and once bound to the correct site, acts enzymatically to cut the target RNA.

By "decoy RNA" is meant an RNA molecule that mimics the natural binding domain for a ligand. The decoy RNA therefore competes with natural binding target for the binding of a specific ligand. For example, it has been shown that over-expression of HIV trans-activation response (TAR) RNA can act as a "decoy" and efficiently binds HIV tat protein, thereby preventing it from binding to TAR sequences encoded in the HIV RNA (Sullenger et al. (1990) Cell, 63, 601- 608). This is meant to be a specific example. Those in the art will recognize that this is but one example, and other instances can be readily generated using techniques generally known in the art.

As used herein, the term "monomers" typically indicates monomers linked by phosphodiester bonds or analogs thereof to form oligonucleotides ranging in size from a few monomeric units, e.g., from about 3-4, to about several hundreds of monomeric units. Analogs of phosphodiester linkages include: phosphorothioate, phosphorodithioate, methylphosphornates, phosphoroselenoate, phosphoramidate, and the like, as more fully described below.

The term "nucleotide" covers naturally occurring nucleotides as well as nonnaturally occurring nucleotides. It should be clear to the person skilled in the art that various nucleotides which previously have been considered "non-naturally occurring" have subsequently been found in nature. Thus, "nucleotides" includes not only the known purine and pyrimidine heterocycles-containing molecules, but also heterocyclic analogues and tautomers thereof. Illustrative examples of other types of nucleotides are molecules containing adenine, guanine, thymine, cytosine, uracil, purine, xanthine, diaminopurine, 8-oxo- N6-methyladenine, 7-deazaxanthine, 7-deazaguanine, N4,N4-ethanocytosin, N6,N6-ethano-2,6-diaminopurine, 5-methylcytosine, 5-(C3-C6)-alkynylcytosine, 5-fluorouracil, 5-bromouracil, pseudoisocytosine, 2-hydroxy-5-methyl-4-triazolopyridin, isocytosine, isoguanin, inosine and the "non-naturally occurring" nucleotides described in Benner et al., U.S. Pat No. 5,432,272. The term "nucleotide" is intended to cover every and all of these examples as well as analogues and tautomers thereof. Especially interesting nucleotides are those containing adenine, guanine, thymine, cytosine, and uracil, which are considered as the naturally occurring nucleotides in relation to therapeutic and diagnostic application in humans. Nucleotides include the natural 2'-deoxy and 2'- hydroxyl sugars, e.g., as described in Kornberg and Baker, DNA Replication, 2nd Ed. (Freeman, San Francisco, 1992) as well as their analogs.

"Analogs" in reference to nucleotides includes synthetic nucleotides having modified base moieties and/or modified sugar moieties (see e.g., described generally by Scheit, Nucleotide Analogs, John Wiley, New York, 1980; Freier & Altmann, (1997) Nucl. Acid. Res., 25(22), 4429- 4443, Toulmé, J.J., (2001) Nature Biotechnology 19:17-18; Manoharan M., (1999) Biochemica et Biophysica Acta 1489:117-139; Freier S. M., (1997) Nucleic Acid Research, 25:4429-4443, Uhlman, E., (2000) Drug Discovery & Development, 3: 203-213, Herdewin P., (2000) Antisense & Nucleic Acid Drug Dev., 10:297-310); 2'-O, 3'-C-linked [3.2.0] bicycloarabinonucleosides (see e.g. N.K Christiensen., et al, (1998) J. Am. Chem. Soc., 120: 5458-5463; Prakash TP, Bhat B. (2007) Curr Top Med Chem. 7(7):641-9; Cho EJ, et al. (2009) Annual Review of Analytical Chemistry, 2, 241-264). Such analogs include synthetic nucleotides designed to enhance binding properties, e.g., duplex or triplex stability, specificity, or the like.

As used herein, "hybridization" means the pairing of substantially complementary strands of oligomeric compounds. One mechanism of pairing involves hydrogen bonding, which may be Watson-Crick, Hoogsteen or reversed Hoogsteen hydrogen bonding, between complementary nucleoside or nucleotide bases (nucleotides) of the strands of oligomeric compounds. For example, adenine and thymine are complementary nucleotides which pair through the formation of hydrogen bonds. Hybridization can occur under varying circumstances.

An antisense compound is "specifically hybridizable" when binding of the compound to the target nucleic acid interferes with the normal function of the target nucleic acid to cause a modulation of function and/or activity, and there is a sufficient degree of complementarity to avoid non-specific binding of the antisense compound to non-target nucleic acid sequences under conditions in which specific binding is desired, i.e., under physiological conditions in the case of in vivo assays or therapeutic treatment, and under conditions in which assays are performed in the case of in vitro assays.

As used herein, the phrase "stringent hybridization conditions" or "stringent conditions" refers to conditions under which a compound of the disclosure will hybridize to its target sequence, but to a minimal number of other sequences. Stringent conditions are sequence-dependent and will be different in different circumstances and in the context of this disclosure, "stringent conditions" under which oligomeric compounds hybridize to a target sequence are determined by the nature and composition of the oligomeric compounds and the assays in which they are being investigated. In general, stringent hybridization conditions comprise low concentrations (<0.15M) of salts with inorganic cations such as Na++ or K++ (i.e., low ionic strength), temperature higher than 20°C - 25° C. below the Tm of the oligomeric compound:target sequence complex, and the presence of denaturants such as formamide, dimethylformamide, dimethyl sulfoxide, or the detergent sodium dodecyl sulfate (SDS). For example, the hybridization rate decreases 1.1% for each 1% formamide. An example of a high stringency hybridization condition is 0.1X sodium chloride-sodium citrate buffer (SSC)/0.1% (w/v) SDS at 60° C. for 30 minutes.

"Complementary," as used herein, refers to the capacity for precise pairing between two nucleotides on one or two oligomeric strands. For example, if a nucleobase at a certain position of an antisense compound is capable of hydrogen bonding with a nucleobase at a certain position of a target nucleic acid, said target nucleic acid being a DNA, RNA, or oligonucleotide molecule, then the position of hydrogen bonding between the oligonucleotide and the target nucleic acid is considered to be a complementary position. The oligomeric compound and the further DNA, RNA, or oligonucleotide molecule are complementary to each other when a sufficient number of complementary positions in each molecule are occupied by nucleotides which can hydrogen bond with each other. Thus, "specifically hybridizable" and "complementary" are terms which are used to indicate a sufficient degree of precise pairing or complementarity over a sufficient number of nucleotides such that stable and specific binding occurs between the oligomeric compound and a target nucleic acid.

It is understood in the art that the sequence of an oligomeric compound need not be 100% complementary to that of its target nucleic acid to be specifically hybridizable. Moreover, an oligonucleotide may hybridize over one or more segments such that intervening or adjacent segments are not involved in the hybridization event (e.g., a loop structure, mismatch or hairpin structure). The oligomeric compounds of the present disclosure comprise at least about 70%, or at least about 75%, or at least about 80%, or at least about 85%, or at least about 90%, or at least about 95%, or at least about 99% sequence complementarity to a target region within the target nucleic acid sequence to which they are targeted. For example, an antisense compound in which 18 of 20 nucleotides of the antisense compound are complementary to a target region, and would therefore specifically hybridize, would represent 90 percent complementarity. In this example, the remaining noncomplementary nucleotides may be clustered or interspersed with complementary nucleotides and need not be contiguous to each other or to complementary nucleotides. As such, an antisense compound which is 18 nucleotides in length having 4 (four) noncomplementary nucleotides which are flanked by two regions of complete complementarity with the target nucleic acid would have 77.8% overall complementarity with the target nucleic acid and would thus fall within the scope of the present disclosure. Percent complementarity of an antisense compound with a region of a target nucleic acid can be determined routinely using BLAST programs (basic local alignment search tools) and PowerBLAST programs known in the art (Altschul et al., (1990) J. Mol. Biol., 215, 403-410; Zhang and Madden, (1997) Genome Res., 7, 649-656). Percent homology, sequence identity or complementarity, can be determined by, for example, the Gap program (Wisconsin Sequence Analysis Package, Version 8 for Unix, Genetics Computer Group, University Research Park, Madison Wis.), using default settings, which uses the algorithm of Smith and Waterman (Adv. Appl. Math., (1981) 2, 482-489).

As used herein, the term "Thermal Melting Point (Tm)" refers to the temperature, under defined ionic strength, pH, and nucleic acid concentration, at which 50% of the oligonucleotides complementary to the target sequence hybridize to the target sequence at equilibrium. Typically, stringent conditions will be those in which the salt concentration is at least about 0.01 to 1.0 M Na ion concentration (or other salts) at pH 7.0 to 8.3 and the temperature is at least about 30°C for short oligonucleotides (e.g., 10 to 50 nucleotide). Stringent conditions may also be achieved with the addition of destabilizing agents such as formamide.

As used herein, "modulation" means either an increase (stimulation) or a decrease (inhibition) in the expression of a gene.

The term "variant," when used in the context of a polynucleotide sequence, may encompass a polynucleotide sequence related to a wild type gene. This definition may also include, for example, "allelic," "splice," "species," or "polymorphic" variants. A splice variant may have significant identity to a reference molecule, but will generally have a greater or lesser number of polynucleotides due to alternate splicing of exons during mRNA processing. The corresponding polypeptide may possess additional functional domains or an absence of domains. Species variants are polynucleotide sequences that vary from one species to another. Of particular utility in the disclosure are variants of wild type gene products. Variants may result from at least one mutation in the nucleic acid sequence and may result in altered mRNAs or in polypeptides whose structure or function may or may not be altered. Any given natural or recombinant gene may have none, one, or many allelic forms. Common mutational changes that give rise to variants are generally ascribed to natural deletions, additions, or substitutions of nucleotides. Each of these types of changes may occur alone, or in combination with the others, one or more times in a given sequence.

The resulting polypeptides generally will have significant amino acid identity relative to each other. A polymorphic variant is a variation in the polynucleotide sequence of a particular gene between individuals of a given species. Polymorphic variants also may encompass "single nucleotide polymorphisms" (SNPs,) or single base mutations in which the polynucleotide sequence varies by one base. The presence of SNPs may be indicative of, for example, a certain population with a propensity for a disease state, that is susceptibility versus resistance.

Derivative polynucleotides include nucleic acids subjected to chemical modification, for example, replacement of hydrogen by an alkyl, acyl, or amino group. Derivatives, e.g., derivative oligonucleotides, may comprise non-naturally-occurring portions, such as altered sugar moieties or inter-sugar linkages. Exemplary among these are phosphorothioate and other sulfur containing species which are known in the art. Derivative nucleic acids may also contain labels, including radionucleotides, enzymes, fluorescent agents, chemiluminescent agents, chromogenic agents, substrates, cofactors, inhibitors, magnetic particles, and the like.

A "derivative" polypeptide or peptide is one that is modified, for example, by glycosylation, pegylation, phosphorylation, sulfation, reduction/alkylation, acylation, chemical coupling, or mild formalin treatment. A derivative may also be modified to contain a detectable label, either directly or indirectly, including, but not limited to, a radioisotope, fluorescent, and enzyme label.

As used herein, the term "animal" or "patient" is meant to include, for example, humans, sheep, elks, deer, mule deer, minks, mammals, monkeys, horses, cattle, pigs, goats, dogs, cats, rats, mice, birds, chicken, reptiles, fish, insects and arachnids.

"Mammal" covers warm blooded mammals that are typically under medical care (e.g., humans and domesticated animals). Examples include feline, canine, equine, bovine, and human, as well as just human.

"Treating" or "treatment" covers the treatment of a disease-state in a mammal, and includes: (a) preventing the disease-state from occurring in a mammal, in particular, when such mammal is predisposed to the disease-state but has not yet been diagnosed as having it; (b) inhibiting the disease-state, e.g., arresting it development; and/or (c) relieving the disease-state, e.g., causing regression of the disease state until a desired endpoint is reached. Treating also includes the amelioration of a symptom of a disease (e.g., lessen the pain or discomfort), wherein such amelioration may or may not be directly affecting the disease (e.g., cause, transmission, expression, etc.).

"Neurodegenerative disease or disorder" refers to a wide range of diseases and disorders of the central and peripheral nervous system including, for example, Parkinson's Disease, Huntington's Disease, Alzheimer's Disease, amyotrophic lateral sclerosis (ALS), dementia, multiple sclerosis and other diseases and disorders associated with neuronal cell death.

"Metabolic disease" refers to a wide range of diseases and disorders of the endocrine system including, for example, insulin resistance, diabetes, obesity, impaired glucose tolerance, high blood cholesterol, hyperglycemia, dyslipidemia and hyperlipidemia.

As used herein, the term "cancer" refers to any malignant tumor, particularly arising in the lung, kidney, or thyroid. The cancer manifests itself as a "tumor" or tissue comprising malignant cells of the cancer. Examples of tumors include sarcomas and carcinomas such as, but not limited to: fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilms' tumor, cervical cancer, testicular tumor, lung carcinoma, small cell lung carcinoma, bladder carcinoma, epithelial carcinoma, glioma, astrocytoma, medulloblastoma, craniopharyngioma, ependymoma, pinealoma, hemangioblastoma, acoustic neuroma, oligodendroglioma, meningioma, melanoma, neuroblastoma, and retinoblastoma. As noted above, the disclosure specifically permits differential diagnosis of lung, kidney, and thyroid tumors.

### Polynucleotide and Oligonucleotide Compositions and Molecules

"SIRT1 protein" refers to a member of the sir2 family of sirtuin deacetylases. In one instance, a SIRT1 protein includes yeast Sir2 (GenBank Accession No. P53685), C. elegans Sir-2.1 (GenBank Accession No. NP.sub.--501912), human SIRT1 (GenBank Accession No. NM.sub.--012238 and NP.sub.--036370 (or AF083106))

SIRT1 "Sirtuins" are proteins that include a SIR2 domain, a domain defined as amino acids sequences that are scored as hits in the Pfam family "SIR2"- PF02146 (attached to the Appendix). This family is referenced in the INTERPRO database as INTERPRO description (entry IPR003000). To identify the presence of a "SIR2" domain in a protein sequence, and make the determination that a polypeptide or protein of interest has a particular profile, the amino acid sequence of the protein can be searched against the Pfam database of HMMs (e.g., the Pfam database, release 9) using the default parameters (http://www.sanger.ac.uk/Software/Pfam/HMM_search). The SIR2 domain is indexed in Pfam as PF02146 and in INTERPRO as INTERPRO description (entry IPR003000). A description of the Pfam database can be found in "The Pfam Protein Families Database" Bateman A et al. (2002) Nucleic Acids Research 30(1):276-280 and Sonhammer et al. (1997) Proteins 28(3):405-420 and a detailed description of HMMs can be found, for example, in Gribskov et al. (1990) Meth. Enzymol. 183:146-159; Gribskov et al. (1987) Proc. Natl. Acad. Sci. USA 84:4355-4358; Krogh et al. (1994) J. Mol. Biol. 235:1501-1531; and Stultz et al. (1993) Protein Sci. 2:305-314.

*Targets:* In one instance, the targets comprise nucleic acid sequences of Sirtuin 1 (SIRT1), including without limitation sense and/or antisense noncoding and/or coding sequences associated with SIRT1.

In preferred instances, antisense oligonucleotides are used to prevent or treat diseases or disorders associated with Sirtuin 1 (SIRT1). The sirtuins (SIRTs) are protein-modifying enzymes that are distributed ubiquitously in all organisms. SIRT1 is a mammalian homologue of yeast nicotinamide-adenine-dinucleotide-dependent deacetylase silent information regulator 2 (known as Sir2), which is the best-characterized SIRT family member. SIRT1 regulates the physiology of cells of the adipocyte lineage. Modulators of SIRT1 activity can be used to ameliorate, treat, or prevent diseases and disorders associated with adipose physiology, e.g., obesity, an obesity-related disease, or a fat-related metabolic disorder.

SIRT1 regulates longevity in several model organisms and is involved in several processes in mammalian cells including cell survival, differentiation, and metabolism. SIRT1 induction, either by SIRT-activating compounds such as resveratrol, or metabolic conditioning associated with caloric restriction, could have neuroprotective qualities and thus delay the neurodegenerative process, thereby promoting longevity (Han SH, (2009) J Clin Neurol. Sep;5(3):120-5.; Michan S, et al. (2007) Biochem J. 404(1): 1-13.).

There are several reports that support an axonal protective role for SIRT1 in the neuronal system. Axonal degeneration is a major morphological characteristic observed in both peripheral neuropathies and neurodegenerative diseases, such as Alzheimer's disease (AD) and amyotrophic lateral sclerosis (Fischer LR, et al. (2004) Exp Neurol 185:232-240; Stokin GB, et al. (2005) Science 307:1282-1288). Axonal degeneration usually occurs in the early stage in degenerative processes and often precedes or correlates closely with clinical symptoms such as cognitive decline (Yamamoto H, et al. (2007) Mol Endocrinol. 21 (8): 1745-1755).

In a preferred instance, the oligonucleotides are specific for polynucleotides of SIRT1, which includes, without limitation noncoding regions. The SIRT1 targets comprise variants of SIRT1; mutants of SIRT1, including SNPs; noncoding sequences of SIRT1; alleles, fragments and the like. Preferably the oligonucleotide is an antisense RNA molecule.

In accordance with instances of the disclosure the target nucleic acid molecule is not limited to SIRT1 polynucleotides alone but extends to any of the isoforms, receptors, homologs, non-coding regions and the like of SIRT1.

In another preferred instance, an oligonucleotide targets a natural antisense sequence (natural antisense to the coding and non-coding regions) of SIRT1 targets, including, without limitation, variants, alleles, homologs, mutants, derivatives, fragments and complementary sequences thereto. Preferably the oligonucleotide is an antisense RNA or DNA molecule.

In another preferred instance, the oligomeric compounds of the present disclosure also include variants in which a different base is present at one or more of the nucleotide positions in the compound. For example, if the first nucleotide is an adenine, variants may be produced which contain thymidine, guanosine, cytidine or other natural or unnatural nucleotides at this position. This may be done at any of the positions of the antisense compound. These compounds are then tested using the methods described herein to determine their ability to inhibit expression of a target nucleic acid.

In some instances, homology, sequence identity or complementarity, between the antisense compound and target is from about 50% to about 60%. In some instances, homology, sequence identity or complementarity, is from about 60% to about 70%. In some instances, homology, sequence identity or complementarity, is from about 70% to about 80%. In some instances, homology, sequence identity or complementarity, is from about 80% to about 90%. In some instances, homology, sequence identity or complementarity, is about 90%, about 92%, about 94%, about 95%, about 96%, about 97%, about 98%, about 99% or about 100%.

An antisense compound is specifically hybridizable when binding of the compound to the target nucleic acid interferes with the normal function of the target nucleic acid to cause a loss of activity, and there is a sufficient degree of complementarity to avoid non-specific binding of the antisense compound to non-target nucleic acid sequences under conditions in which specific binding is desired. Such conditions include, i.e., physiological conditions in the case of in vivo assays or therapeutic treatment, and conditions in which assays are performed in the case of in vitro assays.

An antisense compound, whether DNA, RNA, chimeric, substituted etc, is specifically hybridizable when binding of the compound to the target DNA or RNA molecule interferes with the normal function of the target DNA or RNA to cause a loss of utility, and there is a sufficient degree of complementarily to avoid non-specific binding of the antisense compound to non-target sequences under conditions in which specific binding is desired, i.e., under physiological conditions in the case of in vivo assays or therapeutic treatment, and in the case of in vitro assays, under conditions in which the assays are performed.

In another preferred instance, targeting of SIRT1 including without limitation, antisense sequences which are identified and expanded, using for example, PCR, hybridization etc., one or more of the sequences set forth as SEQ ID NO: 3 to 8, and the like, modulate the expression or function of SIRT1. In one instance, expression or function is up-regulated as compared to a control. In another preferred instance, expression or function is down-regulated as compared to a control.

In another preferred instance, oligonucleotides comprise nucleic acid sequences set forth as SEQ ID NOS: 9 to 66 including antisense sequences which are identified and expanded, using for example, PCR, hybridization etc. These oligonucleotides can comprise one or more modified nucleotides, shorter or longer fragments, modified bonds and the like. Examples of modified bonds or internucleotide linkages comprise phosphorothioate, phosphorodithioate or the like. In another preferred instance, the nucleotides comprise a phosphorus derivative. The phosphorus derivative (or modified phosphate group) which may be attached to the sugar or sugar analog moiety in the modified oligonucleotides of the present disclosure may be a monophosphate, diphosphate, triphosphate, alkylphosphate, alkanephosphate, phosphorothioate and the like. The preparation of the above-noted phosphate analogs, and their incorporation into nucleotides, modified nucleotides and oligonucleotides, *per se,* is also known and need not be described here.

The specificity and sensitivity of antisense is also harnessed by those of skill in the art for therapeutic uses. Antisense oligonucleotides have been employed as therapeutic moieties in the treatment of disease states in animals and man. Antisense oligonucleotides have been safely and effectively administered to humans and numerous clinical trials are presently underway. It is thus established that oligonucleotides can be useful therapeutic modalities that can be configured to be useful in treatment regimes for treatment of cells, tissues and animals, especially humans.

In instances of the present disclosure oligomeric antisense compounds, particularly oligonucleotides, bind to target nucleic acid molecules and modulate the expression and/or function of molecules encoded by a target gene. The functions of DNA to be interfered comprise, for example, replication and transcription. The functions of RNA to be interfered comprise all vital functions such as, for example, translocation of the RNA to the site of protein translation, translation of protein from the RNA, splicing of the RNA to yield one or more mRNA species, and catalytic activity which may be engaged in or facilitated by the RNA. The functions may be up-regulated or inhibited depending on the functions desired.

The antisense compounds, include, antisense oligomeric compounds, antisense oligonucleotides, external guide sequence (EGS) oligonucleotides, alternate splicers, primers, probes, and other oligomeric compounds that hybridize to at least a portion of the target nucleic acid. As such, these compounds may be introduced in the form of single-stranded, double-stranded, partially single-stranded, or circular oligomeric compounds.

Targeting an antisense compound to a particular nucleic acid molecule, in the context of this disclosure, can be a multistep process. The process usually begins with the identification of a target nucleic acid whose function is to be modulated. This target nucleic acid may be, for example, a cellular gene (or mRNA transcribed from the gene) whose expression is associated with a particular disorder or disease state, or a nucleic acid molecule from an infectious agent. In the present disclosure, the target nucleic acid encodes Sirtuin 1 (SIRT1).

The targeting process usually also includes determination of at least one target region, segment, or site within the target nucleic acid for the antisense interaction to occur such that the desired effect, e.g., modulation of expression, will result. Within the context of the present disclosure, the term "region" is defined as a portion of the target nucleic acid having at least one identifiable structure, function, or characteristic. Within regions of target nucleic acids are segments. "Segments" are defined as smaller or sub-portions of regions within a target nucleic acid. "Sites," as used in the present disclosure, are defined as positions within a target nucleic acid.

In a preferred instance, the antisense oligonucleotides bind to the natural antisense sequences of Sirtuin 1 (SIRT1) and modulate the expression and/or function of Sirtuin 1 (SIRT1) (SEQ ID NO: 1). Examples of antisense sequences include SEQ ID NOS: 3 to 66.

In another preferred instance, the antisense oligonucleotides bind to one or more segments of Sirtuin 1 (SIRT1) polynucleotides and modulate the expression and/or function of Sirtuin 1 (SIRT1). The segments comprise at least five consecutive nucleotides of the Sirtuin 1 (SIRT1) sense or antisense polynucleotides.

In another preferred instance, the antisense oligonucleotides are specific for natural antisense sequences of Sirtuin 1 (SIRT1) wherein binding of the oligonucleotides to the natural antisense sequences of Sirtuin 1 (SIRT1) modulate expression and/or function of Sirtuin 1 (SIRT1).

In another preferred instance, oligonucleotide compounds comprise sequences set forth as SEQ ID NOS: 9 to 66, antisense sequences which are identified and expanded, using for example, PCR, hybridization etc These oligonucleotides can comprise one or more modified nucleotides, shorter or longer fragments, modified bonds and the like. Examples of modified bonds or internucleotide linkages comprise phosphorothioate, phosphorodithioate or the like. In another preferred instance, the nucleotides comprise a phosphorus derivative. The phosphorus derivative (or modified phosphate group) which may be attached to the sugar or sugar analog moiety in the modified oligonucleotides of the present disclosure may be a monophosphate, diphosphate, triphosphate, alkylphosphate, alkanephosphate, phosphorothioate and the like. The preparation of the above-noted phosphate analogs, and their incorporation into nucleotides, modified nucleotides and oligonucleotides, per se, is also known and need not be described here.

Since, as is known in the art, the translation initiation codon is typically 5'-AUG (in transcribed mRNA molecules; 5'-ATG in the corresponding DNA molecule), the translation initiation codon is also referred to as the "AUG codon," the "start codon" or the "AUG start codon". A minority of genes has a translation initiation codon having the RNA sequence 5'-GUG, 5'-UUG or 5'-CUG; and 5'-AUA, 5'-ACG and 5'-CUG have been shown to function in vivo. Thus, the terms "translation initiation codon" and "start codon" can encompass many codon sequences, even though the initiator amino acid in each instance is typically methionine (in eukaryotes) or formylmethionine (in prokaryotes). Eukaryotic and prokaryotic genes may have two or more alternative start codons, any one of which may be preferentially utilized for translation initiation in a particular cell type or tissue, or under a particular set of conditions. In the context of the disclosure, "start codon" and "translation initiation codon" refer to the codon or codons that are used in vivo to initiate translation of an mRNA transcribed from a gene encoding Sirtuin 1 (SIRT1), regardless of the sequence(s) of such codons. A translation termination codon (or "stop codon") of a gene may have one of three sequences, i.e., 5'-UAA, 5'-UAG and 5'-UGA (the corresponding DNA sequences are 5'-TAA, 5'- TAG and 5'-TGA, respectively).

The terms "start codon region" and "translation initiation codon region" refer to a portion of such an mRNA or gene that encompasses from about 25 to about 50 contiguous nucleotides in either direction (i.e., 5' or 3') from a translation initiation codon. Similarly, the terms "stop codon region" and "translation termination codon region" refer to a portion of such an mRNA or gene that encompasses from about 25 to about 50 contiguous nucleotides in either direction (i.e., 5' or 3') from a translation termination codon. Consequently, the "start codon region" (or "translation initiation codon region") and the "stop codon region" (or "translation termination codon region") are all regions that may be targeted effectively with the antisense compounds of the present disclosure.

The open reading frame (ORF) or "coding region," which is known in the art to refer to the region between the translation initiation codon and the translation termination codon, is also a region which may be targeted effectively. Within the context of the present disclosure, a targeted region is the intragenic region encompassing the translation initiation or termination codon of the open reading frame (ORF) of a gene.

Another target region includes the 5' untranslated region (5'UTR), known in the art to refer to the portion of an mRNA in the 5' direction from the translation initiation codon, and thus including nucleotides between the 5' cap site and the translation initiation codon of an mRNA (or corresponding nucleotides on the gene). Still another target region includes the 3' untranslated region (3'UTR), known in the art to refer to the portion of an mRNA in the 3' direction from the translation termination codon, and thus including nucleotides between the translation termination codon and 3' end of an mRNA (or corresponding nucleotides on the gene). The 5' cap site of an mRNA comprises an N7-methylated guanosine residue joined to the 5'-most residue of the mRNA via a 5'-5' triphosphate linkage. The 5' cap region of an mRNA is considered to include the 5' cap structure itself as well as the first 50 nucleotides adjacent to the cap site. Another target region for this disclosure is the 5' cap region.

Although some eukaryotic mRNA transcripts are directly translated, many contain one or more regions, known as "introns," which are excised from a transcript before it is translated. The remaining (and therefore translated) regions are known as "exons" and are spliced together to form a continuous mRNA sequence. In one instance, targeting splice sites, i.e., intron-exon junctions or exon-intron junctions, is particularly useful in situations where aberrant splicing is implicated in disease, or where an overproduction of a particular splice product is implicated in disease. An aberrant fusion junction due to rearrangement or deletion is another instance of a target site. mRNA transcripts produced via the process of splicing of two (or more) mRNAs from different gene sources are known as "fusion transcripts". Introns can be effectively targeted using antisense compounds targeted to, for example, DNA or pre-mRNA.

In another preferred instance, the antisense oligonucleotides bind to coding and/or non-coding regions of a target polynucleotide and modulate the expression and/or function of the target molecule.

In another preferred instance, the antisense oligonucleotides bind to natural antisense polynucleotides and modulate the expression and/or function of the target molecule.

In another preferred instance, the antisense oligonucleotides bind to sense polynucleotides and modulate the expression and/or function of the target molecule.

Alternative RNA transcripts can be produced from the same genomic region of DNA. These alternative transcripts are generally known as "variants". More specifically, "pre-mRNA variants" are transcripts produced from the same genomic DNA that differ from other transcripts produced from the same genomic DNA in either their start or stop position and contain both intronic and exonic sequence.

Upon excision of one or more exon or intron regions, or portions thereof during splicing, pre-mRNA variants produce smaller "mRNA variants". Consequently, mRNA variants are processed pre-mRNA variants and each unique pre-mRNA variant must always produce a unique mRNA variant as a result of splicing. These mRNA variants are also known as "alternative splice variants". If no splicing of the pre-mRNA variant occurs then the pre-mRNA variant is identical to the mRNA variant.

Variants can be produced through the use of alternative signals to start or stop transcription. Pre-mRNAs and mRNAs can possess more than one start codon or stop codon. Variants that originate from a pre-mRNA or mRNA that use alternative start codons are known as "alternative start variants" of that pre-mRNA or mRNA. Those transcripts that use an alternative stop codon are known as "alternative stop variants" of that pre-mRNA or mRNA. One specific type of alternative stop variant is the "polyA variant" in which the multiple transcripts produced result from the alternative selection of one of the "polyA stop signals" by the transcription machinery, thereby producing transcripts that terminate at unique polyA sites. Within the context of the disclosure, the types of variants described herein are also instances of target nucleic acids.

The locations on the target nucleic acid to which the antisense compounds hybridize are defined as at least a 5-nucleotide long portion of a target region to which an active antisense compound is targeted.

While the specific sequences of certain exemplary target segments are set forth herein, one of skill in the art will recognize that these serve to illustrate and describe particular instances within the scope of the present disclosure. Additional target segments are readily identifiable by one having ordinary skill in the art in view of this disclosure.

Target segments 5-100 nucleotides in length comprising a stretch of at least five (5) consecutive nucleotides selected from within the illustrative preferred target segments are considered to be suitable for targeting as well.

Target segments can include DNA or RNA sequences that comprise at least the 5 consecutive nucleotides from the 5'-terminus of one of the illustrative preferred target segments (the remaining nucleotides being a consecutive stretch of the same DNA or RNA beginning immediately upstream of the 5'-terminus of the target segment and continuing until the DNA or RNA contains about 5 to about 100 nucleotides). Similarly preferred target segments are represented by DNA or RNA sequences that comprise at least the 5 consecutive nucleotides from the 3'-terminus of one of the illustrative preferred target segments (the remaining nucleotides being a consecutive stretch of the same DNA or RNA beginning immediately downstream of the 3'-terminus of the target segment and continuing until the DNA or RNA contains about 5 to about 100 nucleotides). One having skill in the art armed with the target segments illustrated herein will be able, without undue experimentation, to identify further preferred target segments.

Once one or more target regions, segments or sites have been identified, antisense compounds are chosen which are sufficiently complementary to the target, i.e., hybridize sufficiently well and with sufficient specificity, to give the desired effect.

In instances of the disclosure the oligonucleotides bind to an antisense strand of a particular target. The oligonucleotides are at least 5 nucleotides in length and can be synthesized so each oligonucleotide targets overlapping sequences such that oligonucleotides are synthesized to cover the entire length of the target polynucleotide. The targets also include coding as well as non coding regions.

In one instance, it is preferred to target specific nucleic acids by antisense oligonucleotides. Targeting an antisense compound to a particular nucleic acid, is a multistep process. The process usually begins with the identification of a nucleic acid sequence whose function is to be modulated. This may be, for example, a cellular gene (or mRNA transcribed from the gene) whose expression is associated with a particular disorder or disease state, or a non coding polynucleotide such as for example, non coding RNA (ncRNA).

RNAs can be classified into (1) messenger RNAs (mRNAs), which are translated into proteins, and (2) non-protein-coding RNAs (ncRNAs). ncRNAs comprise microRNAs, antisense transcripts and other Transcriptional Units (TU) containing a high density of stop codons and lacking any extensive "Open Reading Frame". Many ncRNAs appear to start from initiation sites in 3' untranslated regions (3'UTRs) of protein-coding loci. ncRNAs are often rare and at least half of the ncRNAs that have been sequenced by the FANTOM consortium seem not to be polyadenylated. Most researchers have for obvious reasons focused on polyadenylated mRNAs that are processed and exported to the cytoplasm. Recently, it was shown that the set of non-polyadenylated nuclear RNAs may be very large, and that many such transcripts arise from so-called intergenic regions (Cheng, J. et al. (2005) Science 308 (5725), 1149-1154; Kapranov, P. et al. (2005). Genome Res 15 (7), 987-997). The mechanism by which ncRNAs may regulate gene expression is by base pairing with target transcripts. The RNAs that function by base pairing can be grouped into (1) cis encoded RNAs that are encoded at the same genetic location, but on the opposite strand to the RNAs they act upon and therefore display perfect complementarity to their target, and (2) trans-encoded RNAs that are encoded at a chromosomal location distinct from the RNAs they act upon and generally do not exhibit perfect base-pairing potential with their targets.

Without wishing to be bound by theory, perturbation of an antisense polynucleotide by the antisense oligonucleotides described herein can alter the expression of the corresponding sense messenger RNAs. However, this regulation can either be discordant (antisense knockdown results in messenger RNA elevation) or concordant (antisense knockdown results in concomitant messenger RNA reduction). In these cases, antisense oligonucleotides can be targeted to overlapping or non-overlapping parts of the antisense transcript resulting in its knockdown or sequestration. Coding as well as non-coding antisense can be targeted in an identical manner and that either category is capable of regulating the corresponding sense transcripts - either in a concordant or disconcordant manner. The strategies that are employed in identifying new oligonucleotides for use against a target can be based on the knockdown of antisense RNA transcripts by antisense oligonucleotides or any other means of modulating the desired target.
*Strategy 1:* In the case of discordant regulation, knocking down the antisense transcript elevates the expression of the conventional (sense) gene. Should that latter gene encode for a known or putative drug target, then knockdown of its antisense counterpart could conceivably mimic the action of a receptor agonist or an enzyme stimulant.
*Strategy 2:* In the case of concordant regulation, one could concomitantly knock down both antisense and sense transcripts and thereby achieve synergistic reduction of the conventional (sense) gene expression. If, for example, an antisense oligonucleotide is used to achieve knockdown, then this strategy can be used to apply one antisense oligonucleotide targeted to the sense transcript and another antisense oligonucleotide to the corresponding antisense transcript, or a single energetically symmetric antisense oligonucleotide that simultaneously targets overlapping sense and antisense transcripts.

According to the present disclosure, antisense compounds include antisense oligonucleotides, ribozymes, external guide sequence (EGS) oligonucleotides, siRNA compounds, single- or double-stranded RNA interference (RNAi) compounds such as siRNA compounds, and other oligomeric compounds which hybridize to at least a portion of the target nucleic acid and modulate its function. As such, they may be DNA, RNA, DNA-like, RNA-like, or mixtures thereof, or may be mimetics of one or more of these. These compounds may be single-stranded, doublestranded, circular or hairpin oligomeric compounds and may contain structural elements such as internal or terminal bulges, mismatches or loops. Antisense compounds are routinely prepared linearly but can be joined or otherwise prepared to be circular and/or branched. Antisense compounds can include constructs such as, for example, two strands hybridized to form a wholly or partially double-stranded compound or a single strand with sufficient self-complementarity to allow for hybridization and formation of a fully or partially double-stranded compound. The two strands can be linked internally leaving free 3' or 5' termini or can be linked to form a continuous hairpin structure or loop. The hairpin structure may contain an overhang on either the 5' or 3' terminus producing an extension of single stranded character. The double stranded compounds optionally can include overhangs on the ends. Further modifications can include conjugate groups attached to one of the termini, selected nucleotide positions, sugar positions or to one of the internucleoside linkages. Alternatively, the two strands can be linked via a non-nucleic acid moiety or linker group. When formed from only one strand, dsRNA can take the form of a self-complementary hairpin-type molecule that doubles back on itself to form a duplex. Thus, the dsRNAs can be fully or partially double stranded. Specific modulation of gene expression can be achieved by stable expression of dsRNA hairpins in transgenic cell lines, however, in some instances, the gene expression or function is up regulated. When formed from two strands, or a single strand that takes the form of a self-complementary hairpin-type molecule doubled back on itself to form a duplex, the two strands (or duplex-forming regions of a single strand) are complementary RNA strands that base pair in Watson-Crick fashion.

Once introduced to a system, the compounds of the disclosure may elicit the action of one or more enzymes or structural proteins to effect cleavage or other modification of the target nucleic acid or may work via occupancy-based mechanisms. In general, nucleic acids (including oligonucleotides) may be described as "DNA-like" (i.e., generally having one or more 2'-deoxy sugars and, generally, T rather than U bases) or "RNA-like" (i.e., generally having one or more 2'- hydroxyl or 2'-modified sugars and, generally U rather than T bases). Nucleic acid helices can adopt more than one type of structure, most commonly the A- and B-forms. It is believed that, in general, oligonucleotides which have B-form-like structure are "DNA-like" and those which have A-formlike structure are "RNA-like." In some (chimeric) instances, an antisense compound may contain both A- and B-form regions.

In another preferred instance, the desired oligonucleotides or antisense compounds, comprise at least one of: antisense RNA, antisense DNA, chimeric antisense oligonucleotides, antisense oligonucleotides comprising modified linkages, interference RNA (RNAi), short interfering RNA (siRNA); a micro, interfering RNA (miRNA); a small, temporal RNA (stRNA); or a short, hairpin RNA (shRNA); small RNA-induced gene activation (RNAa); small activating RNAs (saRNAs), or combinations thereof.

dsRNA can also activate gene expression, a mechanism that has been termed "small RNA-induced gene activation" or RNAa. dsRNAs targeting gene promoters induce potent transcriptional activation of associated genes. RNAa was demonstrated in human cells using synthetic dsRNAs, termed "small activating RNAs" (saRNAs). It is currently not known whether RNAa is conserved in other organisms.

Small double-stranded RNA (dsRNA), such as small interfering RNA (siRNA) and microRNA (miRNA), have been found to be the trigger of an evolutionary conserved mechanism known as RNA interference (RNAi). RNAi invariably leads to gene silencing via remodeling chromatin to thereby suppress transcription, degrading complementary mRNA, or blocking protein translation. However, in instances described in detail in the examples section which follows, oligonucleotides are shown to increase the expression and/or function of the Sirtuin 1 (SIRT1) polynucleotides and encoded products thereof. dsRNAs may also act as small activating RNAs (saRNA). Without wishing to be bound by theory, by targeting sequences in gene promoters, saRNAs would induce target gene expression in a phenomenon referred to as dsRNA-induced transcriptional activation (RNAa).

In a further instance, the "preferred target segments" identified herein may be employed in a screen for additional compounds that modulate the expression of Sirtuin 1 (SIRT1) polynucleotides. "Modulators" are those compounds that decrease or increase the expression of a nucleic acid molecule encoding Sirtuin 1 (SIRT1) and which comprise at least a 5-nucleotide portion that is complementary to a preferred target segment. The screening method comprises the steps of contacting a preferred target segment of a nucleic acid molecule encoding sense or natural antisense polynucleotides of Sirtuin 1 (SIRT1) with one or more candidate modulators, and selecting for one or more candidate modulators which decrease or increase the expression of a nucleic acid molecule encoding Sirtuin 1 (SIRT1) polynucleotides, e.g. SEQ ID NOS: 9 to 66. Once it is shown that the candidate modulator or modulators are capable of modulating (e.g. either decreasing or increasing) the expression of a nucleic acid molecule encoding Sirtuin 1 (SIRT1) polynucleotides, the modulator may then be employed in further investigative studies of the function of Sirtuin 1 (SIRT1) polynucleotides, or for use as a research, diagnostic, or therapeutic agent in accordance with the present disclosure.

Targeting the natural antisense sequence preferably modulates the function of the target gene. For example, the SIRT1 gene (e.g. accession number NM_012238.3, Fig. 15). In a preferred instance, the target is an antisense polynucleotide of the Sirtuin 1 gene. In a preferred instance, an antisense oligonucleotide targets sense and/or natural antisense sequences of Sirtuin 1 (SIRT1) polynucleotides (e.g. accession number NM_012238.3, Fig. 15), variants, alleles, isoforms, homologs, mutants, derivatives, fragments and complementary sequences thereto. Preferably the oligonucleotide is an antisense molecule and the targets include coding and noncoding regions of antisense and/or sense SIRT1 polynucleotides.

The preferred target segments of the present disclosure may be also be combined with their respective complementary antisense compounds of the present disclosure to form stabilized double-stranded (duplexed) oligonucleotides.

Such double stranded oligonucleotide moieties have been shown in the art to modulate target expression and regulate translation as well as RNA processing via an antisense mechanism. Moreover, the double-stranded moieties may be subject to chemical modifications (Fire et al., (1998) Nature, 391, 806-811; Timmons and Fire, (1998) Nature, 395, 854; Timmons et al., (2001) Gene, 263, 103-112; Tabara et al., (1998) Science, 282, 430-431; Montgomery et al., (1998) Proc. Natl. Acad. Sci. USA, 95, 15502-15507; Tuschl et al., (1999) Genes Dev., 13, 3191-3197; Elbashir et al., (2001) Nature, 411, 494-498; Elbashir et al., (2001) Genes Dev. 15, 188-200). For example, such double-stranded moieties have been shown to inhibit the target by the classical hybridization of antisense strand of the duplex to the target, thereby triggering enzymatic degradation of the target (Tijsterman et al., (2002) Science, 295, 694-697).

In a preferred instance, an antisense oligonucleotide targets Sirtuin 1 (SIRT1) polynucleotides (e.g. accession number NM_012238.3), variants, alleles, isoforms, homologs, mutants, derivatives, fragments and complementary sequences thereto. Preferably the oligonucleotide is an antisense molecule.

In accordance with instances of the disclosure, the target nucleic acid molecule is not limited to Sirtuin 1 (SIRT1) alone but extends to any of the isoforms, receptors, homologs and the like of Sirtuin 1 (SIRT1) molecules.

In another preferred instance, an oligonucleotide targets a natural antisense sequence of SIRT1 polynucleotides, for example, polynucleotides set forth as SEQ ID NO: 3 to 8, and any variants, alleles, homologs, mutants, derivatives, fragments and complementary sequences thereto. Examples of antisense oligonucleotides are set forth as SEQ ID NOS: 9 to 66.

In one instance, the oligonucleotides are complementary to or bind to nucleic acid sequences of Sirtuin 1 (SIRT1) antisense, including without limitation noncoding sense and/or antisense sequences associated with Sirtuin 1 (SIRT1) polynucleotides and modulate expression and/or function of Sirtuin 1 (SIRT1) molecules.

In another preferred instance, the oligonucleotides are complementary to or bind to nucleic acid sequences of SIRT1 natural antisense, set forth as SEQ ID NO: 3 to 8 and modulate expression and/or function of SIRT1 molecules.

In a preferred instance, oligonucleotides comprise sequences of at least 5 consecutive nucleotides of SEQ ID NOS: 9 to 66 and modulate expression and/or function of Sirtuin 1 (SIRT1) molecules.

The polynucleotide targets comprise SIRT1, including family members thereof, variants of SIRT1; mutants of SIRT1, including SNPs; noncoding sequences of SIRT1; alleles of SIRT1; species variants, fragments and the like. Preferably the oligonucleotide is an antisense molecule.

In another preferred instance, the oligonucleotide targeting Sirtuin 1 (SIRT1) polynucleotides, comprise: antisense RNA, interference RNA (RNAi), short interfering RNA (siRNA); micro interfering RNA (miRNA); a small, temporal RNA (stRNA); or a short, hairpin RNA (shRNA); small RNA-induced gene activation (RNAa); or, small activating RNA (saRNA).

In another preferred instance, targeting of Sirtuin 1 (SIRT1) polynucleotides, e.g. SEQ ID NO: 3 to 8, modulates the expression or function of these targets. In one instance, expression or function is up-regulated as compared to a control. In another preferred instance, expression or function is down-regulated as compared to a control.

In another preferred instance, antisense compounds comprise sequences set forth as SEQ ID NOS: 9 to 66. These oligonucleotides can comprise one or more modified nucleotides, shorter or longer fragments, modified bonds and the like.

In another preferred instance, SEQ ID NOS: 9 to 66 comprise one or more LNA nucleotides.

**Table 1 shows exemplary antisense oligonucleotides useful in the methods of the disclosure.**

| **Sequence ID** | **SEQ Name** | **Sequence** |
|---|---|---|
| SEQ ID NO:9 | CUR-0292 | T*T*G*G*T*A*T*T*C*A*C*A*A*G |
| SEQ ID NO:10 | CUR-0293 | A*A*A*C*T*G*G*A*A*A*C*C*T*A |
| SEQ ID NO:11 | CUR-0294 | G*A*T*C*T*T*T*A*T*G*A*G*A*A |
| SEQ ID NO:12 | CUR-0295 | G*A*T*G*G*A*G*A*A*A*T*T*G*G |
| SEQ ID NO:13 | CUR-0296 | A*G*T*C*T*G*A*T*G*G*A*G*A*A |
| SEQ ID NO:14 | CUR-0297 | T*G*T*T*A*A*G*G*G*A*T*G*T*C |
| SEQ ID NO:15 | CUR-0298 | A*A*T*C*T*G*C*T*T*T*T*G*T*T |
| SEQ ID NO:16 | CUR-0299 | A*G*G*G*A*A*T*T*G*A*A*A*T*C |
| SEQ ID NO:17 | CUR-0300 | T*A*A*G*G*C*A*A*G*A*T*T*T*C |
| SEQ ID NO:18 | CUR-0301 | T*A*A*A*T*G*G*A*G*T*T*A*A*G |
| SEQ ID NO:19 | CUR-0302 | T*T*A*T*T*T*A*T*A*G*C*A*C*A |
| SEQ ID NO:20 | CUR-0303 | T*T*G*C*T*T*C*T*G*C*T*T*A*T |
| SEQ ID NO:21 | CUR-0304 | A*A*A*A*A*A*A*T*A*T*T*T*G*C |
| SEQ ID NO:22 | CUR-0305 | C*A*G*C*C*T*T*A*A*A*A*A*A*A |
| SEQ ID NO:23 | CUR-0306 | T*T*T*T*A*A*A*A*C*C*T*C*T*C |
| SEQ ID NO:24 | CUR-0307 | T*A*G*T*T*C*A*G*A*T*T*T*T*T |
| SEQ ID NO:25 | CUR-0308 | A*G*C*A*G*T*T*G*C*T*A*A*A*T |
| SEQ ID NO:26 | CUR-0309 | C*T*G*A*G*T*G*C*A*G*C*A*G*C |
| SEQ ID NO:27 | CUR-0245 | G*T*C*T*G*A*T*G*G*A*G*A |
| SEQ ID NO:28 | CUR-0736/ CUR-0963 | +G*+T*C*T*G*A*T*G*G*+A*+G*+A |
| SEQ ID NO:29 | CUR-0688 | +G*+T*C*T*G*A*T*G*G*A*+G*+A |
| SEQ ID NO:30 | CUR-0740 | +G*mU*mC*+T*mG*mA*+T*mG*mG*+A*mG*mA |
| SEQ ID NO:31 | CUR-0664 | mG*mU*mC*mU*mG*mA*mU*mG*mG*mA*mG*mA |
| SEQ ID NO:32 | sirtas_5 | ACTGACACCTAATTGTATTCACATGAA |
| SEQ ID NO:33 | sirtas_6 | TGAGCAGCAGTTGCTAAATTAGTTCA |
| SEQ ID NO:34 | sirtas_7 | TCTACCTACATTATATCATAGCTCCTA |
| SEQ ID NO:35 | CUR-1230 | T*T*G*G*T*A*T*T*C*A*C*A*A*G*T*G*A*A*A |
| SEQ ID NO:36 | CUR-1231 | T*T*G*C*T*A*A*A*T*T*A*G*T*T*C*A*G*A*T |
| SEQ ID NO:37 | CUR-1232 | G*C*A*G*C*A*G*C*A*G*T*T*G*C*T*A*A*A*T |
| SEQ ID NO:38 | CUR-1233 | G*C*A*G*T*T*G*C*T*A*A*A*T*T*A*G*T*T*C |
| SEQ ID NO:39 | CUR-1302 | G*C*C*A*T*G*T*T*G*C*C*C*A*G*T*C*C*A*G*T |
| SEQ ID NO:40 | CUR-1304 | G*G*G*C*T*C*T*G*C*T*A*C*T*T*A*C*T*T*G*C |
| SEQ ID NO:41 | CUR-1303 | C*C*C*A*G*T*C*T*T*C*A*G*C*C*T*T*G*T*C*T |
| SEQ ID NO:42 | CUR-1305 | G*G*G*T*C*T*C*T*G*T*C*A*T*A*T*G*T*T*C*T*T |
| SEQ ID NO:43 | CUR-1264 | T*T*C*C*T*A*C*C*T*T*C*C*C*T*C*C*A*T*A |
| SEQ ID NO:44 | CUR-1265 | A*T*T*C*C*T*A*C*C*T*T*C*C*C*T*C*C*A*T |
| SEQ ID NO:45 | CUR-1266 | C*C*T*T*A*G*G*G*T*T*G*C*A*G*C*T*A*A*T*T |
| SEQ ID NO:46 | CUR-1294 | A*T*C*C*C*A*G*C*T*A*C*T*C*A*G*G*A*G*G*C |
| SEQ ID NO:47 | CUR-1297 | T*C*T*G*G*C*T*G*A*G*T*G*C*A*G*T*G*G*C*T |
| SEQ ID NO:48 | CUR-1295 | C*C*T*G*G*G*A*G*T*T*G*G*A*G*G*T*T*G*C*A |
| SEQ ID NO:49 | CUR-1296 | C*A*G*A*T*C*C*C*A*T*G*A*A*G*C*C*A*A*G*A*G |
| SEQ ID NO:50 | CUR-1298 | C*T*G*A*C*T*G*C*C*A*T*C*G*A*G*A*A*G*T*G*G |
| SEQ ID NO:51 | CUR-1381 | G*C*C*C*A*T*C*T*G*C*T*T*G*C*T*T*G*A*T |
| SEQ ID NO:52 | CUR-1382 | A*T*C*C*T*C*A*C*C*A*C*A*G*T*C*T*T*G*T |
| SEQ ID NO:53 | CUR-1383 | G*C*T*T*A*C*T*T*C*C*T*C*C*T*C*C*C*T*T*T |
| SEQ ID NO:54 | CUR-1384 | C*C*A*G*G*T*G*A*T*A*G*G*A*G*C*A*G*A*A*C*T |
| SEQ ID NO:55 | CUR-0842 | A*C*C*C*T*C*C*T*T*C*C*T*C*C*C*T*C*T*C*T |
| SEQ ID NO:56 | CUR-0843 | C*C*A*C*T*C*T*C*C*C*T*T*C*T*G*T*C*C*T*C*T |
| SEQ ID NO:57 | CUR-0844 | C*C*T*C*C*T*T*C*C*T*C*C*C*T*C*T*C*T*C*T |
| SEQ ID NO:58 | CUR-0845 | G*T*C*T*G*T*C*C*C*A*T*C*A*T*G*C*C*A*G*G |
| SEQ ID NO:59 | CUR-0846 | T*T*T*C*T*G*A*T*C*C*T*G*C*T*G*C*C*T*C*T |
| SEQ ID NO:60 | CUR-0948 | A*C*C*C*T*C*C*T*T*C*C*T*C*C*C |
| SEQ ID NO:61 | CUR-0949 | C*T*C*C*T*T*C*C*T*C*C*C*T*C |
| SEQ ID NO:62 | CUR-0950 | C*T*C*C*T*T*C*C*T*C*C |
| SEQ ID NO:63 | CUR-0951 | C*T*T*C*C*T*C*C*C*T*C*T*C*T*C |
| SEQ ID NO:64 | CUR-0952 | A*T*C*C*T*G*C*T*G*C*C*T*C*T |
| SEQ ID NO:65 | CUR-1098 | +C*+T*C*C*T*T*C*C*T*C*C*+C*+T*+C |
| SEQ ID NO:66 | CUR-1099 | +A*+C*C*C*T*C*C*T*T*C*C*T*+C*+C*+C |

The modulation of a desired target nucleic acid can be carried out in several ways known in the art. For example, antisense oligonucleotides, siRNA etc. Enzymatic nucleic acid molecules (e.g., ribozymes) are nucleic acid molecules capable of catalyzing one or more of a variety of reactions, including the ability to repeatedly cleave other separate nucleic acid molecules in a nucleotide base sequence-specific manner. Such enzymatic nucleic acid molecules can be used, for example, to target virtually any RNA transcript (Zaug et al., 324, Nature 429 1986; Cech, 260 JAMA 3030, 1988; and Jefferies et al., 17 Nucleic Acids Research 1371, 1989).

Because of their sequence-specificity, trans-cleaving enzymatic nucleic acid molecules show promise as therapeutic agents for human disease (Usman & McSwiggen, (1995) Ann. Rep. Med. Chem. 30, 285-294; Christoffersen and Marr, (1995) J. Med. Chem. 38, 2023-2037). Enzymatic nucleic acid molecules can be designed to cleave specific RNA targets within the background of cellular RNA. Such a cleavage event renders the mRNA non-functional and abrogates protein expression from that RNA. In this manner, synthesis of a protein associated with a disease state can be selectively inhibited.

In general, enzymatic nucleic acids with RNA cleaving activity act by first binding to a target RNA. Such binding occurs through the target binding portion of a enzymatic nucleic acid which is held in close proximity to an enzymatic portion of the molecule that acts to cleave the target RNA. Thus, the enzymatic nucleic acid first recognizes and then binds a target RNA through complementary base pairing, and once bound to the correct site, acts enzymatically to cut the target RNA. Strategic cleavage of such a target RNA will destroy its ability to direct synthesis of an encoded protein. After an enzymatic nucleic acid has bound and cleaved its RNA target, it is released from that RNA to search for another target and can repeatedly bind and cleave new targets.

Several approaches such as in vitro selection (evolution) strategies (Orgel, (1979) Proc. R. Soc. London, B 205, 435) have been used to evolve new nucleic acid catalysts capable of catalyzing a variety of reactions, such as cleavage and ligation of phosphodiester linkages and amide linkages, (Joyce, (1989) Gene, 82, 83-87; Beaudry et al., (1992) Science 257, 635-641; Joyce, (1992) Scientific American 267, 90-97; Breaker et al., (1994) TIBTECH 12, 268; Bartel et al., (1993) Science 261:1411- 1418; Szostak, (1993) TIBS 17, 89-93; Kumar et al., (1995) FASEB J., 9, 1183; Breaker, (1996) Curr. Op. Biotech., 7, 442).

The development of ribozymes that are optimal for catalytic activity would contribute significantly to any strategy that employs RNA-cleaving ribozymes for the purpose of regulating gene expression. The hammerhead ribozyme, for example, functions with a catalytic rate (kcat) of about 1 min-1 in the presence of saturating (10 mM) concentrations of Mg2+ cofactor. An artificial "RNA ligase" ribozyme has been shown to catalyze the corresponding self-modification reaction with a rate of about 100 min-1. In addition, it is known that certain modified hammerhead ribozymes that have substrate binding arms made of DNA catalyze RNA cleavage with multiple turn-over rates that approach 100 min-1. Finally, replacement of a specific residue within the catalytic core of the hammerhead with certain nucleotide analogues gives modified ribozymes that show as much as a 10-fold improvement in catalytic rate. These findings demonstrate that ribozymes can promote chemical transformations with catalytic rates that are significantly greater than those displayed in vitro by most natural self-cleaving ribozymes. It is then possible that the structures of certain selfcleaving ribozymes may be optimized to give maximal catalytic activity, or that entirely new RNA motifs can be made that display significantly faster rates for RNA phosphodiester cleavage.

Intermolecular cleavage of an RNA substrate by an RNA catalyst that fits the "hammerhead" model was first shown in 1987 (Uhlenbeck, O. C. (1987) Nature, 328: 596-600). The RNA catalyst was recovered and reacted with multiple RNA molecules, demonstrating that it was truly catalytic.

Catalytic RNAs designed based on the "hammerhead" motif have been used to cleave specific target sequences by making appropriate base changes in the catalytic RNA to maintain necessary base pairing with the target sequences (Haseloff and Gerlach, (1988) Nature, 334, 585; Walbot and Bruening, (1988) Nature, 334, 196; Uhlenbeck, O. C. (1987) Nature, 328: 596-600; Koizumi, M., et al. (1988) FEBS Lett., 228: 228-230). This has allowed use of the catalytic RNA to cleave specific target sequences and indicates that catalytic RNAs designed according to the "hammerhead" model may possibly cleave specific substrate RNAs in vivo. (see Haseloff and Gerlach, (1988) Nature, 334, 585; Walbot and Bruening, (1988) Nature, 334, 196; Uhlenbeck, O. C. (1987) Nature, 328: 596-600).

RNA interference (RNAi) has become a powerful tool for modulating gene expression in mammals and mammalian cells. This approach requires the delivery of small interfering RNA (siRNA) either as RNA itself or as DNA, using an expression plasmid or virus and the coding sequence for small hairpin RNAs that are processed to siRNAs. This system enables efficient transport of the pre-siRNAs to the cytoplasm where they are active and permit the use of regulated and tissue specific promoters for gene expression.

In a preferred instance, an oligonucleotide or antisense compound comprises an oligomer or polymer of ribonucleic acid (RNA) and/or deoxyribonucleic acid (DNA), or a mimetic, chimera, analog or homolog thereof. This term includes oligonucleotides composed of naturally occurring nucleotides, sugars and covalent internucleoside (backbone) linkages as well as oligonucleotides having non-naturally occurring portions which function similarly. Such modified or substituted oligonucleotides are often desired over native forms because of desirable properties such as, for example, enhanced cellular uptake, enhanced affinity for a target nucleic acid and increased stability in the presence of nucleases.

According to the present disclosure, the oligonucleotides or "antisense compounds" include antisense oligonucleotides (e.g. RNA, DNA, mimetic, chimera, analog or homolog thereof), ribozymes, external guide sequence (EGS) oligonucleotides, siRNA compounds, single- or double-stranded RNA interference (RNAi) compounds such as siRNA compounds, saRNA, aRNA, and other oligomeric compounds which hybridize to at least a portion of the target nucleic acid and modulate its function. As such, they may be DNA, RNA, DNA-like, RNA-like, or mixtures thereof, or may be mimetics of one or more of these. These compounds may be single-stranded, double-stranded, circular or hairpin oligomeric compounds and may contain structural elements such as internal or terminal bulges, mismatches or loops. Antisense compounds are routinely prepared linearly but can be joined or otherwise prepared to be circular and/or branched. Antisense compounds can include constructs such as, for example, two strands hybridized to form a wholly or partially double-stranded compound or a single strand with sufficient self-complementarity to allow for hybridization and formation of a fully or partially double-stranded compound. The two strands can be linked internally leaving free 3' or 5' termini or can be linked to form a continuous hairpin structure or loop. The hairpin structure may contain an overhang on either the 5' or 3' terminus producing an extension of single stranded character. The double stranded compounds optionally can include overhangs on the ends. Further modifications can include conjugate groups attached to one of the termini, selected nucleotide positions, sugar positions or to one of the internucleoside linkages. Alternatively, the two strands can be linked via a non-nucleic acid moiety or linker group. When formed from only one strand, dsRNA can take the form of a self-complementary hairpin-type molecule that doubles back on itself to form a duplex. Thus, the dsRNAs can be fully or partially double stranded. Specific modulation of gene expression can be achieved by stable expression of dsRNA hairpins in transgenic cell lines (Hammond et al., (1991) Nat. Rev. Genet., 2, 110-119; Matzke et al., (2001) Curr. Opin. Genet. Dev., 11, 221-227; Sharp, (2001) Genes Dev., 15, 485-490). When formed from two strands, or a single strand that takes the form of a self-complementary hairpin-type molecule doubled back on itself to form a duplex, the two strands (or duplex-forming regions of a single strand) are complementary RNA strands that base pair in Watson-Crick fashion.

Once introduced to a system, the compounds of the disclosure may elicit the action of one or more enzymes or structural proteins to effect cleavage or other modification of the target nucleic acid or may work via occupancy-based mechanisms. In general, nucleic acids (including oligonucleotides) may be described as "DNA-like" (i.e., generally having one or more 2'-deoxy sugars and, generally, T rather than U bases) or "RNA-like" (i.e., generally having one or more 2'- hydroxyl or 2'-modified sugars and, generally U rather than T bases). Nucleic acid helices can adopt more than one type of structure, most commonly the A- and B-forms. It is believed that, in general, oligonucleotides which have B-form-like structure are "DNA-like" and those which have A-formlike structure are "RNA-like." In some (chimeric) instances, an antisense compound may contain both A- and B-form regions.

The antisense compounds in accordance with this disclosure can comprise an antisense portion from about 5 to about 80 nucleotides (i.e. from about 5 to about 80 linked nucleosides) in length. This refers to the length of the antisense strand or portion of the antisense compound. In other words, a single-stranded antisense compound of the disclosure comprises from 5 to about 80 nucleotides, and a double-stranded antisense compound of the disclosure (such as a dsRNA, for example) comprises a sense and an antisense strand or portion of 5 to about 80 nucleotides in length. One of ordinary skill in the art will appreciate that this comprehends antisense portions of 5, 6, 7,8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, or 80 nucleotides in length, or any range therewithin.

In one instance, the antisense compounds of the disclosure have antisense portions of 10 to 50 nucleotides in length. One having ordinary skill in the art will appreciate that this embodies oligonucleotides having antisense portions of 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 nucleotides in length, or any range therewithin. In some instances, the oligonucleotides are 15 nucleotides in length.

In one instance, the antisense or oligonucleotide compounds of the disclosure have antisense portions of 12 or 13 to 30 nucleotides in length. One having ordinary skill in the art will appreciate that this embodies antisense compounds having antisense portions of 12, 13, 14, 15, 16, 17, 18, 19, 20, 21, 22, 23, 24, 25, 26, 27, 28, 29 or 30 nucleotides in length, or any range therewithin.

In a preferred instance, administration of at least one oligonucleotide targeting any one or more polynucleotides of Sirtuin 1 (SIRT1), prevents or treats diseases associated with abnormal expression or function of Sirtuin 1 (SIRT1) polynucleotides and encoded products thereof, or other related diseases. Examples of diseases which can be treated with the antisense oligonucleotides comprise: cancer (e.g., breast cancer, colorectal cancer, CCL, CML, prostate cancer), a neurodegenerative disease or disorder (e.g., Alzheimer's Disease (AD), Huntington's disease, Parkinson's disease, Amyotrophic Lateral Sclerosis (ALS), Multiple Sclerosis, and disorders caused by polyglutamine aggregation); skeletal muscle disease (e.g., Duchene muscular dystrophy, skeletal muscle atrophy, Becker's dystrophy, or myotonic dystrophy); a metabolic disease (e.g., insulin resistance, diabetes, obesity, impaired glucose tolerance, high blood cholesterol, hyperglycemia, dyslipidemia and hyperlipidemia); adult-onset diabetes, diabetic nephropathy, neuropathy (e.g., sensory neuropathy, autonomic neuropathy, motor neuropathy, retinopathy); bone disease (e.g., osteoporosis), a blood disease (e.g., a leukemia); liver disease (e.g., due to alcohol abuse or hepatitis); obesity; bone resorption, age-related macular degeneration, AIDS related dementia, ALS, Bell's Palsy, atherosclerosis, a cardiac disease (e.g., cardiac dysrhymias, chronic congestive heart failure, ischemic stroke, coronary artery disease and cardiomyopathy), chronically degenerative disease (e.g., cardiac muscle disease), chronic renal failure, type 2 diabetes, ulceration, cataract, presbiopia, glomerulonephritis, Guillan-Barre syndrome, hemorrhagic stroke, rheumatoid arthritis, inflammatory bowel disease, SLE, Crohn's disease, osteoarthritis, osteoporosis, Chronic Obstructive Pulmonary Disease (COPD), pneumonia, skin aging, urinary incontinence, a disease or disorder associated with mitochondrial dysfunction (e.g., mitochondrial myopathy, encephalopathy, Leber's disease, Leigh cnccphalopathia, Pearson's disease, lactic acidosis, 'mitochondrial encephalopathy, lactic acidosis and stroke like symptoms' (MELAS) etc.) and a disease or disorder associated with neuronal cell death, aging or other condition characterized by unwanted cell loss.

In instances of the present disclosure, therapeutic and/or cosmetic regimes and related tailored treatments are provided to subjects requiring skin treatments or at risk of developing conditions for which they would require skin treatments. Diagnosis can be made, e.g., based on the subject's SIRT1 status. A patient's SIRT1 expression levels in a given tissue such as skin can be determined by methods known to those of skill in the art and described elsewhere herein, e.g., by analyzing tissue using PCR or antibody-based detection methods.

A preferred instance of the present disclosure provides a composition for skin treatment and/or a cosmetic application comprising SIRT1 antisense oligonucleotides, e.g., to upregulate expression of SIRT1 in the skin. Examples of antisense oligonucleotides are set forth as SEQ ID NOS: 3 to 66. U.S. Pat. No. 7,544,497, "Compositions for manipulating the lifespan and stress response of cells and organisms,"
describes potential cosmetic use for agents that modulate SIRT1 activity by reducing the Kₘ of the SIRT1 protein for its substrate. In instances, cells are treated in vivo with the oligonucleotides of the present disclosure, to increase cell lifespan or prevent apoptosis. For example, skin can be protected from aging, e.g., developing wrinkles, by treating skin, e.g., epithelial cells, as described herein. In an exemplary instance, skin is contacted with a pharmaceutical or cosmetic composition comprising a SIRT1 antisense compound as described herein. Exemplary skin afflictions or skin conditions include disorders or diseases associated with or caused by inflammation, sun damage or natural aging. For example, the compositions find utility in the prevention or treatment of contact dermatitis (including irritant contact dermatitis and allergic contact dermatitis), atopic dermatitis (also known as allergic eczema), actinic keratosis, keratinization disorders (including eczema), epidermolysis bullosa diseases (including penfigus), exfoliative dermatitis, seborrheic dermatitis, erythemas (including erythema multiforme and erythema nodosum), damage caused by the sun or other light sources, discoid lupus erythematosus, dermatomyositis, skin cancer and the effects of natural aging.

SIRT1 has been reported to interfere with dihydrotestosterone-induced androgen receptor signaling. (See, e.g., Fu, et al., 2006, "Hormonal Control of Androgen Receptor Function through SIRT1," Molecular and Cellular Biology 26(21): 8122-8135.)

In instances of the present disclosure, a composition comprising SIRT1 antisense oligonucleotides, e.g., to upregulate expression of SIRT1 in the scalp and inhibit androgen receptor signaling, thereby preventing androgenetic alopecia (hair loss). In instances, a patient suffering from alopecia is administered either a topical or systemic formulation.

In an instance, an antisense oligonucleotide described herein is incorporated into a topical formulation containing a topical carrier that is generally suited to topical drug administration and comprising any such material known in the art. The topical carrier may be selected so as to provide the composition in the desired form, e.g., as an ointment, lotion, cream, microemulsion, gel, oil, solution, or the like, and may be comprised of a material of either naturally occurring or synthetic origin. It is preferable that the selected carrier not adversely affect the active agent or other components of the topical formulation. Examples of suitable topical carriers for use herein include water, alcohols and other nontoxic organic solvents, glycerin, mineral oil, silicone, petroleum jelly, lanolin, fatty acids, vegetable oils, parabens, waxes, and the like. Formulations may be colorless, odorless ointments, lotions, creams, microemulsions and gels.

Antisense oligonucleotides of the disclosure may be incorporated into ointments, which generally are semisolid preparations which are typically based on petrolatum or other petroleum derivatives. The specific ointment base to be used, as will be appreciated by those skilled in the art, is one that will provide for optimum drug delivery, and, preferably, will provide for other desired characteristics as well, e.g., emolliency or the like. As with other carriers or vehicles, an ointment base should be inert, stable, nonirritating and nonsensitizing. As explained in Remington's Pharmaceutical Sciences (Mack Pub. Co.), ointment bases may be grouped into four classes: oleaginous bases; emulsifiable bases; emulsion bases; and water-soluble bases. Oleaginous ointment bases include, for example, vegetable oils, fats obtained from animals, and semisolid hydrocarbons obtained from petroleum. Emulsifiable ointment bases, also known as absorbent ointment bases, contain little or no water and include, for example, hydroxystearin sulfate, anhydrous lanolin and hydrophilic petrolatum. Emulsion ointment bases are either water-in-oil (W/O) emulsions or oil-in-water (O/W) emulsions, and include, for example, cetyl alcohol, glyceryl monostearate, lanolin and stearic acid. Exemplary water-soluble ointment bases are prepared from polyethylene glycols (PEGs) of varying molecular weight (see, e.g., Remington's, supra).

Antisense oligonucleotides of the disclosure may be incorporated into lotions, which generally are preparations to be applied to the skin surface without friction, and are typically liquid or semiliquid preparations in which solid particles, including the active agent, are present in a water or alcohol base. Lotions are usually suspensions of solids, and may comprise a liquid oily emulsion of the oil-in-water type. Lotions are preferred formulations for treating large body areas, because of the ease of applying a more fluid composition. It is generally necessary that the insoluble matter in a lotion be finely divided. Lotions will typically contain suspending agents to produce better dispersions as well as compounds useful for localizing and holding the active agent in contact with the skin, e.g., methylcellulose, sodium carboxymethylcellulose, or the like. An exemplary lotion formulation for use in conjunction with the present method contains propylene glycol mixed with a hydrophilic petrolatum such as that which may be obtained under the trademark Aquaphor.sup.RTM from Beiersdorf, Inc. (Norwalk, Conn.).

Antisense oligonucleotides of the disclosure may be incorporated into creams, which generally are viscous liquid or semisolid emulsions, either oil-in-water or water-in-oil. Cream bases are water-washable, and contain an oil phase, an emulsifier and an aqueous phase. The oil phase is generally comprised of petrolatum and a fatty alcohol such as cetyl or stearyl alcohol; the aqueous phase usually, although not necessarily, exceeds the oil phase in volume, and generally contains a humectant. The emulsifier in a cream formulation, as explained in Remington's, supra, is generally a nonionic, anionic, cationic or amphoteric surfactant.

Antisense oligonucleotides of the disclosure may be incorporated into microemulsions, which generally are thermodynamically stable, isotropically clear dispersions of two immiscible liquids, such as oil and water, stabilized by an interfacial film of surfactant molecules (Encyclopedia of Pharmaceutical Technology (New York: Marcel Dekker, 1992), volume 9). For the preparation of microemulsions, surfactant (emulsifier), co-surfactant (co-emulsifier), an oil phase and a water phase are necessary. Suitable surfactants include any surfactants that are useful in the preparation of emulsions, e.g., emulsifiers that are typically used in the preparation of creams. The co-surfactant (or "co-emulsifer") is generally selected from the group of polyglycerol derivatives, glycerol derivatives and fatty alcohols. Preferred emulsifier/co-emulsifier combinations are generally although not necessarily selected from the group consisting of: glyceryl monostearate and polyoxyethylene stearate; polyethylene glycol and ethylene glycol palmitostearate; and caprilic and capric triglycerides and oleoyl macrogolglycerides. The water phase includes not only water but also, typically, buffers, glucose, propylene glycol, polyethylene glycols, preferably lower molecular weight polyethylene glycols (e.g., PEG 300 and PEG 400), and/or glycerol, and the like, while the oil phase will generally comprise, for example, fatty acid esters, modified vegetable oils, silicone oils, mixtures of mono- di- and triglycerides, mono- and di-esters of PEG (e.g., oleoyl macrogol glycerides), etc.

Antisense oligonucleotides of the disclosure may be incorporated into gel formulations, which generally are semisolid systems consisting of either suspensions made up of small inorganic particles (two-phase systems) or large organic molecules distributed substantially uniformly throughout a carrier liquid (single phase gels). Single phase gels can be made, for example, by combining the active agent, a carrier liquid and a suitable gelling agent such as tragacanth (at 2 to 5%), sodium alginate (at 2-10%), gelatin (at 2-15%), methylcellulose (at 3-5%), sodium carboxymethylcellulose (at 2-5%), carbomer (at 0.3-5%) or polyvinyl alcohol (at 10-20%) together and mixing until a characteristic semisolid product is produced. Other suitable gelling agents include methylhydroxycellulose, polyoxyethylene-polyoxypropylene, hydroxyethylcellulose and gelatin. Although gels commonly employ aqueous carrier liquid, alcohols and oils can be used as the carrier liquid as well.

Various additives, known to those skilled in the art, may be included in formulations, e.g., topical formulations. Examples of additives include, but are not limited to, solubilizers, skin permeation enhancers, opacifiers, preservatives (e.g., anti-oxidants), gelling agents, buffering agents, surfactants (particularly nonionic and amphoteric surfactants), emulsifiers, emollients, thickening agents, stabilizers, humectants, colorants, fragrance, and the like. Inclusion of solubilizers and/or skin permeation enhancers is particularly preferred, along with emulsifiers, emollients and preservatives. An optimum topical formulation comprises approximately: 2 wt. % to 60 wt. %, preferably 2 wt. % to 50 wt. %, solubilizer and/or skin permeation enhancer; 2 wt. % to 50 wt. %, preferably 2 wt. % to 20 wt. %, emulsifiers; 2 wt. % to 20 wt. % emollient; and 0.01 to 0.2 wt. % preservative, with the active agent and carrier (e.g., water) making of the remainder of the formulation.

A skin permeation enhancer serves to facilitate passage of therapeutic levels of active agent to pass through a reasonably sized area of unbroken skin. Suitable enhancers are well known in the art and include, for example: lower alkanols such as methanol ethanol and 2-propanol; alkyl methyl sulfoxides such as dimethylsulfoxide (DMSO), decylmethylsulfoxide (C.sub.10 MSO) and tetradecylmethyl sulfboxide; pyrrolidones such as 2-pyrrolidone, N-methyl-2-pyrrolidone and N-(-hydroxyethyl)pyrrolidone; urea; N,N-diethyl-m-toluamide; C.sub.2-C.sub.6 alkanediols; miscellaneous solvents such as dimethyl formamide (DMF), N,N-dimethylacetamide (DMA) and tetrahydrofurfuryl alcohol; and the 1-substituted azacycloheptan-2-ones, particularly 1-n-dodecylcyclazacycloheptan-2-one (laurocapram; available under the trademark Azone.sup.RTM from Whitby Research Incorporated, Richmond, Va.).

Examples of solubilizers include, but are not limited to, the following: hydrophilic ethers such as diethylene glycol monoethyl ether (ethoxydiglycol, available commercially as Transcutol.sup.RTM) and diethylene glycol monoethyl ether oleate (available commercially as Soficutol.sup.RTM); polyethylene castor oil derivatives such as polyoxy 35 castor oil, polyoxy 40 hydrogenated castor oil, etc.; polyethylene glycol, particularly lower molecular weight polyethylene glycols such as PEG 300 and PEG 400, and polyethylene glycol derivatives such as PEG-8 caprylic/capric glycerides (available commercially as Labrasol.sup.RTM); alkyl methyl sulfoxides such as DMSO; pyrrolidones such as 2-pyrrolidone and N-methyl-2-pyrrolidone; and DMA. Many solubilizers can also act as absorption enhancers. A single solubilizer may be incorporated into the formulation, or a mixture of solubilizers may be incorporated therein.

Suitable emulsifiers and co-emulsifiers include, without limitation, those emulsifiers and co-emulsifiers described with respect to microemulsion formulations. Emollients include, for example, propylene glycol, glycerol, isopropyl myristate, polypropylene glycol-2 (PPG-2) myristyl ether propionate, and the like.

Other active agents may also be included in formulations, e.g., other antiinflammatory agents, analgesics, antimicrobial agents, antifungal agents, antibiotics, vitamins, antioxidants, and sunblock agents commonly found in sunscreen formulations including, but not limited to, anthranilates, benzophenones (particularly benzophenone-3), camphor derivatives, cinnamates (e.g., octyl methoxycinnamate), dibenzoyl methanes (e.g., butyl methoxydibenzoyl methane), p-aminobenzoic acid (PABA) and derivatives thereof, and salicylates (e.g., octyl salicylate).

In another preferred instance, the oligomeric compounds of the present disclosure also include variants in which a different base is present at one or more of the nucleotide positions in the compound. For example, if the first nucleotide is an adenosine, variants may be produced which contain thymidine, guanosine or cytidine at this position. This may be done at any of the positions of the antisense or dsRNA compounds. These compounds are then tested using the methods described herein to determine their ability to inhibit expression of a target nucleic acid.

In some instances, homology, sequence identity or complementarity, between the antisense compound and target is from about 40% to about 60%. In some instances, homology, sequence identity or complementarity, is from about 60% to about 70%. In some instances, homology, sequence identity or complementarity, is from about 70% to about 80%. In some instances, homology, sequence identity or complementarity, is from about 80% to about 90%. In some instances, homology, sequence identity or complementarity, is about 90%, about 92%, about 94%, about 95%, about 96%, about 97%, about 98%, about 99% or about 100%.

In another preferred instance, the antisense oligonucleotides, such as for example, nucleic acid molecules set forth in SEQ ID NOS: 9 to 66 comprise one or more substitutions or modifications. In one instance, the nucleotides are substituted with locked nucleic acids (LNA).

In another preferred instance, the oligonucleotides target one or more regions of the nucleic acid molecules sense and/or antisense of coding and/or non-coding sequences associated with SIRT1 and the sequences set forth as SEQ ID NOS: 1 to 8. The oligonucleotides are also targeted to overlapping regions of SEQ ID NOS: 1 to 8.

Certain preferred oligonucleotides of this disclosure are chimeric oligonucleotides. "Chimeric oligonucleotides" or "chimeras," in the context of this disclosure, are oligonucleotides which contain two or more chemically distinct regions, each made up of at least one nucleotide. These oligonucleotides typically contain at least one region of modified nucleotides that confers one or more beneficial properties (such as, for example, increased nuclease resistance, increased uptake into cells, increased binding affinity for the target) and a region that is a substrate for enzymes capable of cleaving RNA:DNA or RNA:RNA hybrids. By way of example, RNase H is a cellular endonuclease which cleaves the RNA strand of an RNA:DNA duplex. Activation of RNase H, therefore, results in cleavage of the RNA target, thereby greatly enhancing the efficiency of antisense modulation of gene expression. Consequently, comparable results can often be obtained with shorter oligonucleotides when chimeric oligonucleotides are used, compared to phosphorothioate deoxyoligonucleotides hybridizing to the same target region. Cleavage of the RNA target can be routinely detected by gel electrophoresis and, if necessary, associated nucleic acid hybridization techniques known in the art. In one preferred instance, a chimeric oligonucleotide comprises at least one region modified to increase target binding affinity, and, usually, a region that acts as a substrate for RNAse H. Affinity of an oligonucleotide for its target (in this case, a nucleic acid encoding ras) is routinely determined by measuring the Tm of an oligonucleotide/target pair, which is the temperature at which the oligonucleotide and target dissociate; dissociation is detected spectrophotometrically. The higher the Tm, the greater is the affinity of the oligonucleotide for the target.

Chimeric antisense compounds of the disclosure may be formed as composite structures of two or more oligonucleotides, modified oligonucleotides, oligonucleosides and/or oligonucleotides mimetics as described above. Such; compounds have also been referred to in the art as hybrids or gapmers. Representative United States patents that teach the preparation of such hybrid structures comprise, but are not limited to, US patent nos. 5,013,830; 5,149,797; 5, 220,007; 5,256,775; 5,366,878; 5,403,711; 5,491,133; 5,565,350; 5,623,065; 5,652,355; 5,652,356; and 5,700,922.

In another preferred instance, the region of the oligonucleotide which is modified comprises at least one nucleotide modified at the 2' position of the sugar, most preferably a 2'-Oalkyl, 2'-O-alkyl-O-alkyl or 2'-fluoro-modified nucleotide. In other preferred instances, RNA modifications include 2'-fluoro, 2'-amino and 2' O-methyl modifications on the ribose of pyrimidines, abasic residues or an inverted base at the 3' end of the RNA. Such modifications are routinely incorporated into oligonucleotides and these oligonucleotides have been shown to have a higher Tm (i.e., higher target binding affinity) than; 2'-deoxyoligonucleotides against a given target. The effect of such increased affinity is to greatly enhance RNAi oligonucleotide inhibition of gene expression. RNAse H is a cellular endonuclease that cleaves the RNA strand of RNA:DNA duplexes; activation of this enzyme therefore results in cleavage of the RNA target, and thus can greatly enhance the efficiency of RNAi inhibition. Cleavage of the RNA target can be routinely demonstrated by gel electrophoresis. In another preferred instance, the chimeric oligonucleotide is also modified to enhance nuclease resistance. Cells contain a variety of exo- and endo-nucleases which can degrade nucleic acids. A number of nucleotide and nucleoside modifications have been shown to make the oligonucleotide into which they are incorporated more resistant to nuclease digestion than the native oligodeoxynucleotide. Nuclease resistance is routinely measured by incubating oligonucleotides with cellular extracts or isolated nuclease solutions and measuring the extent of intact oligonucleotide remaining over time, usually by gel electrophoresis. Oligonucleotides which have been modified to enhance their nuclease resistance survive intact for a longer time than unmodified oligonucleotides. A variety of oligonucleotide modifications have been demonstrated to enhance or confer nuclease resistance. Oligonucleotides which contain at least one phosphorothioate modification are presently more preferred. In some cases, oligonucleotide modifications which enhance target binding affinity are also, independently, able to enhance nuclease resistance. Some desirable modifications can be found in De Mesmaeker et al. (1995) Acc. Chem. Res., 28:366-374.

Specific examples of some preferred oligonucleotides envisioned for this disclosure include those comprising modified backbones, for example, phosphorothioates, phosphotriesters, methyl phosphonates, short chain alkyl or cycloalkyl intersugar linkages or short chain heteroatomic or heterocyclic intersugar linkages. Most preferred are oligonucleotides with phosphorothioate backbones and those with heteroatom backbones, particularly CH2 --NH--O--CH2, CH,--N(CH3)--O--CH2 [known as a methylene(methylimino) or MMI backbone], CH2 --O--N (CH3)--CH2, CH2 -N (CH3)--N (CH3)--CH2 and O--N (CH3)--CH2 --CH2 backbones, wherein the native phosphodiester backbone is represented as O--P--O--CH,). The amide backbones disclosed by De Mesmaeker et al. (1995) Acc. Chem. Res. 28:366-374 are also preferred. Also preferred are oligonucleotides having morpholino backbone structures (Summerton and Weller, U.S. Pat. No. 5,034,506). In other preferred instances, such as the peptide nucleic acid (PNA) backbone, the phosphodiester backbone of the oligonucleotide is replaced with a polyamide backbone, the nucleotides being bound directly or indirectly to the aza nitrogen atoms of the polyamide backbone (Nielsen et al. (1991) Science 254, 1497). Oligonucleotides may also comprise one or more substituted sugar moieties. Preferred oligonucleotides comprise one of the following at the 2' position: OH, SH, SCH3, F, OCN, OCH3 OCH3, OCH3 O(CH2)n CH3, O(CH2)n NH2 or O(CH2)n CH3 where n is from 1 to about 10; C1 to C10 lower alkyl, alkoxyalkoxy, substituted lower alkyl, alkaryl or aralkyl; Cl; Br; CN; CF3 ; OCF3; O--, S--, or N-alkyl; O--, S--, or N-alkenyl; SOCH3; SO2 CH3; ONO2; NO2; N3; NH2; heterocycloalkyl; heterocycloalkaryl; aminoalkylamino; polyalkylamino; substituted silyl; an RNA cleaving group; a reporter group; an intercalator; a group for improving the pharmacokinetic properties of an oligonucleotide; or a group for improving the pharmacodynamic properties of an oligonucleotide and other substituents having similar properties. A preferred modification includes 2'-methoxyethoxy [2'-O-CH2 CH2 OCH3, also known as 2'-O-(2-methoxyethyl)] (Martin et al., (1995) Helv. Chim. Acta, 78, 486). Other preferred modifications include 2'-methoxy (2'-O--CH3), 2'- propoxy (2'-OCH2 CH2CH3) and 2'-fluoro (2'-F). Similar modifications may also be made at other positions on the oligonucleotide, particularly the 3' position of the sugar on the 3' terminal nucleotide and the 5' position of 5' terminal nucleotide. Oligonucleotides may also have sugar mimetics such as cyclobutyls in place of the pentofuranosyl group.

Oligonucleotides may also include, additionally or alternatively, nucleobase (often referred to in the art simply as "base") modifications or substitutions. As used herein, "unmodified" or "natural" nucleotides include adenine (A), guanine (G), thymine (T), cytosine (C) and uracil (U). Modified nucleotides include nucleotides found only infrequently or transiently in natural nucleic acids, e.g., hypoxanthine, 6-methyladenine, 5-Me pyrimidines, particularly 5-methylcytosine (also referred to as 5-methyl-2' deoxycytosine and often referred to in the art as 5-Me-C), 5- hydroxymethylcytosine (HMC), glycosyl HMC and gentobiosyl HMC, as well as synthetic nucleotides, e.g., 2-aminoadenine, 2-(methylamino)adenine, 2-(imidazolylalkyl)adenine, 2- (aminoalklyamino)adenine or other heterosubstituted alkyladenines, 2-thiouracil, 2-thiothymine, 5- bromouracil, 5-hydroxymethyluracil, 8-azaguanine, 7-deazaguanine, N6 (6-aminohexyl)adenine and 2,6-diaminopurine. (Kornberg, A., DNA Replication, W. H. Freeman & Co., San Francisco, 1980, pp75-77; Gebeyehu, G., (1987) et al. Nucl. Acids Res. 15:4513). A "universal" base known in the art, e.g., inosine, may be included. 5-Me-C substitutions have been shown to increase nucleic acid duplex stability by 0.6-1.2°C. (Sanghvi, Y. S., in Crooke, S. T. and Lebleu, B., eds., Antisense Research and Applications, CRC Press, Boca Raton, 1993, pp. 276-278) and are presently preferred base substitutions.

Another modification of the oligonucleotides of the disclosure involves chemically linking to the oligonucleotide one or more moieties or conjugates which enhance the activity or cellular uptake of the oligonucleotide. Such moieties include but are not limited to lipid moieties such as a cholesterol moiety, a cholesteryl moiety (Letsinger et al., (1989) Proc. Natl. Acad. Sci. USA 86, 6553), cholic acid (Manoharan et al. (1994) Bioorg. Med. Chem. Let. 4, 1053), a thioether, e.g., hexyl-S-tritylthiol (Manoharan et al. (1992) Ann. N.Y. Acad. Sci. 660, 306; Manoharan et al. (1993) Bioorg. Med. Chem. Let. 3, 2765), a thiocholesterol (Oberhauser et al., (1992) Nucl. Acids Res. 20, 533), an aliphatic chain, e.g., dodecandiol or undecyl residues (Saison-Behmoaras et al. EMBO J. 1991, 10, 111; Kabanov et al. (1990) FEBS Lett. 259, 327; Svinarchuk et al. (1993) Biochimie 75, 49), a phospholipid, e.g., di-hexadecyl-rac-glycerol or triethylammonium 1,2-di-O-hexadecyl-rac-glycero- 3-H-phosphonate (Manoharan et al. (1995) Tetrahedron Lett. 36, 3651; Shea et al. (1990) Nucl. Acids Res. 18, 3777), a polyamine or a polyethylene glycol chain (Manoharan et al. (1995) Nucleosides & Nucleotides, 14, 969), or adamantane acetic acid (Manoharan et al. (1995) Tetrahedron Lett. 36, 3651). Oligonucleotides comprising lipophilic moieties, and methods for preparing such oligonucleotides are known in the art, for example, U.S. Pat. Nos. 5,138,045, 5,218,105 and 5,459,255.

It is not necessary for all positions in a given oligonucleotide to be uniformly modified, and in fact more than one of the aforementioned modifications may be incorporated in a single oligonucleotide or even at within a single nucleoside within an oligonucleotide. The present disclosure also includes oligonucleotides which are chimeric oligonucleotides as hereinbefore defined.

In another instance, the nucleic acid molecule of the present disclosure is conjugated with another moiety including but not limited to abasic nucleotides, polyether, polyamine, polyamides, peptides, carbohydrates, lipid, or polyhydrocarbon compounds. Those skilled in the art will recognize that these molecules can be linked to one or more of any nucleotides comprising the nucleic acid molecule at several positions on the sugar, base or phosphate group.

The oligonucleotides used in accordance with this disclosure may be conveniently and routinely made through the well-known technique of solid phase synthesis. Equipment for such synthesis is sold by several vendors including Applied Biosystems. Any other means for such synthesis may also be employed; the actual synthesis of the oligonucleotides is well within the talents of one of ordinary skill in the art. It is also well known to use similar techniques to prepare other oligonucleotides such as the phosphorothioates and alkylated derivatives. It is also well known to use similar techniques and commercially available modified amidites and controlled-pore glass (CPG) products such as biotin, fluorescein, acridine or psoralen-modified amidites and/or CPG (available from Glen Research, Sterling VA) to synthesize fluorescently labeled, biotinylated or other modified oligonucleotides such as cholesterol-modified oligonucleotides.

In accordance with the disclosure, use of modifications such as the use of LNA monomers to enhance the potency, specificity and duration of action and broaden the routes of administration of oligonucleotides comprised of current chemistries such as MOE, ANA, FANA, PS etc (Uhlman, et al. (2000) Current Opinions in Drug Discovery & Development Vol. 3 No 2). This can be achieved by substituting some of the monomers in the current oligonucleotides by LNA monomers. The LNA modified oligonucleotide may have a size similar to the parent compound or may be larger or preferably smaller. It is preferred that such LNA-modified oligonucleotides contain less than about 70%, more preferably less than about 60%, most preferably less than about 50% LNA monomers and that their sizes are between about 5 and 25 nucleotides, more preferably between about 12 and 20 nucleotides.

Preferred modified oligonucleotide backbones comprise, but not limited to, phosphorothioates, chiral phosphorothioates, phosphorodithioates, phosphotriesters, aminoalkylphosphotriesters, methyl and other alkyl phosphonates comprising 3'alkylene phosphonates and chiral phosphonates, phosphinates, phosphoramidates comprising 3'-amino phosphoramidate and aminoalkylphosphoramidates, thionophosphoramidates, thionoalkylphosphonates, thionoalkylphosphotriesters, and boranophosphates having normal 3'-5' linkages, 2'-5' linked analogs of these, and those having inverted polarity wherein the adjacent pairs of nucleoside units are linked 3'-5' to 5'-3' or 2'-5' to 5'-2'. Various salts, mixed salts and free acid forms are also included.

Representative United States patents that teach the preparation of the above phosphorus containing linkages comprise, but are not limited to, US patent nos. 3,687,808; 4,469,863; 4,476,301; 5,023,243; 5, 177,196; 5,188,897; 5,264,423; 5,276,019; 5,278,302; 5,286,717; 5,321,131; 5,399,676; 5,405,939; 5,453,496; 5,455, 233; 5,466,677; 5,476,925; 5,519,126; 5,536,821; 5,541,306; 5,550,111; 5,563, 253; 5,571,799; 5,587,361; and 5,625,050.

Preferred modified oligonucleotide backbones that do not include a phosphorus atom therein have backbones that are formed by short chain alkyl or cycloalkyl internucleoside linkages, mixed heteroatom and alkyl or cycloalkyl internucleoside linkages, or one or more short chain heteroatomic or heterocyclic internucleoside linkages. These comprise those having morpholino linkages (formed in part from the sugar portion of a nucleoside); siloxane backbones; sulfide, sulfoxide and sulfone backbones; formacetyl and thioformacetyl backbones; methylene formacetyl and thioformacetyl backbones; alkene containing backbones; sulfamate backbones; methyleneimino and methylenehydrazino backbones; sulfonate and sulfonamide backbones; amide backbones; and others having mixed N, O, S and CH2 component parts.

Representative United States patents that teach the preparation of the above oligonucleosides comprise, but are not limited to, US patent nos. 5,034,506; 5,166,315; 5,185,444; 5,214,134; 5,216,141; 5,235,033; 5,264, 562; 5, 264,564; 5,405,938; 5,434,257; 5,466,677; 5,470,967; 5,489,677; 5,541,307; 5,561,225; 5,596, 086; 5,602,240; 5,610,289; 5,602,240; 5,608,046; 5,610,289; 5,618,704; 5,623, 070; 5,663,312; 5,633,360; 5,677,437; and 5,677,439 .

In other preferred oligonucleotide mimetics, both the sugar and the internucleoside linkage, i.e., the backbone, of the nucleotide units are replaced with novel groups. The base units are maintained for hybridization with an appropriate nucleic acid target compound. One such oligomeric compound, an oligonucleotide mimetic that has been shown to have excellent hybridization properties, is referred to as a peptide nucleic acid (PNA). In PNA compounds, the sugar-backbone of an oligonucleotide is replaced with an amide containing backbone, in particular an aminoethylglycine backbone. The nucleobases are retained and are bound directly or indirectly to aza nitrogen atoms of the amide portion of the backbone. Representative United States patents that teach the preparation of PNA compounds comprise, but are not limited to, US patent nos. 5,539,082; 5,714,331; and 5,719,262.

Further teaching of PNA compounds can be found in Nielsen, et al. (1991) Science 254, 1497-1500.

In another preferred instance of the disclosure the oligonucleotides with phosphorothioate backbones and oligonucleosides with heteroatom backbones, and in particular- CH2-NH-O-CH2-,-CH2-N (CH3)-O-CH2-known as a methylene (methylimino) or MMI backbone,- CH2-O-N (CH3)-CH2-,-CH2N(CH3)-N(CH3) CH2-and-O-N(CH3)-CH2-CH2- wherein the native phosphodiester backbone is represented as-O-P-O-CH2- of the above referenced US patent no. 5,489,677, and the amide backbones of the above referenced US patent no. 5,602,240. Also preferred are oligonucleotides having morpholino backbone structures of the above-referenced US patent no. 5,034,506.

Modified oligonucleotides may also contain one or more substituted sugar moieties. Preferred oligonucleotides comprise one of the following at the 2' position: OH; F; O-, S-, or N-alkyl; O-, S-, or N-alkenyl; O-, S-or N-alkynyl; or O alkyl-O-alkyl, wherein the alkyl, alkenyl and alkynyl may be substituted or unsubstituted C to CO alkyl or C2 to CO alkenyl and alkynyl. Particularly preferred are O (CH2)n OmCH3, O(CH2)n,OCH3, O(CH2)nNH2, O(CH2)nCH3, O(CH2)nONH2, and O(CH2nON(CH2)nCH3)2 where n and m can be from 1 to about 10. Other preferred oligonucleotides comprise one of the following at the 2' position: C to CO, (lower alkyl, substituted lower alkyl, alkaryl, aralkyl, O-alkaryl or O-aralkyl, SH, SCH3, OCN, Cl, Br, CN, CF3, OCF3, SOCH3, SO2CH3, ONO2, NO2, N3, NH2, heterocycloalkyl, heterocycloalkaryl, aminoalkylamino, polyalkylamino, substituted silyl, an RNA cleaving group, a reporter group, an intercalator, a group for improving the pharmacokinetic properties of an oligonucleotide, or a group for improving the pharmacodynamic properties of an oligonucleotide, and other substituents having similar properties. A preferred modification comprises 2'-methoxyethoxy (2'-O-CH2CH2OCH3, also known as 2'-O-(2- methoxyethyl) or 2'-MOE) (Martin et al., (1995) Helv. Chim. Acta, 78, 486-504) i.e., an alkoxyalkoxy group. A further preferred modification comprises 2'-dimethylaminooxyethoxy, i.e. , a O(CH2)2ON(CH3)2 group, also known as 2'-DMAOE, as described in examples herein below, and 2'- dimethylaminoethoxyethoxy (also known in the art as 2'-O-dimethylaminoethoxyethyl or 2'- DMAEOE), i.e., 2'-O-CH2-O-CH2-N (CH2)2.

Other preferred modifications comprise 2'-methoxy (2'-O CH3), 2'-aminopropoxy (2'-O CH2CH2CH2NH2) and 2'-fluoro (2'-F). Similar modifications may also be made at other positions on the oligonucleotide, particularly the 3' position of the sugar on the 3' terminal nucleotide or in 2'-5' linked oligonucleotides and the 5' position of 5' terminal nucleotide. Oligonucleotides may also have sugar mimetics such as cyclobutyl moieties in place of the pentofuranosyl sugar. Representative United States patents that teach the preparation of such modified sugar structures comprise, but are not limited to, US patent nos. 4,981,957; 5,118,800; 5,319,080; 5,359,044; 5,393,878; 5,446,137; 5,466,786; 5,514, 785; 5,519,134; 5,567,811; 5,576,427; 5,591,722; 5,597,909; 5,610,300; 5,627,053; 5,639,873; 5,646, 265; 5,658,873; 5,670,633; and 5,700,920 .

Oligonucleotides may also comprise nucleobase (often referred to in the art simply as "base") modifications or substitutions. As used herein, "unmodified" or "natural" nucleotides comprise the purine bases adenine (A) and guanine (G), and the pyrimidine bases thymine (T), cytosine (C) and uracil (U). Modified nucleotides comprise other synthetic and natural nucleotides such as 5-methylcytosine (5-me-C), 5-hydroxymethyl cytosine, xanthine, hypoxanthine, 2- aminoadenine, 6-methyl and other alkyl derivatives of adenine and guanine, 2-propyl and other alkyl derivatives of adenine and guanine, 2-thiouracil, 2-thiothymine and 2-thiocytosine, 5-halouracil and cytosine, 5-propynyl uracil and cytosine, 6-azo uracil, cytosine and thymine, 5-uracil (pseudo-uracil), 4-thiouracil, 8-halo, 8-amino, 8-thiol, 8-thioalkyl, 8-hydroxyl and other 8-substituted adenines and guanines, 5-halo particularly 5-bromo, 5-trifluoromethyl and other 5-substituted uracils and cytosines, 7-methylquanine and 7-methyladenine, 8-azaguanine and 8-azaadenine, 7-deazaguanine and 7-deazaadenine and 3-deazaguanine and 3-deazaadenine.

Further, nucleotides comprise those disclosed in United States Patent No. 3,687,808, those disclosed in 'The Concise Encyclopedia of Polymer Science And Engineering', pages 858-859, Kroschwitz, J.I., ed. John Wiley & Sons, 1990, those disclosed by Englisch et al., 'Angewandle Chemie, International Edition', 1991, 30, page 613, and those disclosed by Sanghvi, Y.S., Chapter 15, 'Antisense Research and Applications', pages 289-302, Crooke, S.T. and Lebleu, B. ea., CRC Press, 1993. Certain of these nucleotides are particularly useful for increasing the binding affinity of the oligomeric compounds of the disclosure. These comprise 5-substituted pyrimidines, 6- azapyrimidines and N-2, N-6 and 0-6 substituted purines, comprising 2-aminopropyladenine, 5- propynyluracil and 5-propynylcytosine. 5-methylcytosine substitutions have been shown to increase nucleic acid duplex stability by 0.6-1.2°C (Sanghvi, Y.S., Crooke, S.T. and Lebleu, B., eds, 'Antisense Research and Applications', CRC Press, Boca Raton, 1993, pp. 276-278) and are presently preferred base substitutions, even more particularly when combined with 2'-Omethoxyethyl sugar modifications.

Representative United States patents that teach the preparation of the above noted modified nucleotides as well as other modified nucleotides comprise, but are not limited to, US patent nos. 3,687,808, as well as 4,845,205; 5,130,302; 5,134,066; 5,175, 273; 5, 367,066; 5,432,272; 5,457,187; 5,459,255; 5,484,908; 5,502,177; 5,525,711; 5,552,540; 5,587,469; 5,596,091; 5,614,617; 5,750,692, and 5,681,941.

Another modification of the oligonucleotides of the disclosure involves chemically linking to the oligonucleotide one or more moieties or conjugates, which enhance the activity, cellular distribution, or cellular uptake of the oligonucleotide.

Such moieties comprise but are not limited to, lipid moieties such as a cholesterol moiety (Letsinger et al., (1989) Proc. Natl. Acad. Sci. USA, 86, 6553-6556), cholic acid (Manoharan et al., (1994) Bioorg. Med. Chem. Let., 4, 1053-1060), a thioether, e.g., hexyl-S-tritylthiol (Manoharan et al., (1992) Ann. N. Y. Acad. Sci., 660, 306-309; Manoharan et al., (1993) Bioorg. Med. Chem. Let., 3, 2765-2770), a thiocholesterol (Oberhauser et al., (1992) Nucl. Acids Res., 20, 533-538), an aliphatic chain, e.g., dodecandiol or undecyl residues (Kabanov et al., (1990) FEBS Lett., 259, 327-330; Svinarchuk et al., (1993) Biochimie 75, 49-54), a phospholipid, e.g., di-hexadecyl-rac-glycerol or triethylammonium 1,2-di-O-hexadecyl-rac-glycero-3-H-phosphonate (Manoharan et al., (1995) Tetrahedron Lett., 36, 3651-3654; Shea et al., (1990) Nucl. Acids Res., 18, 3777-3783), a polyamine or a polyethylene glycol chain (Mancharan et al., (1995) Nucleosides & Nucleotides, 14, 969-973), or adamantane acetic acid (Manoharan et al., (1995) Tetrahedron Lett., 36, 3651-3654), a palmityl moiety (Mishra et al., (1995) Biochim. Biophys. Acta, 1264, 229-237), or an octadecylamine or hexylamino-carbonyl-t oxycholesterol moiety (Crooke et al., (1996) J. Pharmacol. Exp. Ther., 277, 923-937).

Representative United States patents that teach the preparation of such oligonucleotides conjugates comprise, but are not limited to, US patent nos. 4,828,979; 4,948,882; 5,218,105; 5,525,465; 5,541,313; 5,545,730; 5,552, 538; 5,578,717, 5,580,731; 5,580,731; 5,591,584; 5,109,124; 5,118,802; 5,138,045; 5,414,077; 5,486, 603; 5,512,439; 5,578,718; 5,608,046; 4,587,044; 4,605,735; 4,667,025; 4,762, 779; 4,789,737; 4,824,941; 4,835,263; 4,876,335; 4,904,582; 4,958,013; 5,082, 830; 5,112,963; 5,214,136; 5,082,830; 5,112,963; 5,214,136; 5, 245,022; 5,254,469; 5,258,506; 5,262,536; 5,272,250; 5,292,873; 5,317,098; 5,371,241, 5,391, 723; 5,416,203, 5,451,463; 5,510,475; 5,512,667; 5,514,785; 5, 565,552; 5,567,810; 5,574,142; 5,585,481; 5,587,371; 5,595,726; 5,597,696; 5,599,923; 5,599, 928 and 5,688,941.

*Drug discovery:* The compounds of the present disclosure can also be applied in the areas of drug discovery and target validation. The present disclosure comprehends the use of the compounds and preferred target segments identified herein in drug discovery efforts to elucidate relationships that exist between Sirtuin 1 (SIRT1) polynucleotides and a disease state, phenotype, or condition. These methods include detecting or modulating Sirtuin 1 (SIRT1) polynucleotides comprising contacting a sample, tissue, cell, or organism with the compounds of the present disclosure, measuring the nucleic acid or protein level of Sirtuin 1 (SIRT1) polynucleotides and/or a related phenotypic or chemical endpoint at some time after treatment, and optionally comparing the measured value to a non-treated sample or sample treated with a further compound of the disclosure. These methods can also be performed in parallel or in combination with other experiments to determine the function of unknown genes for the process of target validation or to determine the validity of a particular gene product as a target for treatment or prevention of a particular disease, condition, or phenotype.

### Assessing Up-regulation or Inhibition of Gene Expression:

Transfer of an exogenous nucleic acid into a host cell or organism can be assessed by directly detecting the presence of the nucleic acid in the cell or organism. Such detection can be achieved by several methods well known in the art. For example, the presence of the exogenous nucleic acid can be detected by Southern blot or by a polymerase chain reaction (PCR) technique using primers that specifically amplify nucleotide sequences associated with the nucleic acid. Expression of the exogenous nucleic acids can also be measured using conventional methods including gene expression analysis. For instance, mRNA produced from an exogenous nucleic acid can be detected and quantified using a Northern blot and reverse transcription PCR (RT-PCR).

Expression of RNA from the exogenous nucleic acid can also be detected by measuring an enzymatic activity or a reporter protein activity. For example, antisense modulatory activity can be measured indirectly as a decrease or increase in target nucleic acid expression as an indication that the exogenous nucleic acid is producing the effector RNA. Based on sequence conservation, primers can be designed and used to amplify coding regions of the target genes. Initially, the most highly expressed coding region from each gene can be used to build a model control gene, although any coding or non coding region can be used. Each control gene is assembled by inserting each coding region between a reporter coding region and its poly(A) signal. These plasmids would produce an mRNA with a reporter gene in the upstream portion of the gene and a potential RNAi target in the 3' non-coding region. The effectiveness of individual antisense oligonucleotides would be assayed by modulation of the reporter gene. Reporter genes useful in the methods of the present disclosure include acetohydroxyacid synthase (AHAS), alkaline phosphatase (AP), beta galactosidase (LacZ), beta glucoronidase (GUS), chloramphenicol acetyltransferase (CAT), green fluorescent protein (GFP), red fluorescent protein (RFP), yellow fluorescent protein (YFP), cyan fluorescent protein (CFP), horseradish peroxidase (HRP), luciferase (Luc), nopaline synthase

(NOS), octopine synthase (OCS), and derivatives thereof. Multiple selectable markers are available that confer resistance to ampicillin, bleomycin, chloramphenicol, gentamycin, hygromycin, kanamycin, lincomycin, methotrexate, phosphinothricin, puromycin, and tetracycline. Methods to determine modulation of a reporter gene are well known in the art, and include, but are not limited to, fluorometric methods (e.g. fluorescence spectroscopy, Fluorescence Activated Cell Sorting (FACS), fluorescence microscopy), antibiotic resistance determination.

SIRT1 protein and mRNA expression can be assayed using methods known to those of skill in the art and described elsewhere herein. For example, immunoassays such as the ELISA can be used to measure protein levels. SIRT1 antibodies for ELISAs are available commercially, e.g., from R&D Systems (Minneapolis, MN), Abcam, Cambridge, MA.

In instances, SIRT1 expression (e.g., mRNA or protein) in a sample (e.g., cells or tissues in vivo or in vitro) treated using an antisense oligonucleotide of the disclosure is evaluated by comparison with SIRT1 expression in a control sample. For example, expression of the protein or nucleic acid can be compared using methods known to those of skill in the art with that in a mock-treated or untreated sample. Alternatively, comparison with a sample treated with a control antisense oligonucleotide (e.g., one having an altered or different sequence) can be made depending on the information desired. In another instance, a difference in the expression of the SIRT1 protein or nucleic acid in a treated vs. an untreated sample can be compared with the difference in expression of a different nucleic acid (including any standard deemed appropriate by the researcher, e.g., a housekeeping gene) in a treated sample vs. an untreated sample.

Observed differences can be expressed as desired, e.g., in the form of a ratio or fraction, for use in a comparison with control. In instances, the level of SIRT1 mRNA or protein, in a sample treated with an antisense oligonucleotide of the present disclosure, is increased or decreased by about 1.25-fold to about 10-fold or more relative to an untreated sample or a sample treated with a control nucleic acid. In instances, the level of SIRT1 mRNA or protein is increased or decreased by at least about 1.25-fold, at least about 1.3-fold, at least about 1.4-fold, at least about 1.5-fold, at least about 1.6-fold, at least about 1.7-fold, at least about 1.8-fold, at least about 2-fold, at least about 2.5-fold, at least about 3-fold, at least about 3.5-fold, at least about 4-fold, at least about 4.5-fold, at least about 5-fold, at least about 5.5-fold, at least about 6-fold, at least about 6.5-fold, at least about 7-fold, at least about 7.5-fold, at least about 8-fold, at least about 8.5-fold, at least about 9-fold, at least about 9.5-fold, or at least about 10-fold or more.

### Kits, Research Reagents, Diagnostics, and Therapeutics

The compounds of the present disclosure can be utilized for diagnostics, therapeutics, and prophylaxis, and as research reagents and components of kits. Furthermore, antisense oligonucleotides, which are able to inhibit gene expression with exquisite specificity, are often used by those of ordinary skill to elucidate the function of particular genes or to distinguish between functions of various members of a biological pathway.

For use in kits and diagnostics and in various biological systems, the compounds of the present disclosure, either alone or in combination with other compounds or therapeutics, are useful as tools in differential and/or combinatorial analyses to elucidate expression patterns of a portion or the entire complement of genes expressed within cells and tissues.

As used herein the term "biological system" or "system" is defined as any organism, cell, cell culture or tissue that expresses, or is made competent to express products of the Sirtuin 1 (SIRT1) genes. These include, but are not limited to, humans, transgenic animals, cells, cell cultures, tissues, xenografts, transplants and combinations thereof.

As one non limiting example, expression patterns within cells or tissues treated with one or more antisense compounds are compared to control cells or tissues not treated with antisense compounds and the patterns produced are analyzed for differential levels of gene expression as they pertain, for example, to disease association, signaling pathway, cellular localization, expression level, size, structure or function of the genes examined. These analyses can be performed on stimulated or unstimulated cells and in the presence or absence of other compounds that affect expression patterns.

Examples of methods of gene expression analysis known in the art include DNA arrays or microarrays (Brazma and Vilo, (2000) FEBS Lett., 480, 17-24; Celis, et al., (2000) FEBS Lett., 480, 2-16), SAGE (serial analysis of gene expression) (Madden, et al., (2000) Drug Discov. Today, 5, 415- 425), READS (restriction enzyme amplification of digested cDNAs) (Prashar and Weissman, (1999) Methods Enzymol., 303, 258-72), TOGA (total gene expression analysis) (Sutcliffe, et al., (2000) Proc. Natl. Acad. Sci. U.S.A., 97, 1976-81), protein arrays and proteomics (Celis, et al., (2000) FEBS Lett., 480, 2-16; Jungblut, et al., Electrophoresis, 1999, 20, 2100-10), expressed sequence tag (EST) sequencing (Celis, et al., FEBS Lett., 2000, 480, 2-16; Larsson, et al., J. Biotechnol., 2000, 80, 143-57), subtractive RNA fingerprinting (SuRF) (Fuchs, et al., (2000) Anal. Biochem. 286, 91-98; Larson, et al., (2000) Cytometry 41, 203-208), subtractive cloning, differential display (DD) (Jurecic and Belmont, (2000) Curr. Opin. Microbiol. 3, 316-21), comparative genomic hybridization (Carulli, et al., (1998) J. Cell Biochem. Suppl., 31, 286-96), FISH (fluorescent in situ hybridization) techniques (Going and Gusterson, (1999) Eur. J. Cancer, 35, 1895-904) and mass spectrometry methods (To, Comb. (2000) Chem. High Throughput Screen, 3, 235-41).

The compounds of the disclosure are useful for research and diagnostics, because these compounds hybridize to nucleic acids encoding Sirtuin 1 (SIRT1). For example, oligonucleotides that hybridize with such efficiency and under such conditions as disclosed herein as to be effective Sirtuin 1 (SIRT1) modulators are effective primers or probes under conditions favoring gene amplification or detection, respectively. These primers and probes are useful in methods requiring the specific detection of nucleic acid molecules encoding Sirtuin 1 (SIRT1) and in the amplification of said nucleic acid molecules for detection or for use in further studies of Sirtuin 1 (SIRT1). Hybridization of the antisense oligonucleotides, particularly the primers and probes, of the disclosure with a nucleic acid encoding Sirtuin 1 (SIRT1) can be detected by means known in the art. Such means may include conjugation of an enzyme to the oligonucleotide, radiolabeling of the oligonucleotide, or any other suitable detection means. Kits using such detection means for detecting the level of Sirtuin 1 (SIRT1) in a sample may also be prepared.

The specificity and sensitivity of antisense are also harnessed by those of skill in the art for therapeutic uses. Antisense compounds have been employed as therapeutic moieties in the treatment of disease states in animals, including humans. Antisense oligonucleotide drugs have been safely and effectively administered to humans and numerous clinical trials are presently underway. It is thus established that antisense compounds can be useful therapeutic modalities that can be configured to be useful in treatment regimes for the treatment of cells, tissues and animals, especially humans.

For therapeutics, an animal, preferably a human, suspected of having a disease or disorder which can be treated by modulating the expression of Sirtuin 1 (SIRT1) polynucleotides is treated by administering antisense compounds in accordance with this disclosure. For example, in one non-limiting instance, the methods comprise the step of administering to the animal in need of treatment, a therapeutically effective amount of Sirtuin 1 (SIRT1) modulator. The Sirtuin 1 (SIRT1) modulators of the present disclosure effectively modulate the activity of the Sirtuin 1 (SIRT1) or modulate the expression of the Sirtuin 1 (SIRT1) protein. In one instance, the activity or expression of Sirtuin 1 (SIRT1) in an animal is inhibited by about 10% as compared to a control. Preferably, the activity or expression of Sirtuin 1 (SIRT1) in an animal is inhibited by about 30%. More preferably, the activity or expression of Sirtuin 1 (SIRT1) in an animal is inhibited by 50% or more. Thus, the oligomeric compounds modulate expression of Sirtuin 1 (SIRT1) mRNA by at least 10%, by at least 50%, by at least 25%, by at least 30%, by at least 40%, by at least 50%, by at least 60%, by at least 70%, by at least 75%, by at least 80%, by at least 85%, by at least 90%, by at least 95%, by at least 98%, by at least 99%, or by 100% as compared to a control.

In one instance, the activity or expression of Sirtuin 1 (SIRT1) and/or in an animal is increased by about 10% as compared to a control. Preferably, the activity or expression of Sirtuin 1 (SIRT1) in an animal is increased by about 30%. More preferably, the activity or expression of Sirtuin 1 (SIRT1) in an animal is increased by 50% or more. Thus, the oligomeric compounds modulate expression of Sirtuin 1 (SIRT1) mRNA by at least 10%, by at least 50%, by at least 25%, by at least 30%, by at least 40%, by at least 50%, by at least 60%, by at least 70%, by at least 75%, by at least 80%, by at least 85%, by at least 90%, by at least 95%, by at least 98%, by at least 99%, or by 100% as compared to a control.

For example, the reduction of the expression of Sirtuin 1 (SIRT1) may be measured in serum, blood, adipose tissue, liver or any other body fluid, tissue or organ of the animal. Preferably, the cells contained within said fluids, tissues or organs being analyzed contain a nucleic acid molecule encoding Sirtuin 1 (SIRT1) peptides and/or the Sirtuin 1 (SIRT1) protein itself.

The compounds of the disclosure can be utilized in pharmaceutical compositions by adding an effective amount of a compound to a suitable pharmaceutically acceptable diluent or carrier. Use of the compounds and methods of the disclosure may also be useful prophylactically.

### Conjugates

Another modification of the oligonucleotides of the disclosure involves chemically linking to the oligonucleotide one or more moieties or conjugates that enhance the activity, cellular distribution or cellular uptake of the oligonucleotide. These moieties or conjugates can include conjugate groups covalently bound to functional groups such as primary or secondary hydroxyl groups. Conjugate groups of the disclosure include intercalators, reporter molecules, polyamines, polyamides, polyethylene glycols, polyethers, groups that enhance the pharmacodynamic properties of oligomers, and groups that enhance the pharmacokinetic properties of oligomers. Typicalconjugate groups include cholesterols, lipids, phospholipids, biotin, phenazine, folate, phenanthridine, anthraquinone, acridine, fluoresceins, rhodamines, coumarins, and dyes. Groups that enhance the pharmacodynamic properties, in the context of this disclosure, include groups that improve uptake, enhance resistance to degradation, and/or strengthen sequence-specific hybridization with the target nucleic acid. Groups that enhance the pharmacokinetic properties, in the context of this disclosure, include groups that improve uptake, distribution, metabolism or excretion of the compounds of the present disclosure. Representative conjugate groups are disclosed in International Patent Application No. PCT/US92/09196, filed Oct. 23, 1992, and U.S. Pat. No. 6,287,860.

Conjugate moieties include, but are not limited to, lipid moieties such as a cholesterol moiety, cholic acid, a thioether, e.g., hexyl-5- tritylthiol, a thiocholesterol, an aliphatic chain, e.g., dodecandiol or undecyl residues, a phospholipid, e.g., di-hexadecyl-rac-glycerol or triethylammonium 1,2-di-O-hexadecyl-rac-glycero-3-Hphosphonate, a polyamine or a polyethylene glycol chain, or adamantane acetic acid, a palmityl moiety, or an octadecylamine or hexylamino-carbonyl-oxycholesterol moiety. Oligonucleotides of the disclosure may also be conjugated to active drug substances, for example, aspirin, warfarin, phenylbutazone, ibuprofen, suprofen, fenbufen, ketoprofen, (S)-(+)-pranoprofen, carprofen, dansylsarcosine, 2,3,5-triiodobenzoic acid, flufenamic acid, folinic acid, a benzothiadiazide, chlorothiazide, a diazepine, indomethicin, a barbiturate, a cephalosporin, a sulfa drug, an antidiabetic, an antibacterial or an antibiotic.

Representative United States patents that teach the preparation of such oligonucleotides conjugates include, but are not limited to, U.S. Pat. Nos. 4,828,979; 4,948,882; 5,218,105; 5,525,465; 5,541,313; 5,545,730; 5,552,538; 5,578,717, 5,580,731; 5,580,731; 5,591,584; 5,109,124; 5,118,802; 5,138,045; 5,414,077; 5,486,603; 5,512,439; 5,578,718; 5,608,046; 4,587,044; 4,605,735; 4,667,025; 4,762,779; 4,789,737; 4,824,941; 4,835,263; 4,876,335; 4,904,582; 4,958,013; 5,082,830; 5,112,963; 5,214,136; 5,082,830; 5,112,963; 5,214,136; 5,245,022; 5,254,469; 5,258,506; 5,262,536; 5,272,250; 5,292,873; 5,317,098; 5,371,241, 5,391,723; 5,416,203, 5,451,463; 5,510,475; 5,512,667; 5,514,785; 5,565,552; 5,567,810; 5,574,142; 5,585,481; 5,587,371; 5,595,726; 5,597,696; 5,599,923; 5,599,928 and 5,688,941.

### Formulations

The compounds of the disclosure may also be admixed, encapsulated, conjugated or otherwise associated with other molecules, molecule structures or mixtures of compounds, as for example, liposomes, receptor-targeted molecules, oral, rectal, topical or other formulations, for assisting in uptake, distribution and/or absorption. Representative United States patents that teach the preparation of such uptake, distribution and/or absorption-assisting formulations include, but are not limited to, U.S. Pat. Nos. 5,108,921; 5,354,844; 5,416,016; 5,459,127; 5,521,291; 5,543,165; 5,547,932; 5,583,020; 5,591,721; 4,426,330; 4,534,899; 5,013,556; 5,108,921; 5,213,804; 5,227,170; 5,264,221; 5,356,633; 5,395,619; 5,416,016; 5,417,978; 5,462,854; 5,469,854; 5,512,295; 5,527,528; 5,534,259; 5,543,152; 5,556,948; 5,580,575; and 5,595,756.

Although, the antisense oligonucleotides do not need to be administered in the context of a vector in order to modulate a target expression and/or function, instances of the disclosure relates to expression vector constructs for the expression of antisense oligonucleotides, comprising promoters, hybrid promoter gene sequences and possess a strong constitutive promoter activity, or a promoter activity which can be induced in the desired case.

In an instance, disclosure practice involves administering at least one of the foregoing antisense oligonucleotides with a suitable nucleic acid delivery system. In one instance, that system includes a non-viral vector operably linked to the polynucleotide. Examples of such nonviral vectors include the oligonucleotide alone (e.g. any one or more of SEQ ID NOS: 9 to 66) or in combination with a suitable protein, polysaccharide or lipid formulation.

Additionally suitable nucleic acid delivery systems include viral vector, typically sequence from at least one of an adenovirus, adenovirus-associated virus (AAV), helper-dependent adenovirus, retrovirus, or hemagglutinatin virus of Japan-liposome (HVJ) complex. Preferably, the viral vector comprises a strong eukaryotic promoter operably linked to the polynucleotide e.g., a cytomegalovirus (CMV) promoter.

Additionally preferred vectors include viral vectors, fusion proteins and chemical conjugates. Retroviral vectors include Moloney murine leukemia viruses and HIV-based viruses. One preferred HIV-based viral vector comprises at least two vectors wherein the gag and pol genes are from an HIV genome and the env gene is from another virus. DNA viral vectors are preferred. These vectors include pox vectors such as orthopox or avipox vectors, herpesvirus vectors such as a herpes simplex I virus (HSV) vector [Geller, A.I. et al., (1995) J. Neurochem, 64: 487; Lim, F., et al., in DNA Cloning: Mammalian Systems, D. Glover, Ed. (Oxford Univ. Press, Oxford England) (1995); Geller, A.I. et al., (1993) Proc Natl. Acad. Sci.: U.S.A.:90 7603; Geller, A.I., et al., (1990) Proc Natl. Acad. Sci USA: 87:1149], Adenovirus Vectors (LeGal LaSalle et al., Science, 259:988 (1993); Davidson, et al., (1993) Nat. Genet. 3: 219; Yang, et al., (1995) J. Virol. 69: 2004) and Adeno-associated Virus Vectors (Kaplitt, M.G., et al., (1994) Nat. Genet. 8:148).

The antisense compounds of the disclosure encompass any pharmaceutically acceptable salts, esters, or salts of such esters, or any other compound which, upon administration to an animal, including a human, is capable of providing (directly or indirectly) the biologically active metabolite or residue thereof.

The term "pharmaceutically acceptable salts" refers to physiologically and pharmaceutically acceptable salts of the compounds of the disclosure: i.e., salts that retain the desired biological activity of the parent compound and do not impart undesired toxicological effects thereto. For oligonucleotides, preferred examples of pharmaceutically acceptable salts and their uses are further described in U.S. Pat. No. 6,287,860 .

The present disclosure also includes pharmaceutical compositions and formulations that include the antisense compounds of the disclosure. The pharmaceutical compositions of the present disclosure may be administered in a number of ways depending upon whether local or systemic treatment is desired and upon the area to be treated. Administration may be topical (including ophthalmic and to mucous membranes including vaginal and rectal delivery), pulmonary, e.g., by inhalation or insufflation of powders or aerosols, including by nebulizer; intratracheal, intranasal, epidermal and transdermal), oral or parenteral. Parenteral administration includes intravenous, intraarterial, subcutaneous, intraperitoneal or intramuscular injection or infusion; or intracranial, e.g., intrathecal or intraventricular, administration.

For treating tissues in the central nervous system, administration can be made by, e.g., injection or infusion into the cerebrospinal fluid. Administration of antisense RNA into cerebrospinal fluid is described, e.g., in U.S. Pat. App. Pub. No. 2007/0117772, "Methods for slowing familial ALS disease progression," .

When it is intended that the antisense oligonucleotide of the present disclosure be administered to cells in the central nervous system, administration can be with one or more agents capable of promoting penetration of the subject antisense oligonucleotide across the blood-brain barrier. Injection can be made, e.g., in the entorhinal cortex or hippocampus. Delivery of neurotrophic factors by administration of an adenovirus vector to motor neurons in muscle tissue is described in, e.g., U.S. Pat. No. 6,632,427, "Adenoviral-vector-mediated gene transfer into medullary motor neurons," . Delivery of vectors directly to the brain, e.g., the striatum, the thalamus, the hippocampus, or the substantia nigra, is known in the art and described, e.g., in U.S. Pat. No. 6,756,523, "Adenovirus vectors for the transfer of foreign genes into cells of the central nervous system particularly in brain," . Administration can be rapid as by injection or made over a period of time as by slow infusion or administration of slow release formulations.

The subject antisense oligonucleotides can also be linked or conjugated with agents that provide desirable pharmaceutical or pharmacodynamic properties. For example, the antisense oligonucleotide can be coupled to any substance, known in the art to promote penetration or transport across the blood-brain barrier, such as an antibody to the transferrin receptor, and administered by intravenous injection. The antisense compound can be linked with a viral vector, for example, that makes the antisense compound more effective and/or increases the transport of the antisense compound across the blood-brain barrier. Osmotic blood brain barrier disruption can also be accomplished by, e.g., infusion of sugars including, but not limited to, meso erythritol, xylitol, D(+) galactose, D(+) lactose, D(+) xylose, dulcitol, myo-inositol, L(-) fructose, D(-) mannitol, D(+) glucose, D(+) arabinose, D(-) arabinose, cellobiose, D(+) maltose, D(+) raffinose, L(+) rhamnose, D(+) melibiose, D(-) ribose, adonitol, D(+) arabitol, L(-) arabitol, D(+) fucose, L(-) fucose, D(-) lyxose, L(+) lyxose, and L(-) lyxose, or amino acids including, but not limited to, glutamine, lysine, arginine, asparagine, aspartic acid, cysteine, glutamic acid, glycine, histidine, leucine, methionine, phenylalanine, proline, serine, threonine, tyrosine, valine, and taurine. Methods and materials for enhancing blood brain barrier penetration are described, e.g., in U. S. Patent No. 4,866,042, "Method for the delivery of genetic material across the blood brain barrier," 6,294,520, "Material for passage through the blood-brain barrier," and 6,936,589, "Parenteral delivery systems," .

The subject antisense compounds may be admixed, encapsulated, conjugated or otherwise associated with other molecules, molecule structures or mixtures of compounds, for example, liposomes, receptor-targeted molecules, oral, rectal, topical or other formulations, for assisting in uptake, distribution and/or absorption. For example, cationic lipids may be included in the formulation to facilitate oligonucleotide uptake. One such composition shown to facilitate uptake is LIPOFECTIN (available from GIBCO-BRL, Bethesda, MD).

Oligonucleotides with at least one 2'-O-methoxyethyl modification are believed to be particularly useful for oral administration. Pharmaceutical compositions and formulations for topical administration may include transdermal patches, ointments, lotions, creams, gels, drops, suppositories, sprays, liquids and powders. Conventional pharmaceutical carriers, aqueous, powder or oily bases, thickeners and the like may be necessary or desirable. Coated condoms, gloves and the like may also be useful.

The pharmaceutical formulations of the present disclosure, which may conveniently be presented in unit dosage form, may be prepared according to conventional techniques well known in the pharmaceutical industry. Such techniques include the step of bringing into association the active ingredients with the pharmaceutical carrier(s) or excipient(s). In general, the formulations are prepared by uniformly and intimately bringing into association the active ingredients with liquid carriers or finely divided solid carriers or both, and then, if necessary, shaping the product.

The compositions of the present disclosure may be formulated into any of many possible dosage forms such as, but not limited to, tablets, capsules, gel capsules, liquid syrups, soft gels, suppositories, and enemas. The compositions of the present disclosure may also be formulated as suspensions in aqueous, non-aqueous or mixed media. Aqueous suspensions may further contain substances that increase the viscosity of the suspension including, for example, sodium carboxymethylcellulose, sorbitol and/or dextran. The suspension may also contain stabilizers.

Pharmaceutical compositions of the present disclosure include, but are not limited to, solutions, emulsions, foams and liposome-containing formulations. The pharmaceutical compositions and formulations of the present disclosure may comprise one or more penetration enhancers, carriers, excipients or other active or inactive ingredients.

Emulsions are typically heterogeneous systems of one liquid dispersed in another in the form of droplets usually exceeding 0.1 µm in diameter. Emulsions may contain additional components in addition to the dispersed phases, and the active drug that may be present as a solution in either the aqueous phase, oily phase or itself as a separate phase. Microemulsions are included as an instance of the present disclosure. Emulsions and their uses are well known in the art and are further described in U.S. Pat. No. 6,287,860.

Formulations of the present disclosure include liposomal formulations. As used in the present disclosure, the term "liposome" means a vesicle composed of amphiphilic lipids arranged in a spherical bilayer or bilayers. Liposomes are unilamellar or multilamellar vesicles which have a membrane formed from a lipophilic material and an aqueous interior that contains the composition to be delivered. Cationic liposomes are positively charged liposomes that are believed to interact with negatively charged DNA molecules to form a stable complex. Liposomes that are pH-sensitive or negatively-charged are believed to entrap DNA rather than complex with it. Both cationic and noncationic liposomes have been used to deliver DNA to cells.

Liposomes also include "sterically stabilized" liposomes, a term which, as used herein, refers to liposomes comprising one or more specialized lipids. When incorporated into liposomes, these specialized lipids result in liposomes with enhanced circulation lifetimes relative to liposomeslacking such specialized lipids. Examples of sterically stabilized liposomes are those in which part of the vesicle-forming lipid portion of the liposome comprises one or more glycolipids or is derivatized with one or more hydrophilic polymers, such as a polyethylene glycol (PEG) moiety. Liposomes and their uses are further described in U.S. Pat. No. 6,287,860.

The pharmaceutical formulations and compositions of the present disclosure may also include surfactants. The use of surfactants in drug products, formulations and in emulsions is well known in the art. Surfactants and their uses are further described in U.S. Pat. No. 6,287,860.

In one instance, the present disclosure employs various penetration enhancers to effect the efficient delivery of nucleic acids, particularly oligonucleotides. In addition to aiding the diffusion of non-lipophilic drugs across cell membranes, penetration enhancers also enhance the permeability of lipophilic drugs. Penetration enhancers may be classified as belonging to one of five broad categories, i.e., surfactants, fatty acids, bile salts, chelating agents, and non-chelating nonsurfactants. Penetration enhancers and their uses are further described in U.S. Pat. No. 6,287,860.

One of skill in the art will recognize that formulations are routinely designed according to their intended use, i.e. route of administration.

Preferred formulations for topical administration include those in which the oligonucleotides of the disclosure are in admixture with a topical delivery agent such as lipids, liposomes, fatty acids, fatty acid esters, steroids, chelating agents and surfactants. Preferred lipids and liposomes include neutral (e.g. dioleoyl-phosphatidyl DOPE ethanolamine, dimyristoylphosphatidyl choline DMPC, distearolyphosphatidyl choline) negative (e.g. dimyristoylphosphatidyl glycerol DMPG) and cationic (e.g. dioleoyltetramethylaminopropyl DOTAP and dioleoyl-phosphatidyl ethanolamine DOTMA).

For topical or other administration, oligonucleotides of the disclosure may be encapsulated within liposomes or may form complexes thereto, in particular to cationic liposomes. Alternatively, oligonucleotides may be complexed to lipids, in particular to cationic lipids. Preferred fatty acids and esters, pharmaceutically acceptable salts thereof, and their uses are further described in U.S. Pat. No. 6,287,860.

Compositions and formulations for oral administration include powders or granules, microparticulates, nanoparticulates, suspensions or solutions in water or non-aqueous media, capsules, gel capsules, sachets, tablets or minitablets. Thickeners, flavoring agents, diluents, emulsifiers, dispersing aids or binders may be desirable. Preferred oral formulations are those in which oligonucleotides of the disclosure are administered in conjunction with one or more penetration enhancers surfactants and chelators. Preferred surfactants include fatty acids and/or esters or salts thereof, bile acids and/or salts thereof. Preferred bile acids/salts and fatty acids and their uses are further described in U.S. Pat. No. 6,287,860 .

Also preferred are combinations of penetration enhancers, for example, fatty acids/salts in combination with bile acids/salts. A particularly preferred combination is the sodium salt of lauric acid, capric acid and UDCA. Further penetration enhancers include polyoxyethylene-9-lauryl ether, polyoxyethylene-20-cetyl ether. Oligonucleotides of the disclosure may be delivered orally, in granular form including sprayed dried particles, or complexed to form micro or nanoparticles. Oligonucleotide complexing agents and their uses are further described in U.S. Pat. No. 6,287,860.

Compositions and formulations for parenteral, intrathecal or intraventricular administration may include sterile aqueous solutions that may also contain buffers, diluents and other suitable additives such as, but not limited to, penetration enhancers, carrier compounds and other pharmaceutically acceptable carriers or excipients.

Certain instances of the disclosure provide pharmaceutical compositions containing one or more oligomeric compounds and one or more other chemotherapeutic agents that function by a non-antisense mechanism. Examples of such chemotherapeutic agents include but are not limited to cancer chemotherapeutic drugs such as daunorubicin, daunomycin, dactinomycin, doxorubicin, epirubicin, idarubicin, esorubicin, bleomycin, mafosfamide, ifosfamide, cytosine arabinoside, bischloroethyl- nitrosurea, busulfan, mitomycin C, actinomycin D, mithramycin, prednisone, hydroxyprogesterone, testosterone, tamoxifen, dacarbazine, procarbazine, hexamethylmelamine, pentamethylmelamine, mitoxantrone, amsacrine, chlorambucil, methylcyclohexylnitrosurea, nitrogen mustards, melphalan, cyclophosphamide, 6-mercaptopurine, 6-thioguanine, cytarabine, 5- azacytidine, hydroxyurea, deoxycoformycin, 4-hydroxyperoxycyclo-phosphoramide, 5-fluorouracil (5-FU), 5-fluorodeoxyuridine (5-FUdR), methotrexate (MTX), colchicine, taxol, vincristine, vinblastine, etoposide (VP-16), trimetrexate, irinotecan, topotecan, gemcitabine, teniposide, cisplatin and diethylstilbestrol (DES). When used with the compounds of the disclosure, such chemotherapeutic agents may be used individually (e.g., 5-FU and oligonucleotide), sequentially (e.g., 5-FU and oligonucleotide for a period of time followed by MTX and oligonucleotide), or in combination with one or more other such chemotherapeutic agents (e.g., 5-FU, MTX and oligonucleotide, or 5-FU, radiotherapy and oligonucleotide). Anti-inflammatory drugs, including but not limited to nonsteroidal anti-inflammatory drugs and corticosteroids, and antiviral drugs, including but not limited to ribivirin, vidarabine, acyclovir and ganciclovir, may also be combined in compositions of the disclosure. Combinations of antisense compounds and other non-antisense drugs are also within the scope of this disclosure. Two or more combined compounds may be used together or sequentially.

In another related instance, compositions of the disclosure may contain one or more antisense compounds, particularly oligonucleotides, targeted to a first nucleic acid and one or more additional antisense compounds targeted to a second nucleic acid target. For example, the first target may be a particular antisense sequence of Sirtuin 1 (SIRT1), and the second target may be a region from another nucleotide sequence. Alternatively, compositions of the disclosure may contain two or more antisense compounds targeted to different regions of the same Sirtuin 1 (SIRT1) nucleic acid target. Numerous examples of antisense compounds are illustrated herein and others may be selected from among suitable compounds known in the art. Two or more combined compounds may be used together or sequentially.

### Dosing:

The formulation of therapeutic compositions and their subsequent administration (dosing) is believed to be within the skill of those in the art. Dosing is dependent on severity and responsiveness of the disease state to be treated, with the course of treatment lasting from several days to several months, or until a cure is effected or a diminution of the disease state is achieved. Optimal dosing schedules can be calculated from measurements of drug accumulation in the body of the patient. Persons of ordinary skill can easily determine optimum dosages, dosing methodologies and repetition rates. Optimum dosages may vary depending on the relative potency of individual oligonucleotides, and can generally be estimated based on EC50s found to be effective in in vitro and in vivo animal models. In general, dosage is from 0.01 µg to 100 g per kg of body weight, and may be given once or more daily, weekly, monthly or yearly, or even once every 2 to 20 years. Persons of ordinary skill in the art can easily estimate repetition rates for dosing based on measured residence times and concentrations of the drug in bodily fluids or tissues. Following successful treatment, it may be desirable to have the patient undergo maintenance therapy to prevent the recurrence of the disease state, wherein the oligonucleotide is administered in maintenance doses, ranging from 0.01 µg to 100 g per kg of body weight, once or more daily, to once every 20 years.

In instances, a patient is treated with a dosage of drug that is at least about 1, at least about 2, at least about 3, at least about 4, at least about 5, at least about 6, at least about 7, at least about 8, at least about 9, at least about 10, at least about 15, at least about 20, at least about 25, at least about 30, at least about 35, at least about 40, at least about 45, at least about 50, at least about 60, at least about 70, at least about 80, at least about 90, or at least about 100 mg/kg body weight. Certain injected dosages of antisense oligonucleotides are described, e.g., in U.S. Pat. No. 7,563,884, "Antisense modulation of PTP1B expression,".

By their citation of various references in this document, Applicants do not admit any particular reference is "prior art" to their invention. Embodiments of inventive compositions and methods are illustrated in the following examples.

### EXAMPLES

The following non-limiting Examples serve to illustrate selected embodiments of the invention. It will be appreciated that variations in proportions and alternatives in elements of the components shown will be apparent to those skilled in the art and are within the scope of instances of the present disclosure.

### Example 1: Design of antisense oligonucleotides specific for a nucleic acid molecule antisense to a Sirtuin 1 (S1RT1) and/or a sense strand of Sirtuin 1 (SIRT1) polynucleotide

As indicated above the term "oligonucleotide specific for" or "oligonucleotide targets" refers to an oligonucleotide having a sequence (i) capable of forming a stable complex with a portion of the targeted gene, or (ii) capable of forming a stable duplex with a portion of an mRNA transcript of the targeted gene.

Selection of appropriate oligonucleotides is facilitated by using computer programs that automatically align nucleic acid sequences and indicate regions of identity or homology. Such programs are used to compare nucleic acid sequences obtained, for example, by searching databases such as GenBank or by sequencing PCR products. Comparison of nucleic acid sequences from a range of species allows the selection of nucleic acid sequences that display an appropriate degree of identity between species. In the case of genes that have not been sequenced, Southern blots are performed to allow a determination of the degree of identity between genes in target species and other species. By performing Southern blots at varying degrees of stringency, as is well known in the art, it is possible to obtain an approximate measure of identity. These procedures allow the selection of oligonucleotides that exhibit a high degree of complementarity to target nucleic acid sequences in a subject to be controlled and a lower degree of complementarity to corresponding nucleic acid sequences in other species. One skilled in the art will realize that there is considerable latitude in selecting appropriate regions of genes for use in the present disclosure.

An antisense compound is "specifically hybridizable" when binding of the compound to the target nucleic acid interferes with the normal function of the target nucleic acid to cause a modulation of function and/or activity, and there is a sufficient degree of complementarity to avoid non-specific binding of the antisense compound to non-target nucleic acid sequences under conditions in which specific binding is desired, i.e., under physiological conditions in the case of in vivo assays or therapeutic treatment, and under conditions in which assays are performed in the case of in vitro assays

The hybridization properties of the oligonucleotides described herein can be determined by one or more in vitro assays as known in the art. For example, the properties of the oligonucleotides described herein can be obtained by determination of binding strength between the target natural antisense and a potential drug molecules using melting curve assay.

The binding strength between the target natural antisense and a potential drug molecule (Molecule) can be estimated using any of the established methods of measuring the strength of intermolecular interactions, for example, a melting curve assay.

Melting curve assay determines the temperature at which a rapid transition from double-stranded to single-stranded conformation occurs for the natural antisense/Molecule complex. This temperature is widely accepted as a reliable measure of the interaction strength between the two molecules.

A melting curve assay can be performed using a cDNA copy of the actual natural antisense RNA molecule or a synthetic DNA or RNA nucleotide corresponding to the binding site of the Molecule. Multiple kits containing all necessary reagents to perform this assay are available (e.g. Applied Biosystems Inc. MeltDoctor kit). These kits include a suitable buffer solution containing one of the double strand DNA (dsDNA) binding dyes (such as ABI HRM dyes, SYBR Green, SYTO, etc.). The properties of the dsDNA dyes are such that they emit almost no fluorescence in free form, but are highly fluorescent when bound to dsDNA.

To perform the assay the cDNA or a corresponding oligonucleotide are mixed with Molecule in concentrations defined by the particular manufacturer's protocols. The mixture is heated to 95 °C to dissociate all pre-formed dsDNA complexes, then slowly cooled to room temperature or other lower temperature defined by the kit manufacturer to allow the DNA molecules to anneal. The newly formed complexes are then slowly heated to 95 °C with simultaneous continuous collection of data on the amount of fluorescence that is produced by the reaction. The fluorescence intensity is inversely proportional to the amounts of dsDNA present in the reaction. The data can be collected using a real time PCR instrument compatible with the kit (e.g.ABI's StepOne Plus Real Time PCR System or LightTyper instrument, Roche Diagnostics, Lewes, UK).

Melting peaks are constructed by plotting the negative derivative of fluorescence with respect to temperature (-d(Fluorescence)/dT) on the y-axis) against temperature (x-axis) using appropriate software (for example LightTyper (Roche) or SDS Dissociation Curve, ABI). The data is analyzed to identify the temperature of the rapid transition from dsDNA complex to single strand molecules. This temperature is called Tm and is directly proportional to the strength of interaction between the two molecules. Typically, Tm will exceed 40 °C.

### Example 2: Modulation of SIRT1 polynucleotides

### Treatment of HepG2 cells with antisense oligonucleotides

HepG2 cells from ATCC (cat# HB-8065) were grown in growth media (MEM/EBSS (Hyclone cat #SH30024, or Mediatech cat # MT-10-010-CV) +10% FBS (Mediatech cat# MT35- 011-CV)+ penicillin/streptomycin (Mediatech cat# MT30-002-CI)) at 37°C and 5% CO₂. One day before the experiment the cells were replated at the density of 1.5 × 10⁵/ml into 6 well plates and incubated at 37°C and 5% CO₂. On the day of the experiment the media in the 6 well plates was changed to fresh growth media. All antisense oligonucleotides were diluted to the concentration of 20 µM. Two µl of this solution was incubated with 400 µl of Opti-MEM media (Gibco cat#31985-070) and 4 µl of Lipofectamine 2000 (Invitrogen cat# 11668019) at room temperature for 20 min and applied to each well of the 6 well plates with HepG2 cells. A Similar mixture including 2 µl of water instead of the oligonucleotide solution was used for the mock-transfected controls. After 3-18 h of incubation at 37°C and 5% CO₂ the media was changed to fresh growth media. 48 h after addition of antisense oligonucleotides the media was removed and RNA was extracted from the cells using SV Total RNA Isolation System from Promega (cat # Z3105) or RNeasy Total RNA Isolation kit from Qiagen (cat# 74181) following the manufacturers' instructions. 600 ng of RNA was added to the reverse transcription reaction performed using Verso cDNA kit from Thermo Scientific (cat#AB1453B) or High Capacity cDNA Reverse Transcription Kit (cat# 4368813) as described in the manufacturer's protocol. The cDNA from this reverse transcription reaction was used to monitor gene expression by real time PCR using ABI Taqman Gene Expression Mix (cat#4369510) and primers/probes designed by ABI (Applied Biosystems Taqman Gene Expression Assay: Hs00202021_m1 by Applied Biosystems Inc., Foster City CA). The following PCR cycle was used: 50°C for 2 min, 95°C for 10 min, 40 cycles of (95°C for 15 seconds, 60°C for 1 min) using StepOne Plus Real Time PCR Machine (Applied Biosystems).

Fold change in gene expression after treatment with antisense oligonucleotides was calculated based on the difference in 18S-normalized dCt values between treated and mock-transfected samples.

### Results:

Real time PCR results show that the levels of the SIRT1 mRNA in HepG2 cells significantly increased 48 h after treatment with some antisense oligonucleotides to SIRT1 antisense CV396200 (Figure 2, 3A).

Real Time PCR results show that levels of SIRT1 mRNA in HepG2 cells are significantly increased in one of the oligonucleotides designed to SIRT1 antisense CV396200 (Fig 6).

Real Time PCR results show that levels of SIRT1 mRNA in HepG2 cells are significantly increased in two of the oligonucleotides designed to SIRT1 antisense CV428275 (Fig 7).

The results show that a significant increase in SIRT1 mRNA levels in HepG2 cells 48 hours after treatment with one of the oligonucleotides designed to SIRT antisense BE717453. (Fig 8).

The results show that show that the levels of the SIRT1 mRNA in HepG2 cells are significantly increased 48 h after treatment with three of the oligonucleotides designed to SIRT1 antisense AV718812 respectively (Fig 9).

Real time PCR results show that the levels of SIRT1 mRNA in HepG2 cells are significantly increased 48 h after treatment with two of the oligos designed to SIRT1 antisense AW169958 (Fig 10).

### Treatment of 3T3 cells with antisense oligonucleotides

3T3 cells from ATCC (cat# CRL-1658) were grown in growth media (MEM/EBSS (Hyclone cat #SH30024, or Mediatech cat # MT-10-010-CV) +10% FBS (Mediatech cat# MT35- 011-CV)+ penicillin/streptomycin (Mediatech cat# MT30-002-CI)) at 37°C and 5% CO₂. One day before the experiment the cells were replated at the density of 1.5 × 10⁵/ml into 6 well plates and incubated at 37°C and 5% CO₂. On the day of the experiment the media in the 6 well plates was changed to fresh growth media. All antisense oligonucleotides were diluted to the concentration of 20 µM. Two µl of this solution was incubated with 400 µl of Opti-MEM media (Gibco cat#31985-070) and 4 µl of Lipofectamine 2000 (Invitrogen cat# 11668019) at room temperature for 20 min and applied to each well of the 6 well plates with 3T3 cells. A Similar mixture including 2 µl of water instead of the oligonucleotide solution was used for the mock-transfected controls. After 3-18 h of incubation at 37°C and 5% CO₂ the media was changed to fresh growth media. 48 h after addition of antisense oligonucleotides the media was removed and RNA was extracted from the cells using SV Total RNA Isolation System from Promega (cat # Z3105) or RNeasy Total RNA Isolation kit from Qiagen (cat# 74181) following the manufacturers' instructions. 600 ng of RNA was added to the reverse transcription reaction performed using Verso cDNA kit from Thermo Scientific (cat#AB1453B) or High Capacity cDNA Reverse Transcription Kit (cat# 4368813) as described in the manufacturer's protocol. The cDNA from this reverse transcription reaction was used to monitor gene expression by real time PCR using ABI Taqman Gene Expression Mix (cat#4369510) and primers/probes designed by ABI (Applied Biosystems Taqman Gene Expression Assay: Hs00202021_m1 by Applied Biosystems Inc., Foster City CA). The following PCR cycle was used: 50°C for 2 min, 95°C for 10 min, 40 cycles of (95°C for 15 seconds, 60°C for 1 min) using StepOne Plus Real Time PCR Machine (Applied Biosystems).

Fold change in gene expression after treatment with antisense oligonucleotides was calculated based on the difference in 18S-normalized dCt values between treated and mock-transfected samples.

### Results:

Real time PCR results show that the levels of SIRT1 mRNA are significantly increased in 3T3 cells 48 h after treatment with three of the oligonucleotides designed to SIRT1 mouse antisense AK044604 (Fig 11).

Real time PCR results show that the levels of SIRT1 mRNA are significantly increased in 3T3 cells 48 h after treatment with five of the oligonucleotides designed to SIRT1 mouse antisense AK044604 (Fig 12).

Real time PCR results show that the levels of SIRT1 mRNA are significantly increased in 3T3 cells 48 h after treatment with two of the oligonucleotides designed to SIRT1 mouse antisense AK044604 (Fig 13).

Real time PCR results show that the levels of SIRT1 mRNA are significantly increased in 3T3 cells 48 h after treatment with two of the oligonucleotides designed to SIRT1 mouse antisense AK044604 (Fig 14).

### Treatment of Vero 76 cells with antisense oligonucleotides:

Vero76 cells from ATCC (cat# CRL-1587) were grown in growth media (MEM/EBSS (Hyclone cat #SH30024, or Mediatech cat # MT-10-010-CV) +10% FBS (Mediatech cat# MT35- 011-CV)+ penicillin/streptomycin (Mediatech cat# MT30-002-CI)) at 37°C and 5% CO₂. One day before the experiment the cells were replated at the density of 1.5 × 10⁵/ml into 6 well plates and incubated at 37°C and 5% CO₂. On the day of the experiment the media in the 6 well plates was changed to fresh growth media. All antisense oligonucleotides were diluted in water to the concentration of 20 µM. 2 µl of this solution was incubated with 400 µl of Opti-MEM media (Gibco cat#31985-070) and 4 ul of Lipofectamine 2000 (Invitrogen cat# 11668019) at room temperature for 20 min and applied to each well of the 6 well plates with Vero76 cells. Similar mixture including 2 µl of water instead of the oligonucleotide solution was used for the mock- transfected controls. After 3-18 h of incubation at 37°C and 5% CO₂ the media was changed to fresh growth media. 48 h after addition of antisense oligonucleotides the media was removed and RNA was extracted from the cells using SV Total RNA Isolation System from Promcga (cat # Z3105) or RNcasy Total RNA Isolation kit from Qiagen (cat# 74181), following the manufacturers' instructions. 600 ng of RNA was added to the reverse transcription reaction performed using Verso cDNA kit from Thermo Scientific (cat#AB1453B) as described in the manufacturer's protocol. The cDNA from this reverse transcription reaction was used to monitor gene expression by real time PCR using ABI Taqman Gene Expression Mix (cat#4369510) and primers/probes designed by ABI (Applied Biosystems Taqman Gene Expression Assay: Hs00202021_m1 by Applied Biosystems Inc., Foster City CA). The following PCR cycle was used: 50°C for 2 min, 95°C for 10 min, 40 cycles of (95°C for 15 seconds, 60°C for 1 min) using StepOne Plus Real Time PCR Machine (Applied Biosystems). Fold change in gene expression after treatment with antisense oligonucleotides was calculated based on the difference in 18S-normalized dCt values between treated and mock-transfected samples.

### Results:

Real time PCR results show that the levels of the SIRT1 mRNA in Vero cells significantly increased 48 h after treatment with antisense oligonucleotides to SIRT1 antisense CV396200 (Figure 3B).

### Example 3: Modulation of SIRT1 Gene Expression

### Materials and Methods

### Treatment of HepG2 cells with naked antisense oligonucleotides:

HepG2 cells from ATCC (cat# HB-8065) were grown in growth media (MEM/EBSS (Hyclone cat #SH30024, or Mediatech cat # MT-10-010-CV) +10% FBS (Mediatech cat# MT35- 011-CV) + penicillin/streptomycin (Mediatech cat# MT30-002-CI)) at 37°C and 5% CO₂. One day before the experiment the cells were replated at the density of 0.5 × 10⁵/ml into 6 well plates and incubated at 37°C and 5% CO₂. On the day of the experiment the media in the 6 well plates was replaced with 1.5 ml/well of fresh growth media. All antisense oligonucleotides were diluted in water to the concentration of 20 µM. 2 µl of this solution was incubated with 400 µl of Opti-MEM media (Gibco cat#31985-070) and 4 ul of Lipofectamine 2000 (Invitrogen cat# 11668019) at room temperature for 20 min and applied to each well of the 6 well plates with HepG2 cells. Similar mixture including 2 µl of water instead of the oligonucleotide solution was used for the mock- transfected controls. After 3-18 h of incubation at 37°C and 5% CO₂ the media was changed to fresh growth media. 72 h after addition of antisense oligonucleotides the cells were redosed as described in above. 48h after the second dosing of antisense oligonucleotides the media was removed and RNA was extracted from the cells using SV Total RNA Isolation System from Promega (cat # Z3105) or RNeasy Total RNA Isolation kit from Qiagen (cat# 74181) following the manufacturers' instructions. 600 ng of RNA was added to the reverse transcription reaction performed using Verso cDNA kit from Thermo Scientific (cat#AB1453B) as described in the manufacturer's protocol. The cDNA from this reverse transcription reaction was used to monitor gene expression by real time PCR using ABI Taqman Gene Expression Mix (cat#4369510) and primers/probes designed by ABI (Applied Biosystems Taqman Gene Expression Assay: Hs00202021_ml by Applied Biosystems Inc., Foster City CA). The following PCR cycle was used: 50°C for 2 min, 95°C for 10 min, 40 cycles of (95°C for 15 seconds, 60°C for 1 min) using StepOne Plus Real Time PCR Machine (Applied Biosystems). Fold change in gene expression after treatment with antisense oligonucleotides was calculated based on the difference in 18S-normalized dCt values between treated and mock-transfected samples.

Primers and probe for the custom designed Taqman assay for exon 4: AACTGGAGCTGGGGTGTCTGTTTCA (SEQ ID NO: 67) the SIRT1 natural antisense CV396200.
Forward Primer Seq. CCATCAGACGACATCCCTTAACAAA (SEQ ID NO: 68)
Reverse Primer Seq. ACATTATATCATAGCTCCTAAAGGAGATGCA (SEQ ID NO: 69)
Reporter Seq. CAGAGTTTCAATTCCC (SEQ ID NO: 70)

### Results:

The results show that the levels of the SIRT1 mRNA in HepG2 cells are significantly increased 48 h after treatment with one of the siRNAs designed to sirtas (sirtas_5, P=0.01). In the same samples the levels of sirtas RNA were significantly decreased after treatment with sirtas_5, but unchanged after treatment with sirtas_6 and sirtas_7, which also had no effect on the SIRT1 mRNA levels (Fig. 1B). sirtas_5, sirtas_6 and sirtas_7 correspond to SEQ ID NO: 32, 33 and 34 respectively.

### Treatment of primary monkey hepatocytes

Primary monkey hepatocytes were introduced into culture by RxGen Inc. and plated in 6 well plates. They were treated with oligonucleotides as follows. The media in the 6 well plates was changed to fresh growth media consisting of William's Medium E (Sigma cat#W4128) supplemented with 5% FBS, 50 U/ml penicillin and 50 ug/ml streptomycin, 4 ug/ml insulin, 1 uM dexamethasone, 10 ug/ml Fungin (InVivogen, San Diego CA). All antisense oligonucleotides were diluted to the concentration of 20 µM. 2 µl of this solution was incubated with 400 µl of Opti-MEM media (Gibco cat#31985-070) and 4 µl of Lipofectamine 2000 (Invitrogen cat# 11668019) at room temperature for 20 min and applied to each well of the 6 well plates with cells. Similar mixture including 2 µl of water instead of the oligonucleotide solution was used for the mock-transfected controls. After 3-18 h of incubation at 37°C and 5% CO₂ the media was changed to fresh growth media. 48 h after addition of antisense oligonucleotides the media was removed and RNA was extracted from the cells using SV Total RNA Isolation System from Promega (cat # Z3105) or RNeasy Total RNA Isolation kit from Qiagen (cat# 74181) following the manufacturers' instructions. 600 ng of RNA was added to the reverse transcription reaction performed using Verso cDNA kit from Thermo Scientific (cat#AB1453B) as described in the manufacturer's protocol. The cDNA from this reverse transcription reaction was used to monitor gene expression by real time PCR using ABI Taqman Gene Expression Mix (cat#4369510) and primers/probes designed by ABI (Applied Biosystems Taqman Gene Expression Assay: Hs00978340_m1 by Applied Biosystems Inc., Foster City CA). The following PCR cycle was used: 50°C for 2 min, 95°C for 10 min, 40 cycles of (95°C for 15 seconds, 60°C for 1 min) using Mx4000 thermal cycler (Stratagene). Fold change in gene expression after treatment with antisense oligonucleotides was calculated based on the difference in 18S-normalized dCt values between treated and mock-transfected samples.

### Results:

The results are shown in Figure 5. Real time PCR results show an increase in SIRT1 mRNA levels after treatment with an oligonucleotide against SIRT1 antisense.

### Example 4: Efficacy and Duration of Action Study of CUR 963 in the African Green Monkey

The objective of this study was to assess and compare the effect of antisense knockdown of the discordant noncoding antisense sequences that regulate the SIRT1 genes following intravenous administration in a nonhuman primate model. The antisense oligonucleotide test articles designed to inhibit the SIRT1 regulatory sequences were designated as CUR 963.
CUR 963: +G*+T*C*T*G*A*T*G*G*+A*+G*+A (SEQ ID NO: 28).
CUR 962 (control): +G*+C*T*A*G*T*C*T*G*+T*+T*+G (SEQ ID NO: 71).

### REGULATORY TEST GUIDELINES

This study was designed in accordance with accepted toxicological principles and to comply with International Conference of Harmonization (ICH) Harmonized Tripartite Guidelines (Non-Clinical Safety Studies for the Conduct of Human Clinical Trials for Pharmaceuticals ICH M3(m), 2000 November 9), and generally accepted procedures for the testing of therapeutic agents.

### TEST AND CONTROL ARTICLES

### Test Article Identity and Preparation

The test article, CUR-963, is a chemically stabilized antisense oligonucleotide. The vehicle for intravenous delivery is phosphate-buffered saline (PBS).

### Vehicle characterization

For the PBS vehicle, the composition, batch number, expiry date and storage conditions (temperature and light/dark) was obtained from the supplier.

### Test Article Storage and Handling

The test substance and vehicle were stored according to the received storage conditions supplied by the Sponsor and manufacturer, accordingly.

### Analysis of the Test Article Formulations

Samples of the test article formulation will be cryopreserved for analysis of the concentration, stability and homogeneity of the test substance formulations.

### TEST SYSTEM RATIONALE

The primate is a suitable non rodent species, acceptable to regulatory authorities as an indicator of potential hazards, and for which extensive background data are available. The African green monkey specifically is a highly clinically relevant model of multiple human physiologic and disease states.

The intravenous route of administration corresponds to a possible human therapeutic route. The dose of the test articles was based on the results of the dose finding studies of analogous compounds previously performed in the African green monkey.

African green monkeys were chosen as the primate of choice as the test substances' target sequences are conserved across species with 100 % homology in primates. Additionally, the test substance is a synthetic oligonucleotide. Consequently, dosing in primates allows for a superior assessment of the efficacy of these compounds that would be more reflective of the uptake likely to be seen in humans than in any other species.

### ANIMALS

### Species

*Chlorocebus sabaeus,* non-human primate

### Breed

African green monkey indigenous to St. Kitts.

### Source

RxGen, Lower Bourryeau, St. Kitts, West Indies.

### Expected Age

The test animals were adults.

### Expected Body Weight

The monkeys weigh approximately 3-4 kg. The actual range may vary but will be documented in the data.

### Sex

The test animals were adult females.

### Number of Animals

Ten animals were screened to ensure identification of 8 animals appropriate for enrollment in the study.

### Number on Study

Females: 8

### Justification for Number on Study

This study was designed to use the fewest number of animals possible, consistent with the primary objective of evaluating the therapeutic efficacy of the test article in the African green monkey and prior studies of the systemic administration of this type of oligonucleotide in this species.

### Animal Specification

Ten adult African Green monkeys in the weight range of 3 to 4 kg, were employed in the study. The monkeys were drug-naive adult animals humanely trapped from the feral population that inhabits the island. Trapped monkeys were treated with antihelminthics to eliminate any possible intestinal parasite burden and were observed in quarantine for a minimum of 4 weeks prior to screening for study enrollment. The age of trapped monkeys were estimated by size and dentation, with the exclusion of older animals from the study. Prior to study enrollment, a clinical exam was performed on each monkey, including evaluation of locomotion and dexterity. Blood samples were taken and sent to Antech Diagnostics (Memphis, TN) for comprehensive clinical chemistries and a complete blood count and lipid profiles (see sections 9.2 and 319567928 for specifications). Monkeys with abnormal lab values, as determined by comparison to the established normal range for monkeys in the St. Kitts colony, were excluded from the study. In order to identify 8 monkeys that satisfy this criterion, 10 monkeys were screened, with the screening of additional animals as needed. Before study initiation, the selected monkeys were transferred to individual cages to acclimate to individual housing for a one-week period. Only animals deemed suitable for experimentation were enrolled in the study. The actual (or estimated) age and weight ranges at the start of the study were detailed in the raw data and final report.

### Animal Health and Welfare

The highest standards of animal welfare were followed and adhered to guidelines stipulated by the St. Kitts Department of Agriculture and the U.S. Department of Health and Human Services. All studies will be conducted in accordance with these requirements and all applicable codes of practice for the care and housing of laboratory animals. All applicable standards for veterinary care, operation, and review as contained in the NIH Guide for the Care and Use of Animals. The St. Kitts facility maintains an animal research committee that reviews the protocols and inspects the facilities as required by the Guide. The Foundation has an approved assurance filed with the Office of Laboratory Animal Welfare, as required by the Guide, #A4384-01 (Axion Research Foundation/St. Kitts Biomedical Foundation). There are no special nonhuman primate veterinary care issues and biohazard issues raised by the research specified in this study.

### Housing and Environment

To allow detection of any treatment-related clinical signs, the animals were housed individually prior to surgery and postoperatively until sacrifice. The primate building in which the individual cages were situated were illuminated entirely by ambient light, which at 17 degrees north latitude approximates a 12hr:12hr light-dark cycle as recommended in the U.S. D.H.H.S guidelines. The RxGen primate building was completely ventilated to the outside. Additional air movement was assured by ceiling fans to maintain a constant target temperature of 23-35°C, as is typical of St. Kitts throughout the year. Twenty-four hour extremes of temperature and relative humidity (which also will not be controlled) were measured daily. During the study, the cages were cleaned at regular intervals.

### Diet and Water

Each animal was offered approximately 90 grams per day of a standard monkey chow diet (TekLad, Madison, WI). The specific nutritional composition of the diet was recorded. The water was periodically analyzed for microbiological purity. The criteria for acceptable levels of contaminants in stock diet and water supply were within the analytical specifications established by the diet manufacturer and the periodic facility water evaluations, respectively. The water met all criteria necessary for certification as acceptable for human consumption.

### EXPERIMENTAL DESIGN

### Animal Identification and Randomization

Allocation was done by means of a stratified randomization procedure based on bodyweight and plasma cholesterol profiles. Prior to and after allocation to a group, each animal was identified by a tattoo on the abdomen. Tattoos are placed on all colony animals as a means of identification in the course of routine health inspections. A cage plan was drawn up to identify the individuals housed within, and individual monkeys were further identified by a labeled tag attached to their respective cage. *Group sizes, doses and identification*

### numbers

The animals were assigned to 2 treatment groups, comprised of 4 monkeys in each group. Specific animal identification numbers were provided to each monkey according to the facility numbering system. This system uniquely identifies each monkey by a letter followed by a three digit number, e.g. Y032.

### Route and Frequency of Administration

Animals were dosed once daily on Days 1, 3, and 5 delivered intravenously by manual infusion over ∼10 min. The infusion rate will be 24 mL/kg/h. The animals were sedated with ketamine and xylazine prior to and during the dosing procedure. A venous catheter (Terumo mini vein infusion set, 20 gauge needle, or similar appropriate infusion set) was inserted into the saphenous vein. Dosing took place in each monkey between 8:00 and 10:00 a.m. shortly after the animals wake and prior to feeding. A blood sample to assess plasma cholesterol and other lipid levels as described in Blood Chemistry section below, was collected just prior to each infusion. Blood collection preceded feeding at both sampling intervals to minimize dietary effects on cholesterol measurements.

### Clinical Observations

All visible signs of reaction to treatment were recorded on each day of dosing. In addition, the animals were examined at least once each week for physical attributes such as appearance and general condition.

### Body Weights

Body weights were recorded at weekly intervals during the treatment and posttreatment periods.

### Food Consumption

Individual food consumption was not quantified. Feeding patterns were however monitored and a note made of any major changes.

### Mortality and Morbidity

Mortality and morbidity will be recorded. Any decision regarding premature sacrifice will be made after consultation with the Study Director and with the Sponsor's Monitoring Scientist, if possible. Animals that are found dead or killed prematurely will be subjected to necropsy with collection of liver, kidney, heart and spleen lung tissues for histopathology. In the event of premature sacrifice a blood sample will also be taken (if possible) and the parameters determined. Animals that are found dead after regular working hours will be refrigerated overnight and necropsies performed at the start of the next working day. If the condition of an animal requires premature sacrifice, it will be euthanized by intravenous overdose of sodium pentobarbital. All research is governed by the Principles for Use of Animals. RxGen is required by law to comply with the U.S. Department of Health and Human Services standards for primate facility, which dictates the levels of severity that the procedures within this study, specified as mild, must abide.

### CLINICAL LABORATORY STUDIES

### Fat Biopsies

A subcutaneous fat biopsy was performed on all study monkeys except Y775 on study days 26 by tissue extraction through a 1 cm midline incision inferior to the umbilicus. Biopsies were immediately immersed in a labeled cryotube containing 2 mls of RNAlater (Qiagen) and incubated at **4°C** overnight, after which the RNAlater was aspirated and the sample tube flash frozen in liquid nitrogen. Following transportation in liquid nitrogen total RNA was isolated for real-time qPCR of target genes.

### Results:

Real time PCR results show an increase in SIRT1 mRNA levels in fat biopsies from monkeys dosed with CUR-963, an oligonucleotide designed to SIRT1 antisense CV396200.1, compared to monkeys dosed with CUR-962 (SEQ ID NO.: 71), an oligonucleotide which had no effect on SIRT1 expression *in vitro* (designed to ApoA1 antisense DA327409, data not shown). mRNA levels were determined by real time PCR (Figure 4).

### Example 5: In vivo modulation of Sirtuin 1 (SIRT1) by antisense DNA oligonucleotides

### Treatment with Antisense DNA Oligonucleotides (ASO)

Antisense oligonucleotides (ASO) specific for SIRT1 AS (for example CUR-1098 or CUR1099) are administered to C57B1/6J mice which are fed a high fat diet for 12 weeks to induce obesity and diabetes. (Purushotham A. et al., (2009) Cell Metabolism 9, p. 327-338,). The treatment of the mice with ASO will start at the time of the implementation of the high fat diet. Mice are injected IP once a week with ASO prepared in normal saline, at a concentration of 5mg/kg.

### Measurements of body weight and food intake

Body weight and food intake of mice are measured twice per week, prior to IP injection of the ASO.

### Blood glucose measurements

Fed and fasted blood glucose concentrations are measured each week by taking a sample of blood from the tail vein.

### Glucose Tolerance Tests (GTT)

The GTT will be done totally twice per mouse, halfway through the diet (at week 4) and near the end (at week 10) of the high fat diet. The GTT will inform us about the glucose tolerance of the mice that is the capacity to rapidly clear a glucose bolus from the blood stream. This is a measure for diabetes.

Mice are fasted overnight for 16 hours. Mice are injected IP glucose 2g/kg. This translates into a final volume of 0.2ml 30% (w/v) glucose solution for a mouse of 30g weight. Glucose measurements are taken prior to glucose injection and at 5, 15, 30, 60, 90 and 120 min post-injection. Glucose is measured by cutting the tail tip 1mm from the end of the tail under isoflurane anesthesia prior to IP glucose injection. The blood droplet is aspirated into a strip and glucose concentration is measured with a glucometer. The GTT will be done totally twice per mouse, halfway through the diet (at week 4) and near the end (at week 10) of the high fat diet. The GTT will inform us about the glucose tolerance of the mice that is the capacity to rapidly clear a glucose bolus from the blood stream. This is a measure for diabetes.

### Insulin Tolerance Test (ITT)

Mice are fasted for 6 hours from 9am till 3pm. Mice are then injected IP 0.5-1U Insulin/kg. The insulin concentration will be adjusted such that the final injected volume is 0.1-0.15ml. Blood glucose measurements are taken prior to injection and at 5, 15, 30, 45, and 60 minutes post-injection. Blood is collected exactly as described under GTT. In addition to monitoring the glucose levels, the behavior of the mice is constantly observed during the ITT. Hypoglycemia can manifest as a change in behavior with the animals becoming very quiet and showing discomfort. To prevent hypoglycemia, glucose (1g/kg) is injected IP in a final volume of 0.1-0.15ml as soon as the blood glucose concentration falls below 50mg/ml or signs of discomfort are observed.

### Blood Collection by Facial Vein Puncture

Mice are restrained by the scruff of the neck and base of the tail, slightly compressing the blood vessels of the neck through the tautness of the grip on the neck skin. The sampling site is on the jaw slightly in front of the angle of the mandible. The skin at the sampling site is punctured with an 18G needle or a lancet at a 90° angle until the tip of the needle/lancet just passes through the skin. Blood samples are collected using microhematocrit tubes. After blood has been collected, the grip on the neck is loosened and pressure is applied at the insertion site with a gauze sponge to ensure hemostasis. 0.05-0.2ml of blood will be collected by this method. This procedure will be performed only once in week 5 of the high fat diet and eventually in week 12 if the intracardiac puncture is not working (see below). Blood hormones which regulate the metabolism of glucose and lipids (such as insulin, adiponectin and leptin) are measured using commercially available ELISA kits. (e.g., R&D Systems, Minneapolis, MN, Assay Pro St. Charles, MO, Mabtech, Mariemont, OH)

### Intracardiac Puncture

At the end of the 12 week high fat diet, mice will be anesthetized by continuous isoflurane inhalation. Anesthesia is induced by placing the mice in an induction box, which is supplied with isoflurane and oxygen. Mice will be restrained on their back. The heart is punctured with a 27G needle. Following exsanguineation, the head is decapitated to ensure death. Tissues (liver, pancreas, white and brown adipose tissue, and skeletal muscle) are collected for further investigations (RNA and protein measurements and histology). Around 0.5 - 1 ml of blood will be obtained and used to determine several critical parameters of of glucose and lipid metabolism (glucose, insulin, cholesterol, triglycerides, free fatty acids, leptin, adipokines, corticosteroids, thyroid hormones). If difficulties occur in this method, we will collect blood by facial vein puncture under isoflurane anesthesia instead (see above).

## Claims

1. An antisense oligonucleotide which binds a human Sirtuin 1 (SIRT1) natural antisense transcript for use as a therapeutic compound, wherein the Sirtuin (SIRT1) natural antisense transcript is SEQ ID NO: 7, and wherein said antisense oligonucleotide increases the expression of human Sirtuin 1 (SIRT1) corresponding to SEQ ID NO: 1.

2. An antisense oligonucleotide which binds a human Sirtuin 1 (SIRT1) natural antisense transcript for use in the prevention or treatment of a Sirtuin 1 (SIRT1) associated metabolic disease or disorder selected from insulin resistance, diabetes, adult-onset diabetes, diabetic nephropathy, type 2 diabetes, obesity, impaired glucose tolerance, high blood cholesterol, hyperglycemia, dyslipidemia or hyperlipidemia, or for use in the prevention or treatment of a SIRT1 associated neurodegenerative disease, wherein the Sirtuin (SIRT1) natural antisense transcript is SEQ ID NO: 7, and wherein said antisense oligonucleotide increases the expression of human Sirtuin 1 (SIRT1) corresponding to SEQ ID NO: 1.

3. Use of an antisense oligonucleotide which binds a human Sirtuin 1 (SIRT1) natural antisense transcript for the manufacture of a medicament for the prevention or treatment of a Sirtuin 1 (SIRT1) associated metabolic disease or disorder selected from insulin resistance, diabetes, adult-onset diabetes, diabetic nephropathy, type 2 diabetes, obesity, impaired glucose tolerance, high blood cholesterol, hyperglycemia, dyslipidemia or hyperlipidemia, or for the prevention or treatment of a SIRT1 associated neurodegenerative disease, wherein the Sirtuin (SIRT1) natural antisense transcript is SEQ ID NO: 7, and wherein said antisense oligonucleotide increases the expression of human Sirtuin 1 (SIRT1) corresponding to SEQ ID NO: 1.

4. An antisense oligonucleotide for use according to Claim 2 or use of an antisense oligonucleotide according to Claim 3, wherein the neurodegenerative disease is selected from Alzheimer's, Huntington's, Parkinson's, Amyotrophic Lateral Sclerosis, Multiple Sclerosis, and disorders caused by polyglutamine aggregation, AIDS related dementia or neuropathy (e.g., sensory neuropathy, autonomic neuropathy, motor neuropathy, retinopathy).

5. A method of increasing the expression of Sirtuin 1 (SIRT1) in patient cells or tissues *in vitro* comprising: contacting said cells or tissues with an antisense oligonucleotide which binds a human Sirtuin 1 (SIRT1) natural antisense transcript, wherein the Sirtuin (SIRT1) natural antisense transcript is SEQ ID NO: 7; thereby increasing the expression of human Sirtuin 1 (SIRT1) corresponding to SEQ ID NO: 1.

6. An antisense oligonucleotide for use according to any one of Claims 1, 2, or 4, or use of an antisense oligonucleotide according to any one of Claims 3 or 4, or a method according to Claim 5, wherein:
a. the antisense oligonucleotide is a siRNA compound; or
b. the antisense oligonucleotide is single-stranded.

7. An antisense oligonucleotide for use according to any one of Claims 1, 2, 4, or 6, or use of an antisense oligonucleotide according to any one of Claims 3, 4 or 6, or a method according to Claim 5 or Claim 6, wherein the antisense oligonucleotide comprises one of SEQ ID NOs: 52 or 54.

8. An antisense oligonucleotide for use according to any of Claims 1, 2, 4, 6 or 7, or use of an antisense oligonucleotide according to any one of Claims 3, 4, 6 or 7 or a method according to any one of Claims 5 to 7, wherein the expression of human Sirtuin 1 (SIRT1) corresponding to SEQ ID NO: 1 is increased by at least 10%.

9. An antisense oligonucleotide for use according to any one of Claims 1, 2, 4, 6 to 8 or use of an antisense oligonucleotide according to any one of Claims 3, 4, or 6 to 8 or a method according to any one of Claims 5 to 8, wherein the antisense oligonucleotide further comprises one or more modifications comprising:
a. at least one modified internucleoside linkage selected from: a phosphorothioate, 2'- Omethoxyethyl (MOE), 2'-fluoro, alkylphosphonate, phosphorodithioate, alkylphosphonothioate, phosphoramidate, carbamate, carbonate, phosphate triester, acetamidate, carboxymethyl ester, and combinations thereof;
b. at least one modified nucleotide selected from: a peptide nucleic acid (PNA), a locked nucleic acid (LNA), an arabino-nucleic acid (FANA), an analogue, a derivative, and combinations thereof; or
c. at least one modified sugar moiety selected from: a 2'-O-methoxyethyl modified sugar moiety, a 2'-methoxy modified sugar moiety, a 2'-O-alkyl modified sugar moiety, a bicyclic sugar moiety, and combinations thereof.

10. An antisense oligonucleotide having the features of an antisense oligonucleotide defined in any one of Claims 1 to 9.

11. An antisense oligonucleotide according to Claim 10, wherein the antisense oligonucleotide is between 10 to 30 nucleotides in length.

12. An antisense oligonucleotide according to Claim 10 or Claim 11, wherein the antisense oligonucleotide has at least 90% sequence identity to a complement of a Sirtuin 1 (SIRT1) natural antisense transcript as set forth in SEQ ID NO: 7.

13. A pharmaceutical composition comprising at least one antisense oligonucleotide according to any of Claims 10 to 12 and a pharmaceutically acceptable excipient.

## Patentansprüche

1. Antisense-Oligonukleotid, das ein natürliches Antisense-Transkript von humanem Sirtuin 1 (SIRT1) bindet, zur Verwendung als eine therapeutische Verbindung, wobei das natürliche Antisense-Transkript von Sirtuin (SIRT1) SEQ ID Nr. 7 ist und wobei das Antisense-Oligonukleotid die Expression von humanem Sirtuin 1 (SIRT1) erhöht, das SEQ ID Nr. 1 entspricht.

2. Antisense-Oligonukleotid, das ein natürliches Antisense-Transkript von humanem Sirtuin 1 (SIRT1) bindet, zur Verwendung bei der Verhinderung oder Behandlung einer mit Sirtuin 1 (SIRT1) assoziierten Stoffwechselkrankheit oder - erkrankung, die aus Insulinresistenz, Diabetes, Altersdiabetes, diabetischer Nephropathie, Typ-2-Diabetes, Adipositas, gestörter Glukosetoleranz, hohem Blutcholesterin, Hyperglykämie, Dyslipidämie oder Hyperlipidämie ausgewählt ist, oder zur Verwendung bei der Verhinderung oder Behandlung einer mit SIRT1 assoziierten neurodegenerativen Krankheit, wobei das natürliche Antisense-Transkript von Sirtuin (SIRT1) SEQ ID Nr. 7 ist und wobei das Antisense-Oligonukleotid die Expression von humanem Sirtuin 1 (SIRT1) erhöht, das SEQ ID Nr. 1 entspricht.

3. Verwendung eines Antisense-Oligonukleotids, das ein natürliches Antisense-Transkript von humanem Sirtuin 1 (SIRT1) bindet, zur Herstellung eines Arzneimittels zur Verhinderung oder Behandlung einer mit Sirtuin 1 (SIRT1) assoziierten Stoffwechselkrankheit oder -erkrankung, die aus Insulinresistenz, Diabetes, Altersdiabetes, diabetischer Nephropathie, Typ-2-Diabetes, Adipositas, gestörter Glukosetoleranz, hohem Blutcholesterin, Hyperglykämie, Dyslipidämie oder Hyperlipidämie ausgewählt ist, oder zur Verhinderung oder Behandlung einer mit SIRT1 assoziierten neurodegenerativen Krankheit, wobei das natürliche Antisense-Transkript von Sirtuin (SIRT1) SEQ ID Nr. 7 ist und wobei das Antisense-Oligonukleotid die Expression von humanem Sirtuin 1 (SIRT1) erhöht, das SEQ ID Nr. 1 entspricht.

4. Antisense-Oligonukleotid zur Verwendung nach Anspruch 2 oder Verwendung eines Antisense-Oligonukleotids nach Anspruch 3, wobei die neurodegenerative Krankheit aus Alzheimer-Krankheit, Chorea Huntington, Parkinson-Krankheit, amyotropher Lateralsklerose, multipler Sklerose und Erkrankungen, die von Polyglutamin-Aggregation verursacht werden, AIDS-bedingter Demenz oder Neuropathie (z. B. sensorische Neuropathie, autonome Neuropathie, motorische Neuropathie, Retinopathie) ausgewählt ist.

5. Verfahren zur Erhöhung der Expression von Sirtuin 1 (SIRT1) in Patientenzellen oder -geweben *in vitro,* umfassend: Inkontaktbringen der Zellen oder Gewebe mit einem Antisense-Oligonukleotid, das ein natürliches Antisense-Transkript von humanem Sirtuin 1 (SIRT1) bindet, wobei das natürliche Antisense-Transkript von Sirtuin (SIRT1) SEQ ID Nr. 7 ist; wodurch die Expression von humanem Sirtuin 1 (SIRT1) erhöht wird, das SEQ ID Nr. 1 entspricht.

6. Antisense-Oligonukleotid zur Verwendung nach einem der Ansprüche 1, 2 oder 4 oder Verwendung eines Antisense-Oligonukleotids nach einem der Ansprüche 3 oder 4 oder Verfahren nach Anspruch 5, wobei:
a. das Antisense-Oligonukleotid eine siRNA-Verbindung ist; oder
b. das Antisense-Oligonukleotid einzelsträngig ist.

7. Antisense-Oligonukleotid zur Verwendung nach einem der Ansprüche 1, 2, 4 oder 6 oder Verwendung eines Antisense-Oligonukleotids nach einem der Ansprüche 3, 4 oder 6 oder Verfahren nach Anspruch 5 oder 6, wobei das Antisense-Oligonukleotid eine der SEQ ID Nrn. 52 oder 54 umfasst.

8. Antisense-Oligonukleotid zur Verwendung nach einem der Ansprüche 1, 2, 4, 6 oder 7 oder Verwendung eines Antisense-Oligonukleotids nach einem der Ansprüche 3, 4, 6 oder 7 oder Verfahren nach einem der Ansprüche 5 bis 7, wobei die Expression von humanem Sirtuin 1 (SIRT1), das SEQ ID Nr. 1 entspricht, um mindestens 10 % erhöht wird.

9. Antisense-Oligonukleotid zur Verwendung nach einem der Ansprüche 1, 2, 4, 6 bis 8 oder Verwendung eines Antisense-Oligonukleotids nach einem der Ansprüche 3, 4 oder 6 bis 8 oder Verfahren nach einem der Ansprüche 5 bis 8, wobei das Antisense-Oligonukleotid weiterhin eine oder mehrere Modifikationen umfasst, umfassend:
a. mindestens eine modifizierte Internukleosidverknüpfung, ausgewählt aus: einem Phosphorothioat, 2'-O-Methoxyethyl (MOE), 2'-Fluor, Alkylphosphonat, Phosphorodithioat, Alkylphosphonothioat, Phosphoramidat, Carbamat, Carbonat, Phosphattriester, Acetamidat, Carboxymethylester und Kombinationen davon;
b. mindestens ein modifiziertes Nukleotid, ausgewählt aus: einer Peptidnukleinsäure (PNA), einer Locked-Nukleinsäure (LNA), einer Arabinonukleinsäure (FANA), einem Analogon, einem Derivat und Kombinationen davon; oder
c. mindestens eine modifizierte Zuckereinheit, ausgewählt aus: einer 2'-O-Methoxyethyl-modifizierten Zuckereinheit, einer 2'-Methoxy-modifizierten Zuckereinheit, einer 2'-O-Alkyl-modifizierten Zuckereinheit, einer bicyclischen Zuckereinheit und Kombinationen davon.

10. Antisense-Oligonukleotid mit den Merkmalen eines in einem der Ansprüche 1 bis 9 definierten Antisense-Oligonukleotids.

11. Antisense-Oligonukleotid nach Anspruch 10, wobei das Antisense-Oligonukleotid eine Länge zwischen 10 bis 30 Nukleotiden aufweist.

12. Antisense-Oligonukleotid nach Anspruch 10 oder 11, wobei das Antisense-Oligonukleotid eine Sequenzidentität von mindestens 90 % zu einem Komplement eines natürlichen Antisense-Transkripts von Sirtuin 1 (SIRT1) aufweist, wie in SEQ ID Nr. 7 dargelegt.

13. Pharmazeutische Zusammensetzung, umfassend mindestens ein Antisense-Oligonukleotid nach einem der Ansprüche 10 bis 12 und einen pharmazeutisch annehmbaren Hilfsstoff.

## Revendications

1. Oligonucléotide antisens qui se lie à un transcrit antisens naturel de la Sirtuine 1 (SIRT1) humaine, destiné à une utilisation comme composé thérapeutique, le transcrit antisens naturel de la Sirtuine (SIRT1) étant la SÉQ. ID n° 7, et ledit oligonucléotide antisens augmentant l'expression de la Sirtuine 1 (SIRT1 humaine) correspondant à la SÉQ. ID n° 1.

2. Oligonucléotide antisens qui se lie à un transcrit antisens naturel de la Sirtuine 1 (SIRT1) humaine, destiné à une utilisation dans la prévention ou le traitement d'une maladie ou d'une affection métabolique associée à la Sirtuine 1 (SIRT1), sélectionnée parmi une insulinorésistance, un diabète, un diabète de l'âge mûr, une néphropathie diabétique, un diabète de type 2, une obésité, une tolérance altérée au glucose, un taux élevé de cholestérol sanguin, une hyperglycémie, une dyslipidémie ou une hyperlipidémie, ou destiné à une utilisation dans la prévention ou le traitement d'une maladie neurodégénérative associée à SIRT1, le transcrit antisens naturel de la Sirtuine (SIRT1) étant la SÉQ. ID n° 7, et ledit oligonucléotide antisens augmentant l'expression de la Sirtuine 1 (SIRT1) humaine correspondant à la SÉQ. ID n° 1.

3. Utilisation d'un oligonucléotide antisens qui se lie à un transcrit antisens naturel de la Sirtuine 1 (SIRT1) humaine pour la fabrication d'un médicament destiné à la prévention ou au traitement d'une maladie ou d'une affection métabolique associée à la Sirtuine 1 (SIRT1), sélectionnée parmi une insulinorésistance, un diabète, un diabète de l'âge mûr, une néphropathie diabétique, un diabète de type 2, une obésité, une tolérance altérée au glucose, un taux élevé de cholestérol sanguin, une hyperglycémie, une dyslipidémie ou une hyperlipidémie, ou destiné à la prévention ou au traitement d'une maladie neurodégénérative associée à SIRT1, le transcrit antisens naturel de la Sirtuine (SIRT1) étant la SÉQ. ID n° 7, et ledit oligonucléotide antisens augmentant l'expression de la Sirtuine 1 (SIRT1) humaine correspondant à la SÉQ. ID n° 1.

4. Oligonucléotide antisens destiné à une utilisation selon la revendication 2 ou utilisation d'un oligonucléotide antisens selon la revendication 3, la maladie neurodégénérative étant sélectionnée parmi la maladie d'Alzheimer, la maladie de Huntington, la maladie de Parkinson, la sclérose latérale amyotrophique, la sclérose en plaques, et des affections causées par une agrégation de la polyglutamine, une démence ou une neuropathie liée au sida (p. ex. neuropathie sensitive, neuropathie du système nerveux autonome, neuropathie motrice, rétinopathie).

5. Procédé d'augmentation *in vitro* de l'expression de la Sirtuine 1 (SIRT1) dans des cellules ou des tissus de patients, comprenant : la mise en contact desdites cellules ou desdits tissus avec un oligonucléotide antisens qui se lie à un transcrit antisens naturel de la Sirtuine 1 (SIRT1) humaine, le transcrit antisens naturel de la Sirtuine (SIRT1) étant la SÉQ. ID n° 7, en augmentant de ce fait l'expression de la Sirtuine 1 (SIRT1) humaine correspondant à la SÉQ. ID n° 1.

6. Oligonucléotide antisens destiné à une utilisation selon l'une quelconque des revendications 1, 2 ou 4, ou utilisation d'un oligonucléotide antisens selon l'une quelconque des revendications 3 ou 4, ou procédé selon la revendication 5, dans lequel ou dans laquelle :
a. l'oligonucléotide antisens est un composé d'ARNsi ; ou
b. l'oligonucléotide antisens est monobrin.

7. Oligonucléotide antisens destiné à une utilisation selon l'une quelconque des revendications 1, 2, 4 ou 6, ou utilisation d'un oligonucléotide antisens selon l'une quelconque des revendications 3, 4 ou 6, ou procédé selon la revendication 5 ou la revendication 6, l'oligonucléotide antisens comprenant l'une des SÉQ. ID n^{os} 52 ou 54.

8. Oligonucléotide antisens destiné à une utilisation selon l'une quelconque des revendications 1, 2, 4, 6 ou 7, ou utilisation d'un oligonucléotide antisens selon l'une quelconque des revendications 3, 4, 6 ou 7, ou procédé selon l'une quelconque des revendications 5 à 7, l'expression de la Sirtuine 1 (SIRT1) humaine correspondant à la SÉQ. ID n° 1 étant augmentée d'au moins 10 %.

9. Oligonucléotide antisens destiné à une utilisation selon l'une quelconque des revendications 1, 2, 4, 6 à 8, ou utilisation d'un oligonucléotide antisens selon l'une quelconque des revendications 3, 4, ou 6 à 8, ou procédé selon l'une quelconque des revendications 5 à 8, l'oligonucléotide antisens comportant en outre une ou plusieurs modifications comprenant :
a. au moins une liaison internucléosidique modifiée sélectionnée parmi : un phosphorothioate, 2'-O-méthoxyéthyle (MOE), 2'-fluoro, alkylphosphonate, phosphorodithioate, alkylphosphonothioate, phosphoramidate, carbamate, carbonate, triester de phosphate, acétamidate, ester carboxyméthylique, et des combinaisons de ceux-ci ;
b. au moins un nucléotide modifié sélectionné parmi : un acide nucléique peptidique (PNA), un acide nucléique bloqué (LNA), un acide arabino-nucléique (FANA), un analogue, un dérivé, et des combinaisons de ceux-ci ; ou
c. au moins une fraction sucre modifiée sélectionnée parmi : une fraction sucre modifiée par un groupe 2'-O-méthoxyéthyle, une fraction sucre modifiée par un groupe 2'-méthoxy, une fraction sucre modifiée par un groupe 2'-O-alkyle, une fraction sucre bicyclique, et des combinaisons de celles-ci.

10. Oligonucléotide antisens ayant les caractéristiques d'un oligonucléotide antisens défini dans l'une quelconque des revendications 1 à 9.

11. Oligonucléotide antisens selon la revendication 10, l'oligonucléotide antisens ayant une longueur de 10 à 30 nucléotides.

12. Oligonucléotide antisens selon la revendication 10 ou la revendication 11, l'oligonucléotide antisens ayant une identité de séquence d'au moins 90 % avec un complément d'un transcrit antisens naturel de la Sirtuine 1 (SIRT1) tel qu'énoncé dans la SÉQ. ID n° 7.

13. Composition pharmaceutique comprenant au moins un oligonucléotide antisens selon l'une quelconque des revendications 10 à 12 et un excipient pharmaceutiquement acceptable.
